# FASCICULE DE BREVET EUROPEEN

(11) **EP 1 863 832 B1**
(45) Date de publication et mention de la délivrance du brevet: **04.01.2012**
(21) Numéro de dépôt: 06725441.7
(22) Date de dépôt: 30.03.2006
(51) Int. Cl.: C07K 5/06, C07K 5/08, A61K 38/05, A61K 38/06

(54) **INHIBITEURS D'AGE**
AGE-INHIBITOREN
AGE INHIBITORS

(30) Priorité: 31.03.2005 FR 0503176
(43) Date de publication de la demande: 12.12.2007
(73) Titulaire: CENTRE NATIONAL DE LA RECHERCHE SCIENTIFIQUE (CNRS), 75016 Paris (FR)
(72) Inventeur: SASAKI, Nobumichi, André, F-80260 Flesselles (FR); ACHAB GARCIA ALVAREZ, Maria, Concepcion, F-91190 Gif s/Yvette (FR); WANG-ZHU, Qian, F-91190 Gif s/Yvette (FR); ERMOLENKO, Lioudmila, F-91190 Gif s/Yvette (FR); BAKALA, Joanna, F-75012 Paris (FR); FRANCK, Gisèle, F-94230 Cachan (FR); BAKRIM NHIRI, Naïma, F-91400 Orsay (FR); POTIER, Pierre, décédé (FR)
(74) Mandataire: Warcoin, Jacques
(86) Numéro de dépôt international: PCT/EP2006/061191
(87) Numéro de publication internationale: WO 2006/103274

(56) Documents cités:
- WO-A-92/14456
- WO-A-96/23225
- WO-A-2004/002418

## Description

La réaction de Maillard, glycation non enzymatique, est initiée par la condensation d'un groupe amino présent dans les protéines avec un composé contenant un groupe carbonyle, généralement un sucre. Une multitude de produits résultent des derniers stades de ce processus complexe, qui sont dénommés "produits de glycation avancée" (AGEs). La conséquence de la formation de ces AGEs est la réticulation protéique. Ces réticulations ont été observées dans les protéines à longue durée de vie telles que le collagène, le cristallin, la fibronectine, la tubuline, la myéline, la laminine, l'actine, l'hémoglobine, l'albumine et les lipides associés aux lipoprotéines de basse densité (LDL). Les protéines modifiées par les AGEs augmentent progressivement avec l'âge et on considère qu'elles contribuent au remodelage des tissus normaux. En outre, la formation et l'accumulation accrues d'AGEs ont été liées au développement de cataracte (Nagaraj et al. J. Biol. Chem. (1996) 271, 19338), de l'urémie (Miyata et al. Kidney Int. (1999) 55, 389), d'athérosclérose (Kume et al.. Am. J. Pathol. (1995) 147, 654; Stitt et al. Mol. Med. (1997) 3, 617), de la maladie d'Alzheimer (Münch et al. Biochem. Soc. Trans. (2003) 31 (6), 1397; Lüth et al. Cerebral Cortex (2005) 15(2), 211), de la maladie de Parkinson (Webster et al. Neurotoxicity Res. (2005) /(172), 95), de maladies inflammatoires (Anderson et al. J. Clin. Invest. (1999) 104, 103), de troubles rhumatismaux liés à l'âge, et pardessus tout, de complications cliniques du diabète mellitus (Brownlee, M. Ann. Rev. Med (1995) 461, 223 ; Brinkmann et al. J. Biol Chem. (1998) 273, 18714). Chez les patients diabétiques dont la glycémie est élevée et persistante, le taux de protéines réticulées augmente ce qui entraîne la formation de lésions tissulaires par modification de la structure et de la fonction des protéines impliquées. De plus, les AGEs se lient à des récepteurs membranaires et stimulent des réponses cellulaires. Depuis la découverte de Maillard au début du siècle dernier, on a considéré que le glucose était le sucre qui participait à la réaction de réticulation. Cependant, plus récemment, on a concentré notre attention sur les composés - dicarbonyles tels que le méthylglyoxal (MG), le glyoxal (GO), et la 3-désoxyglucosone (3-DG), en tant qu'agents de réticulation actifs *in vivo* et *in vitro.* On considère que la principale source de MG est la déphosphorylation non enzymatique du triose-dihydroxyacétone phosphate et du glycéraldéhyde-3-phosphate qui sont des métabolites du glucose. Le MG peut aussi être formé par la décomposition spontanée de trioses phosphates ou par le métabolisme de la thréonine ou de l'acétone. Un certain nombre de travaux ont également confirmé la génération d'α-dicarbonyles via l'auto-oxydation du glucose. On suppose que les α-dicarbonyles peuvent être générés lors de la transformation d'une cétoamine, connue comme le produit d'Amadori, un intermédiaire clef dans la réaction de Maillard. Cette cétoamine est elle-même générée par la transformation de l'adduit base de Schiff qui est initialement formé lors de la réaction du glucose avec une amine. En outre, on a rapporté que les bactéries produisaient du MG. La peroxydation lipidique des acides gras polyinsaturés donne aussi des composés carbonyles réactifs, tels que le MG et le GO et ceux caractéristiques des lipides, tels que le malondialdéhyde (MDA) et le 4-hydroxynonénal. En général, ces dicarbonyles très réactifs se lient aux groupes amino, guanidine et sulfhydryles des protéines et forment de manière irréversible des AGEs, tels que la *N_{ε}*-(1-carboxyéthyl)lysine (CEL), la *N*_{ε}-(1-carboxyméthyl)lysine (CML), l'hydroimidazolone dérivée de méthylglyoxal *N*_{δ}-(5-hydro-5-méthyl-4-imidazolon-2-yl)-ornithine (MG-H₁), l'hydroimidazolone dérivée de glyoxal (G-H₁), l'argpyrimidine, le dimère de lysine dérivé du glyoxal, le sel de 1,3-di(*N*^{ε}-lysino)imidazolium (GOLD), le dimère de lysine dérivé du méthylglyoxal, sel de 1,3-di(*N*^{ε}-lysino)-4-méthylimidazolium (MOLD). Le mécanisme d'action *in vivo* de ces composés α-dicarbonyles a été étudié pour essayer de comprendre la progression de la réaction de Maillard dans l'organisme. Dans le cas de sujets diabétiques, une formation et une accumulation accrues des AGEs a lieu, conduisant ainsi à une série de complications à long terme du diabète telles que la néphropathie, la rétinopathie, la neuropathie, les ulcères et des complications micro- et macro-vasculaires (Bucala et al. Diabetes Reviews (1995) 3, 258 ; Ulrich et al. Recent Prog. Horm. Res. (2001) 56, 1; Porta et al. Diabetologia (2002) 45, 1617; Lorenzi et al. Diabetologia (2001) 44, 791; Ziegler et al. Int. Rev. Neurobiol. (2002) 50, 451; Thornallay, P. J. Int. Rev. Neurobiol. (2002) 50, 37 ; Chiarelli et al. Diab. Nutr. Metab. (2000) 13, 192). Et plus particulièrement, des lésions tissulaires rénales causées par les AGEs conduiraient à la perte progressive de la fonction rénale (Makita Z., et al. N. Eng. J. Med. (1991) 325, 836). En effet, chez les patients diabétiques (type 1 et type 2), la concentration plasmatique en méthylglyoxal s'est avérée être 2 à 6 fois supérieure à celle de sujets normaux (McLellan et al. Clin. Sci. (1994) 87, 21).

Un autre facteur associé au vieillissement et aux critères courants de maladies chroniques, telles que le diabète, l'athérosclérose et les maladies vasculaires liées, la polyarthrite rhumatoïde et l'urémie est le stress oxydatif. On définit le stress oxydatif comme un déséquilibre grave entre les systèmes de génération des antioxydants et des oxydants. Une augmentation du stress oxydatif peut avoir un effet profond sur la modification des lipoprotéines, sur la transcription ainsi que sur la fonction et le métabolisme des cellules. Le stress oxydatif peut apparaître via plusieurs mécanismes associés à une production excessive de radicaux oxygènes, tels que l'auto-oxydation du glucose et des protéines glyquées, et la glycation des enzymes anti-oxydantes. En effet, on a rapporté que le MG générait des espèces réactives oxygénées (ERO) (radicaux libres) au cours des réactions de glycation. Ainsi, il est possible de dire que le stress oxydatif et la formation d'AGEs sont inséparablement imbriqués.

Normalement, le système des glyoxalases (glyoxalase I et glyoxalase II) et l'aldose réductase catalysent la détoxication de ces -dicarbonyles en D-lactate, en glycolate et en acétol. Cependant, un dysfonctionnement de ce métabolisme de détoxication entraîne une augmentation de la quantité d'AGEs formés par α-dicarbonyles très réactifs dans l'organisme.

L'inhibition de la formation d'AGEs peut retarder la progression de la physiopathologie des maladies liées aux AGEs et améliorer la qualité de vie durant le vieillissement. On peut ainsi supposer que le piégeage pharmacologique des composés -dicarbonyles est une stratégie thérapeutique de valeur pour empêcher les complications du diabète. Un grand nombre de documents existent concernant le fait qu'une intervention pharmacologique précoce contre les conséquences à long terme de la réticulation empêche le développement des complications ultérieures du diabète. Même s'ils ne peuvent pas guérir le processus pathologique sous-jacent, les inhibiteurs de la formation d'AGEs devraient retarder le développement de complications résultant des troubles fondamentaux. Parmi les médicaments spécifiquement développés en tant qu'inhibiteurs de la formation d'AGEs, l'aminoguanidine (Pimagedine, AG) est l'agent le plus étudié et également le plus utilisé. L'AG est un composé nucléophile qui a deux fonctionnalités réactives clés : la fonction hydrazine nucléophile -NHNH₂ et la fonction guanidine de direction α-dicarbonyle -NH-C(=NH)NH₂. Ces deux groupes fonctionnels liés l'un à l'autre fournissent conjointement un piégeur bifonctionnel et réactif de méthylglyoxal, glyoxal et 3-désoxyglucosone (Brownlee, et al. Science (1986) 232, 1629). Bien que les effets bénéfiques de l'AG contre les complications du diabète aient été largement confirmés chez le modèle de rat diabétique, l'AG est un inhibiteur sélectif bien connu du monoxyde d'azote (NO) et un essai clinique portant sur la prévention de la progression de la néphropathie diabétique par l'AG a été abandonné en raison de préoccupations de sécurité (Oturai et al. APMIS (1996) 104, 259 ; Monnier, V. M. Arch. Biochem. Biophys. (2003) 419, 1). La pyridoxamine (pyridon) est un autre agent qui est capable de prévenir les complications chez le rat diabétique avec une efficacité supérieure à celle de l'aminoguanidine, et il est capable de piéger les produits de la peroxydation lipidique et les composés α-dicarbonyles (Metz et al. Archives of Biochemistry and Biophysics (2003) 419, 41). Un anti-hyperglycémique largement utilisé, la metformine, et employé dans la prise en charge du diabète de type 2, réduirait aussi les taux de méthylglyoxal et de glyoxal à la fois *in vivo* et *in vitro* par la formation de triazépinones (Beisswenger et al. Diabetes Metab. (2003) 29, 6895). Cependant, l'AG s'est avérée être un bien meilleur piégeur (450 fois supérieur) du méthylglyoxal comparé à la metformine (Battah et al. Intern. Congress Series 1245 (2002) 355). D'autres composés possédant des capacités d'inhibition de la formation d'AGEs comprennent la D-pénicillamine (Wondrak Get al. Biochem. Pharmacol. (2002) 63, 361), le LR-90, le méthylène bis (4,4'-(acide 2-chlorophényluréidophénoxyisobutyrique)) (Rahbar et al. Arch. Biochem. Biophys. (2003) 419, 63), la thiamine (Benfotiamine) (Stracke et al. J. Exp. Clin. Endocrinol. Diabetes (2001) 109, 330), la carnosine (β-alanyl-L-histidine), un dipeptide naturel largement répandu dans les tissus mammifères (Hipkiss. A. R Int. J. Biochem. Cell Biol. (1998) 30, 863), la curcumine (Sajithlal et al G. Biochem. Pharmacol. (1998) 56, 1607), un autre composé naturel isolé de *Curcuma longa,* la 2,3-diaminophénazine (NNC39-0028) (Soulis, et al. Diabetologia (1999) 42, 472). Etant donné les impacts marqués des AGEs sur la qualité de vie durant le vieillissement, il existe toujours un besoin de développer des agents efficaces qui peuvent piéger les composés α-dicarbonyles très réactifs, tels que le méthylglyoxal, le glyoxal et la 3-désoxyglucosone et qui ont une faible cytotoxicité et une faible mutagénicité.

De façon surprenante, les présents inventeurs ont découvert une nouvelle classe de composés, capables d'inhiber la formation de produits de glycation avancée en piégeant les composés α-dicarbonyles réactifs.

Certains de ces composés sont déjà connus en tant que tels mais pas dans leur application thérapeutique.

Ainsi, la demande de brevet WO02/100344 décrit l'intermédiaire de synthèse

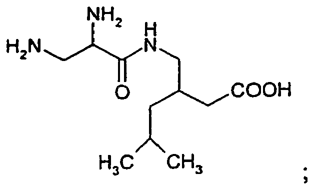

L'article de Jones et al. (Tetrahedron Letters (1988), 29 (31), pages 3856-3856) décrit les intermédiaires de synthèse

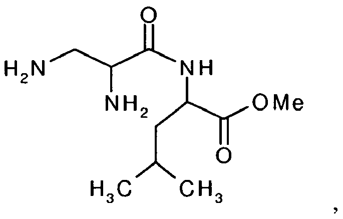

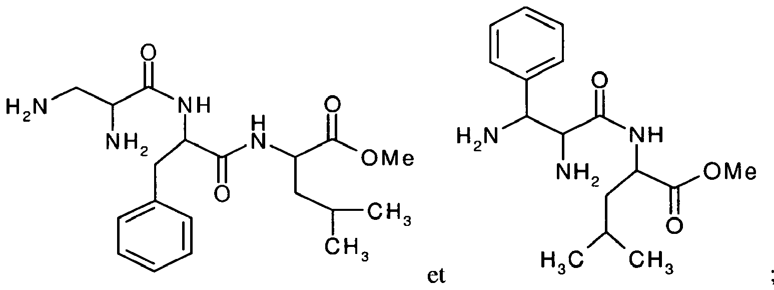

L'article de Kasina et al. (Journal of Medicinal Chemistry (1986), 29 (10), pages 1933-1940) décrit l'intermédiaire de synthèse

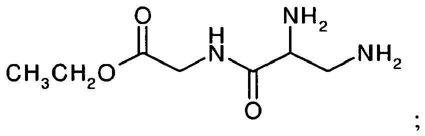

L'article de Tada et al. (Journal of Agricultural And Food Chemistry (1984), 32 (5), pages 992-996) décrit le goût des peptides de formules I pour lesquels R₂ représente un atome d'hydrogène, X représente C=O, R₁ représente -NH-(CH₂)ₘ-COOH et m = 1, 2 ou 3 ;

L'article de Shinoda et al. (Peptide Chemistry (1984), volume date 1983, 21st, pages 43-46) décrits les peptides ayant un goût salé :

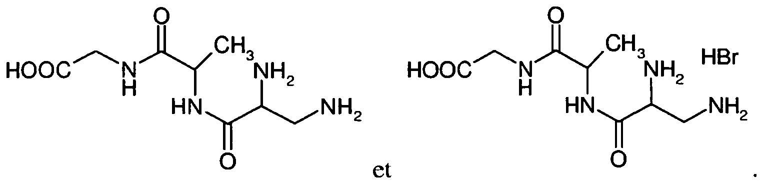

Seule la demande de brevet WO 2004/002418 décrit le peptide de formule suivante : 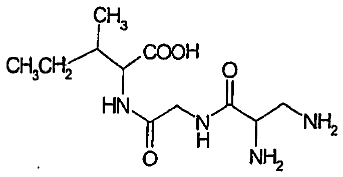 et son application thérapeutique. Toutefois ce document n'indique pas que ce peptide est inhibiteur d'AGEs.

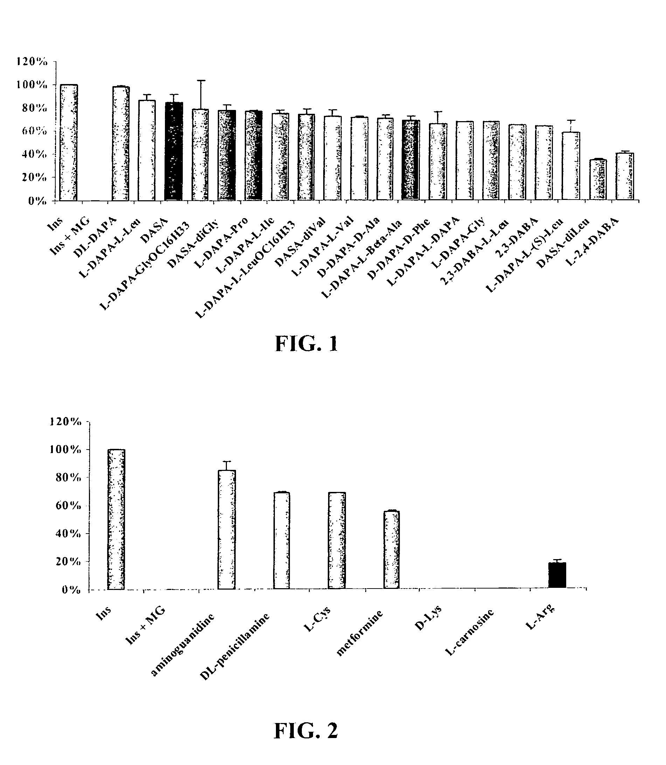

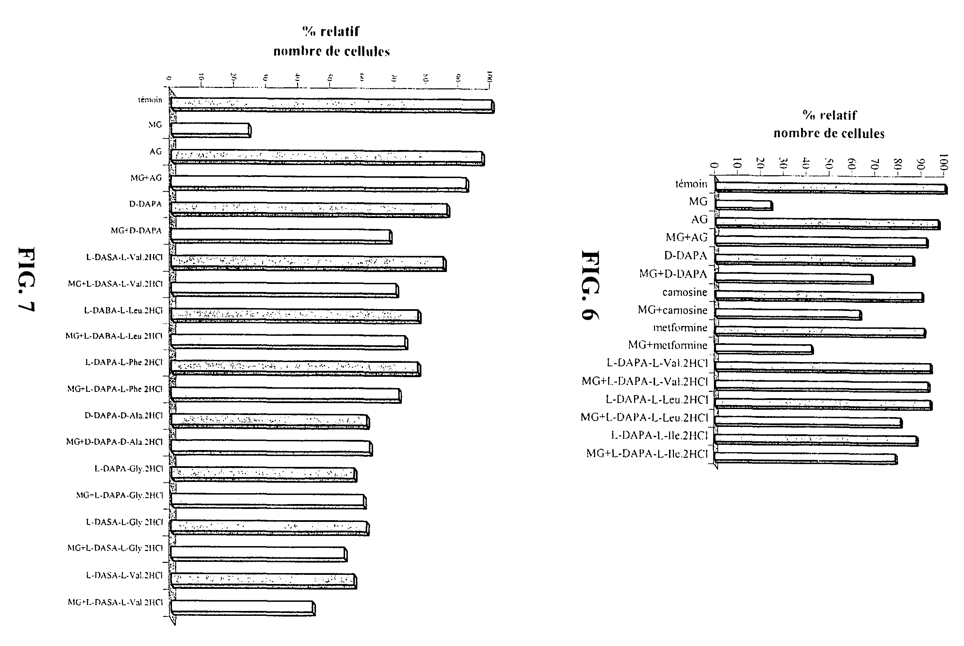

Par ailleurs des dérivés analogues à ceux découverts par les inventeurs, l'acide 2,3-diaminopropionique (DAPA), en particulier, sont décrits dans une demande de brevet (WO 92/14456). Le DAPA serait très susceptible à la décarboxylation par l'ornithine décarboxylase, une enzyme ubiquitaire qui participe à la synthèse d'un grand nombre de polyamines, conduisant à l'éthylènediamine et/ou au 2-aminoacétamide. En vue de faciliter l'élimination par l'urine des produits de condensation des composés α-dicarbonyles et des agents piégeurs, la présence d'un groupe fonctionnel acide tel que -COOH ou SO₃H dans les molécules piégeuses est une nécessité primordiale. Autrement, les produits de condensation seraient remis en circulation par les mécanismes de la réabsorption tubulaire rénale avec le risque d'une libération d'α-dicarbonyles après une autre réaction métabolique. Du point de vue du métabolisme par l'ornithine décarboxylase, les composés découverts par les inventeurs de la présente demande peuvent servir d'agents plus efficaces que le DAPA pour piéger les composés α-dicarbonyles réactifs tels que le méthylglyoxal, le glyoxal, et la 3-désoxyglucosone, en formant des adduits destinés à être éliminés dans l'urine. En effet, le DAPA empêche la modification de l'insuline par MG tel que ceci est illustré sur la figure 1. Cependant, la figure 7 met en évidence que sa cytotoxicité est supérieure et que son efficacité de protection des cellules est inférieure (survie de 68 % des cellules lorsqu'elles sont incubées avec le MG) comparé au L-DAPA-L-Val (93 %), au L-DAPA-L-Leu (81 %) et au L-DAPA-L-Ile (79 %), composés selon la présente invention. Par ailleurs, le DAPA semble être mutagène.

La présente invention concerne donc un composé de formule générale I suivante 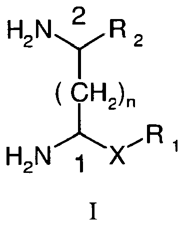 dans laquelle :
X représente CH₂, C=O, C=S ou CHOH, R₁ représente un acide aminé, éventuellement substitué par un ou plusieurs atomes d'halogène, avantageusement de fluor, ou par un ou plusieurs groupes CF₃, et n = 0,1 ou 2
ou XR₁ représentent PO₃H ou SO₃H et n = 0, 1 ou 2;
R₂ représente H, XR1, un groupe alkyle en C₁-C₆ un groupe aralkyle en C₁-C₆ ou un groupe aryle, les groupes alkyles, aralkyles et aryles pouvant être substitués par une amine NH₂, un groupe carboxylique COOH, un ou plusieurs atomes d'halogène, avantageusement de fluor, ou un ou plusieurs groupes CF₃ ;
ou ses sels d'addition pharmaceutiquement acceptables, isomères, énantiomères, diastéréoisomères, ainsi que leurs mélanges,
à l'exception des composés
   - pour lesquels R₂ représente un atome d'hydrogène, X représente C=O, R₁ représente -NH-(CH₂)ₘCOOH et m = 1, 2 ou 3 et n = 0,1 ou 2 ;
   - représentés par les formules suivantes : 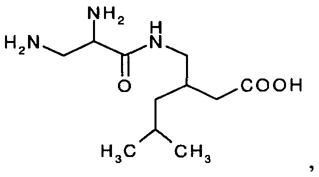 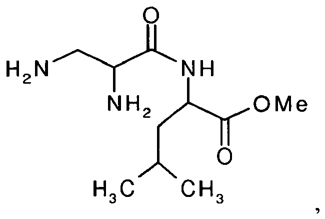 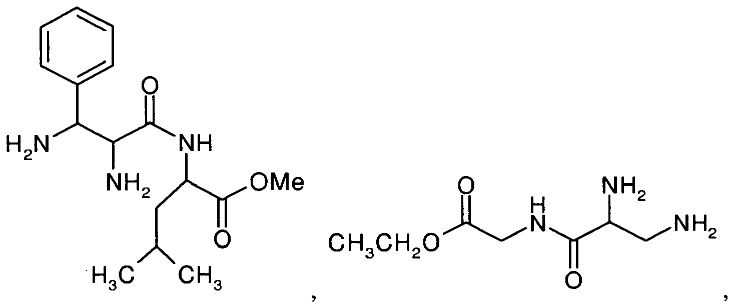 et des composés L-ornithyl-taurine, L-diaminobutyryl-taurine et L-diaminopropionyl taurine.

Par le terme « groupe alkyle en C₁-C₆», on entend au sens de la présente invention tout groupe alkyle de 1 à 6 atomes de carbones, linéaires ou ramifiés. En particulier, il peut s'agir du groupe méthyle, éthyle, n-propyle, iso-propyle, n-butyle, iso-butyle, sec-butyle, t-butyle, n-pentyle et n-hexyle.

Par le terme « groupe aryle », on entend au sens de la présente invention un ou plusieurs cycles aromatiques ayant 5 à 8 atomes de carbones, pouvant être accolés ou fusionnés. En particulier, les groupes aryles peuvent être des groupes phényle ou naphtyle, avantageusement phényle.

Par le terme « groupe aralkyle », on entend au sens de la présente invention tout groupe aryle tel que défini ci-dessus, lié par l'intermédiaire d'un groupe alkyle tel que défini ci-dessus. En particulier un groupe aralkyle est un groupe benzyle.

Par le terme de « sels d'addition pharmaceutiquement acceptables » d'un composé, on entend au sens de la présente invention tout sel qui est pharmaceutiquement acceptable et qui possèdent l'activité pharmacologique souhaitée du composé parent. De tels sels comprennent :
(1) les sels d'addition d'acide formés avec des acides inorganiques tels que l'acide chlorhydrique, l'acide bromhydrique, l'acide sulfurique, l'acide nitrique, l'acide phosphorique et similaires ; ou formés avec des acides organiques tels que l'acide acétique, l'acide benzènesulfonique, l'acide benzoïque, l'acide camphresulfonique, l'acide citrique, l'acide éthane-sulfonique, l'acide fumarique, l'acide glucoheptonique, l'acide gluconique, l'acide glutamique, l'acide glycolique, l'acide hydroxynaphtoïque, l'acide 2-hydroxyéthanesulfonique, l'acide lactique, l'acide maléique, l'acide malique, l'acide mandélique, l'acide méthanesulfonique, l'acide muconique, l'acide 2-naphtalènesulfonique, l'acide propionique, l'acide salicylique, l'acide succinique, l'acide dibenzoyl-L-tartrique, l'acide tartrique, l'acide p-toluènesulfonique, l'acide triméthylacétique, l'acide trifluoroacétique et similaires ; ou
(2) les sels formés lorsqu'un proton acide présent dans le composé parent soit est remplacé par un ion métallique, par exemple un ion de métal alcalin, un ion de métal alcalino-terreux ou un ion d'aluminium ; soit se coordonne avec une base organique ou inorganique. Les bases organiques acceptables comprennent la diéthanolamine, l'éthanolamine, N-méthylglucamine, la triéthanolamine, la trométhamine et similaires. Les bases inorganiques acceptables comprennent l'hydroxyde d'aluminium, l'hydroxyde de calcium, l'hydroxyde de potassium, le carbonate de sodium et l'hydroxyde de sodium.

Les sels pharmaceutiquement acceptables avantageux sont les sels formés à partir d'acide chlorhydrique, d'acide trifluoroacétique, d'acide dibenzoyl-L-tartrique et d'acide phosphorique.

Il devrait être compris que toutes les références aux sels pharmaceutiquement acceptables comprennent les formes d'addition de solvants (solvates) ou les formes cristallines (polymorphes) tels que définis ici, du même sel d'addition d'acide.

La stéréochimie de la position C-1 de la formule I (l'atome de carbone à la jonction des groupes NH₂ et X) peut être *R* ou *S* ou le mélange de ceux-ci. La stéréochimie de la position C-2 (l'atome de carbone à la jonction NH₂ et R₂) peut être *R* ou *S* ou le mélange de ceux-ci.

On entend par « acide aminé », au sens de la présente invention, tous les résidus des acides α-aminés naturels (par exemple Alanine (Ala), Arginine (Arg), Asparagine (Asn), Acide aspartique (Asp), Cystéine (Cys), Glutamine (Gln), Acide glutamique (Glu), Glycine (Gly), Histidine (His), Isoleucine (Ile), Leucine (Leu), Lysine (Lys), Méthionine (Met), Phénylalanine (Phe), Proline (Pro), Sérine (Ser), Thréonine (Thr), Tryptophane (Trp), Tyrosine (Tyr) et Valine (Val)) sous la forme D ou L, ainsi que les acides aminés non naturels (par exemple, la β-alanine, l'allylglycine, la tert-leucine, la Norleucine (Nle), l'acide 3-amino-adipique, l'acide 2-aminobenzoïque, l'acide 3-aminobenzoïque, l'acide 4-aminobenzoïque, l'acide 2-aminobutanoïque, la 4-amino-1-carboxyméthyl pipéridine, l'acide 1-amino-1-cyclobutanecarboxylique, l'acide 4-aminocyclohexaneacétique, l'acide 1-amino-1-cyclohexanecarboxyilique, l'acide (1R,2R)-2-aminocyclohexanecarboxylique, l'acide (1R,2S)-2-aminocyclohexanecarboxylique, l'acide (1S,2R)-2-aminocyclohexanecarboxylique, l'acide (1S,2S)-2-aminocyclohexanecarboxylique, l'acide 3-aminocyclohexanecarboxylique, l'acide 4-aminocyclohexanecarboxylique, l'acide (1R,2R)-2-aminocyclopentanecarboxylique, l'acide (1R,2S)-2-aminocyclopentanecarboxyilique l'acide 1-amino-1-cyclopentanecarboxylique, l'acide 1-amino-1-cyclopropanecarboxylique, l'acide 4-(2-aminoéthoxy)-benzoïque, l'acide 3-aminométhylbenzoïque, l'acide 4-aminométhylbenzoïque, l'acide 2-aminobutanoïque, l'acide 4-aminobutanoïque, l'acide 6-aminohexanoïque, l'acide 1-aminoindane-1-carboxylique, l'acide 4-aminométhyl-phénylacétique, l'acide 4-aminophénylacétique, l'acide 3-amino-2-naphtoïque, l'acide 4-aminophénylbutanoïque, l'acide 4-amino-5-(3-indolyl)-pentanoïque, l'acide (4R,5S)-4-amino-5-méthylheptanoïque, l'acide (R)-4-amino-5-méthylhexanoïque, l'acide (R)-4-amino-6-méthylthiohexanoïque, l'acide (S)-4-amino-pentanoïque, l'acide (R)-4-amino-5-phénylpentanoïque, l'acide 4-aminophénylpropionique, l'acide (R)-4-aminopimérique, l'acide (4R,5R)-4-amino-5-hyroxyhexanoïque, l'acide (R)-4-amino-5-hydroxypentanoïque, l'acide (R)-4-amino-5-(p-hydroxyphényl)-pentanoïque, l'acide 8-aminooctanoïque, l'acide (2S,4R)-4-amino-pyrrolidine-2-carboxylique, l'acide (2S,4S)-4-amino-pyrrolidine-2-carboxylique, l'acide azétidine-2-carboxylique, l'acide (2S,4R)-4-benzyl-pyrrolidine-2-carboxylique, l'acide (S)-4,8-diaminooctanoïque, l'acide tert-butylglycine, le γ-carboxyglutamate, la β-cyclohexylalanine, la citruline, l'acide 2,3-diamino propionique, l'acide hippurique, l'homocyclohexylalanine, la moleucine, l'homophénylalanine, la 4-hydroxyproline, l'acide indoline-2-carboxylique, l'acide isonipécotique, l'α-méthyl-alanine, l'acide nicopetique, la norvaline, l'acide octahydroindole-2-carboxylique, l'omithine, la pénicillamine, la phénylglycine (Phg), l'acide 4-phényl-pyrrolidine-2-carboxylique, l'acide pipécolique, la propargylglycine, la 3-pyridinylalanine, la 4-pyridinylalanine, l'acide 1-pyrrolidine-3-carboxylique, la sarcosine, les statines, l'acide tétrahydroisoquinoline-1-carboxylique, l'acide 1,2,3,4-tétrahydroisoquinoline-3-carboxylique, l'acide tranexamique, la 4,4-difluoro proline, la 4-fluoro proline, la alpha-(3,4-difluorobenzyl)-proline, la gamma-(3,4-difluorobenzyl)-proline, la alpha-(trifluorométhyl)phenylalanine, la hexafluoroleucine, la 5,5,5-trifluoroleucine, la 6,6,6-trifluoronorleucine, la 2-(trifluorométhyl)leucine, la 2-(trifluorométhyl)norleucine, la 4,4,4-trifluorovaline, la 4,4,4,4',4',4'-hexafluorovaline, la pentafluorophenylalanine, la 2,3-difluorophenylalanine, la 2,4-difluorophenylalanine, la 2,5-difluorophenylalanine, la 2,6-difluorophenylalanine, la 3,4-difluorophenylalanine, la 3,5-difluorophenylalanine, la 3,3-difluoro-3-(4-fluorophenyl)alanine, la 2,3-difluorophenylglycine, la 2,4-difluorophenylglycine, la 2,5-difluorophenylglycine, la 3,4-difluorophenylglycine, la 4,4-difluoroéthylglycine, la 4,4,4-trifluoroéthylglycine, l'hexafluoronorleucine). Le terme comprend aussi les acides aminés naturels et non naturels portant un groupe amino-protecteur conventionnel (par exemple un groupe acétyle, *tert-*butyloxycarbonyle, benzyloxycarbonyle ou 9-fluorénylméthylcarbonyle), ainsi que les acides aminés naturels et non naturels protégés à l'extrémité carboxylique (avantageusement par un groupe alkyle en (C₁-C₁₈), un ester, une amide phénylique ou benzylique ou une amide, ce qui donne respectivement des extrémité carboxylique de formule suivante :-CO(alkyle en C₁-C₁₈), -COO(alkyle en C₁-C₁₈), -CONHphényle CONHbenzyle ou CONH₂). Avantageusement l'acide aminé selon la présente invention a son extrémité carboxylique non protégée.

De façon avantageuse l'acide aminé selon la présente invention a son extrémité carboxylique protégée sous forme d'un ester d'alkyle en (C₁-C₁₈) (-COO(alkyle en C₁-C₁₈)), de préférence comme un ester d'alkyle en C₁₃-C₁₈ (-COO(alkyle en C₁₃-C₁₈)).

Avantageusement l'acide aminé est lié au radical X du composé de formule I par l'extrémité N-terminale. De façon avantageuse la liaison ainsi formé est la suivante : -X-NH-R, dans lequel R représente le reste de la molécule de l'acide aminé.

Avantageusement, l'acide aminé selon la présente invention est substitué par un ou plusieurs atomes d'halogène (Br, Cl, I ou F), avantageusement de fluor, ou un ou plusieurs groupes CF₃. Avantageusement cette substitution est présente sur la partie alkyle ou aryle de l'acide aminé. De façon encore plus avantageuse l'atome d'azote n'est pas substitué. L'avantage principal de la substitution par un atome d'halogène, en particulier par un atome de fluor, ou par un groupe CF₃ concerne la biodisponibilité des composés obtenus et en particulier l'amélioration de leur propriété de perméation et de liaisons avec les membranes des cellules.

Avantageusement l'acide aminé est choisi parmi alanine, valine, isoleucine, proline, leucine, phénylalanine, glycine, β-alanine, norleucine, acide aspartique, lysine, ou tert-leucine, de façon avantageuse parmi alanine, valine, isoleucine, proline, phénylalanine, leucine, norleucine ou tert-leucine.

Avantageusement, le radical phényle de la phénylalanine est substitué par un ou plusieurs atomes d'halogène, avantageusement de fluor ou par un ou plusieurs groupes CF₃, avantageusement en position para, moins avantageusement en position ortho ou méta.

De façon avantageuse, le radical butyle de la norleucine est substitué par un ou plusieurs atomes d'halogène, avantageusement de fluor ou par un ou plusieurs groupes CF₃.

Par le terme « groupe alkyle en C₁-C₁₈», on entend au sens de la présente invention tout groupe alkyle de 1 à 18 atomes de carbones, linéaires ou ramifiés. En particulier, il peut s'agir du groupe méthyle, éthyle, n-propyle, iso-propyle, n-butyle, iso-butyle, sec-butyle, t-butyle, n-pentyle et n-hexyle.

Avantageusement le composé selon la présente invention est tel que X représente C=O, CH₂ ou C=S.

De façon avantageuse, le composé selon la présente invention est tel que R₂ représente H ou XR₁, avantageusement XR₁.

Dans un mode de réalisation particulier, le composé selon la présente invention est tel que R₁ représente un acide aminé, avantageusement choisi parmi alanine, valine, isoleucine, proline, leucine, Norleucine, phénylalanine ou tert-leucine.

Avantageusement, le composé selon la présente invention est tel que X est C=O, n = 0, et R₁ est l'alanine, la valine, la leucine, l'isoleucine, la proline, la Norleucine, la phénylalanine ou la *tert*-leucine.

Avantageusement, le composé selon la présente invention est tel que R₂ est XR₁ ou H et n = 0.

Dans un autre mode de réalisation particulier de l'invention, le composé selon la présente invention est représenté par la formule générale II suivante : 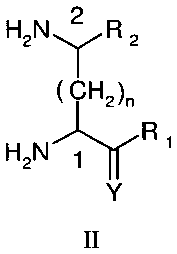 dans laquelle :
R₁ représente -NH-R₃-(C=O)R₄ ou 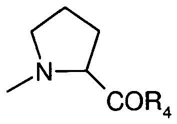 dans lesquelles
R₃ représente
   - un groupe alkyle en C₁-C₁₂, avantageusement en C₁-C₆, éventuellement substitué par un ou plusieurs groupes choisis parmi un atome d'halogène, avantageusement de fluor, un groupe -CF₃, phényle, phénol, -COOH, amine ou phényle substitué par un ou plusieurs atomes d'halogène, avantageusement de fluor, ou par un ou plusieurs groupes CF₃ ;
   - un groupe phényle, éventuellement substitué par une amine, un groupe OH, un ou plusieurs atomes d'halogène, avantageusement de fluor, ou un ou plusieurs groupes CF₃ et
R₄ représente OH, NH₂, un alcoxy en C₁-C₃₀, avantageusement en C₁-C₂₀ ;
R₂ représente H, COR₁, ou un groupe alkyle en C₁-C₆ éventuellement substitué par un ou plusieurs atomes d'halogène, avantageusement de fluor, ou par un ou plusieurs groupes CF₃;
n=0, 1 ou 2 ;
Y représente un atome d'oxygène ou de soufre, avantageusement un atome d'oxygène;
ou ses sels d'addition pharmaceutiquement acceptables, isomères, énantiomères, diastéréoisomères, ainsi que leurs mélanges.

Par le terme « groupe alkyle en C₁-C₁₂», on entend au sens de la présente invention tout groupe alkyle de 1 à 12 atomes de carbones, linéaires ou ramifiés. En particulier, il peut s'agir du groupe méthyle, éthyle, n-propyle, iso-propyle, n-butyle, iso-butyle, sec-butyle, t-butyle, n-pentyle et n-hexyle.

Par le terme « alcoxy en C₁-C₃₀ » On entend tout radical-O-R, dans lequel R est un radical alkyle en C₁-C₃₀ tel que défini ici. Les exemples de radicaux alcoxy comprennent, mais ne sont pas limités à, méthoxy, éthoxy, isopropoxy et similaires.

Avantageusement le composé selon la présente invention est tel que n = 0.

De façon avantageuse R₂ = H ou COR₁.

Dans un mode de réalisation particulier, le composé selon la présente invention est choisi parmi : 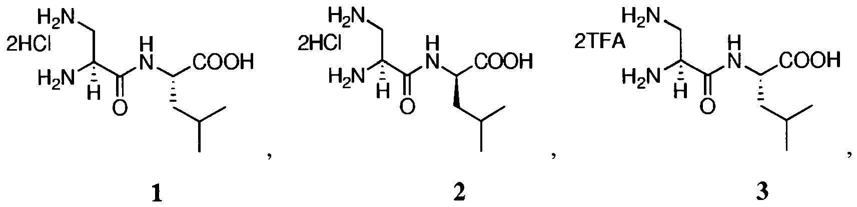 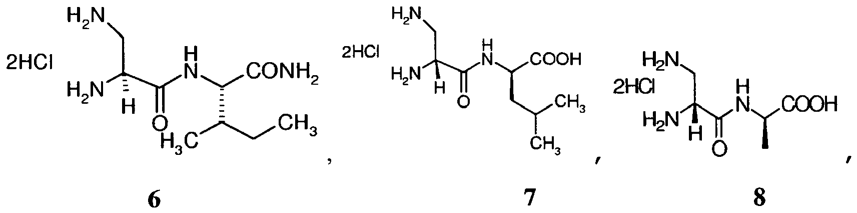 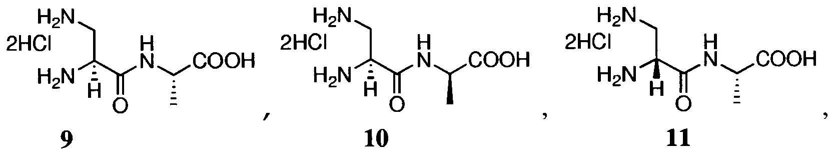 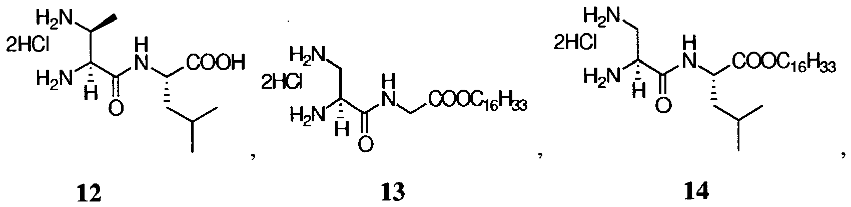 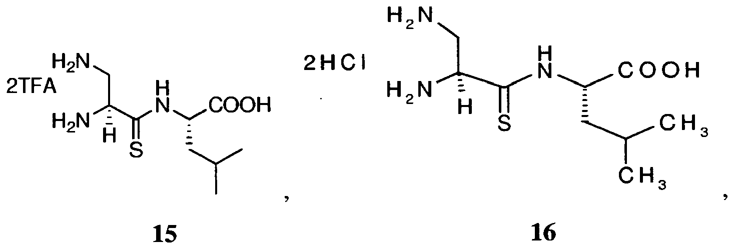 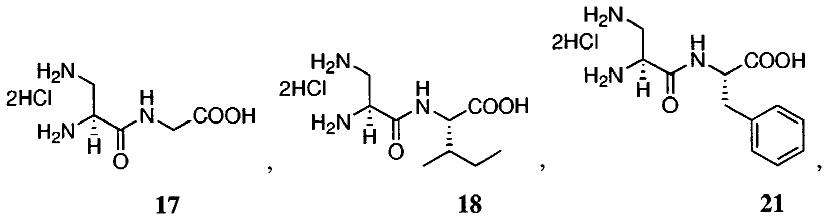 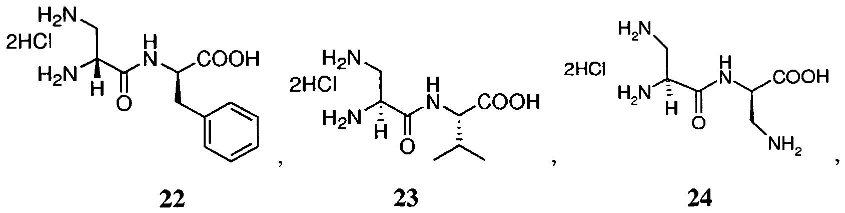 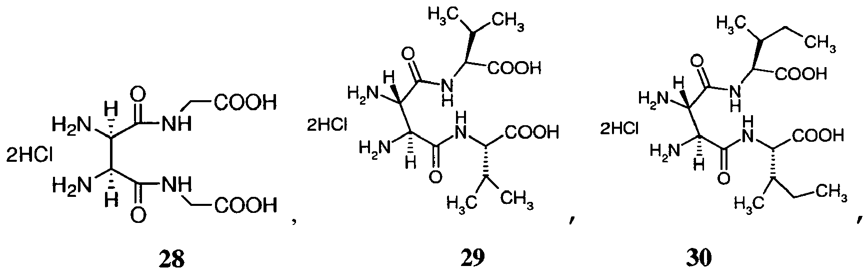 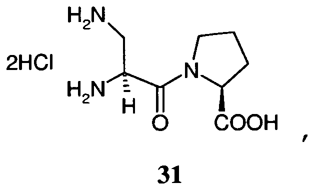 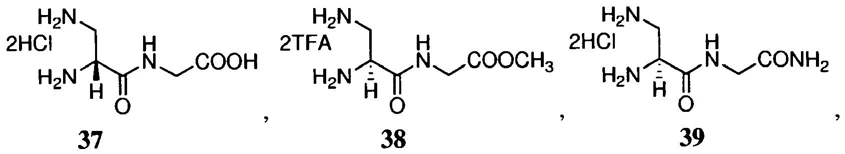 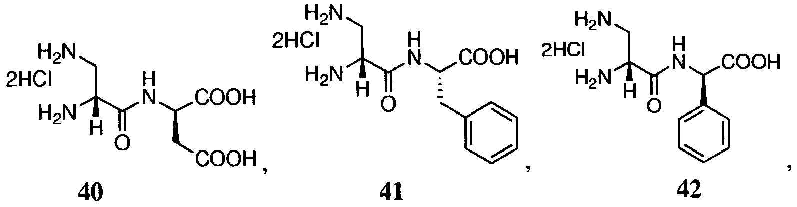 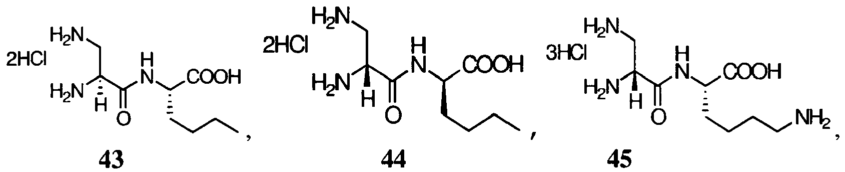 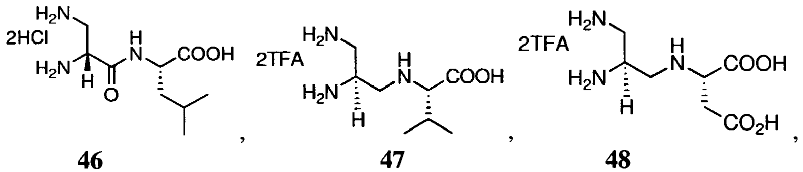 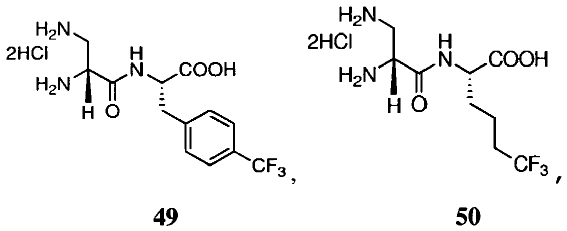 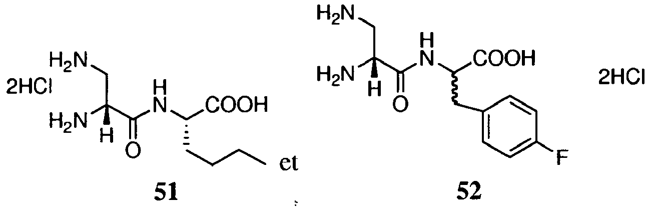 ou ses sels d'addition pharmaceutiquement acceptables, isomères, énantiomères, diastéréoisomères, ainsi que leurs mélanges.

La présente invention concerne en outre l'utilisation d'un composé selon la présente invention pour empêcher l'altération de protéines dans les aliments.

Les aliments peuvent être d'origine animale ou végétale. Les composés selon la présente invention sont administrés en une quantité efficace dans les aliments afin de prévenir l'altération et la dégradation des protéines contenues dans ces aliments. Ceci permet d'augmenter la durée de consommation et de stockage des aliments et de préserver leurs qualités nutritionnelles et organoleptiques.

De plus, la présente invention concerne une composition pharmaceutique ou cosmétique comprenant un composé selon la présente invention et un excipient pharmaceutiquement ou cosmétiquement acceptable.

La présente invention concerne un composé de formule générale I suivante 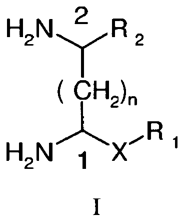 dans laquelle :
X représente CH₂, C=O, C=S ou CHOH, R₁ représente un acide aminé, éventuellement substitué par un ou plusieurs atomes d'halogène, avantageusement de fluor, ou par un ou plusieurs groupes CF₃, et n = 0, 1 ou 2
ou XR₁ représentent PO₃H ou SO₃H et n = 0, 1 ou 2;
R₂ représente H, XR1, un groupe alkyle en C₁-C₆ un groupe aralkyle en C₁-C₆ ou un groupe aryle, les groupes alkyles, aralkyles et aryles pouvant être substitués par une amine NH₂, un groupe carboxylique COOH, un ou plusieurs atomes d'halogène, avantageusement de fluor, ou un ou plusieurs groupes CF₃ ;
ou ses sels d'addition pharmaceutiquement acceptables, isomères, énantiomères, diastéréoisomères, ainsi que leurs mélanges,
et à l'exception du composé 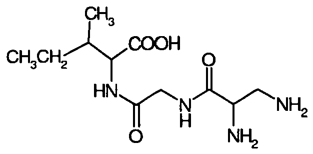 à titre de médicament.

Avantageusement, le composé selon la présente invention à titre de médicament est représenté par la formule générale II suivante 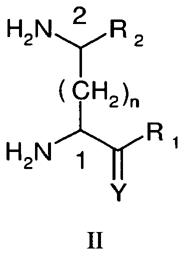 dans laquelle :
R₁ représente NH-R₃-(C=O)R₄ ou 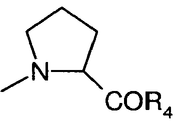 dans lesquelles
R₃ représente
   - un groupe alkyle en C₁-C₁₂, avantageusement en C₁-C₆, éventuellement substitué par un ou plusieurs groupes choisis parmi un atome d'halogène, avantageusement de fluor, un groupe -CF₃, phényle, phénol, -COOH, amine ou phényle substitué par un ou plusieurs atomes d'halogène, avantageusement de fluor, ou par un ou plusieurs groupes CF₃ ;
   - un groupe phényle, éventuellement substitué par une amine, un groupe OH, un ou plusieurs atomes d'halogène, avantageusement de fluor, ou un ou plusieurs groupes CF₃ et
R₄ représente OH, NH₂, un alcoxy en C₁-C₃₀, avantageusement en C₁-C₂₀ ;
R₂ représente H, COR₁, ou un groupe alkyle en C₁-C₆ éventuellement substitué par un ou plusieurs atomes d'halogène, avantageusement de fluor, ou par un ou plusieurs groupes CF₃;
n=0, 1 ou 2 ;
Y représente un atome d'oxygène ou de soufre, avantageusement un atome d'oxygène;
ou ses sels d'addition pharmaceutiquement acceptables, isomères, énantiomères, diastéréoisomères, ainsi que leurs mélanges.

De façon avantageuse, il est choisi parmi 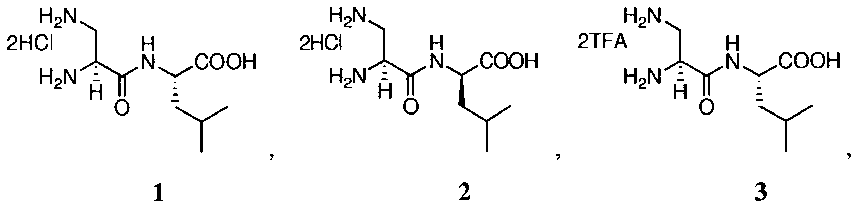 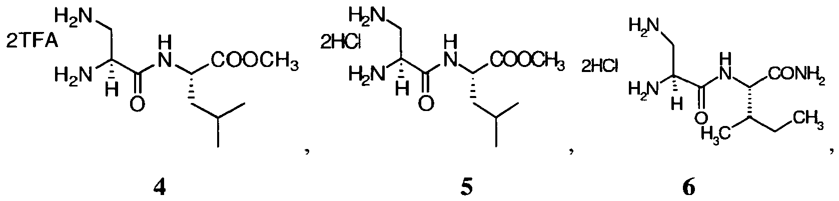 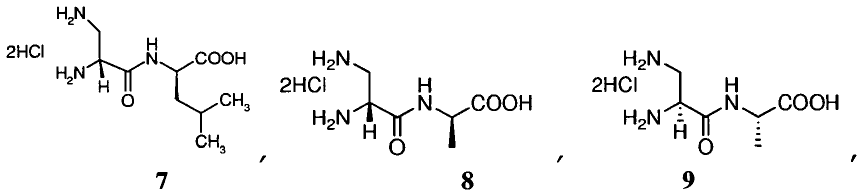 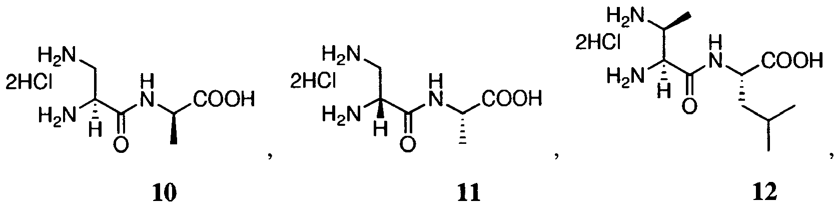 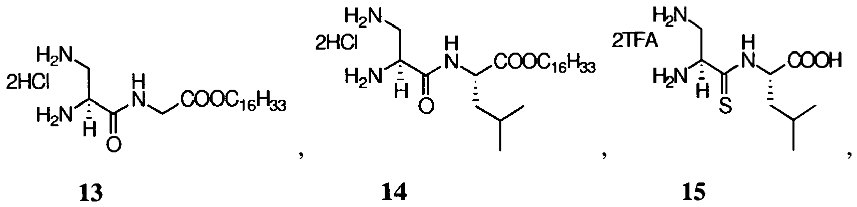 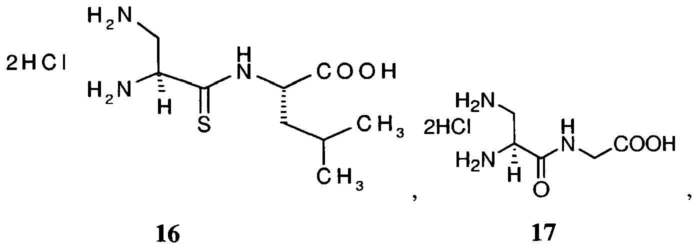 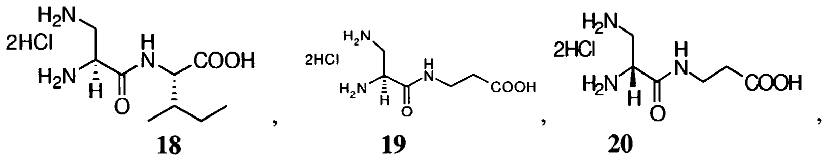 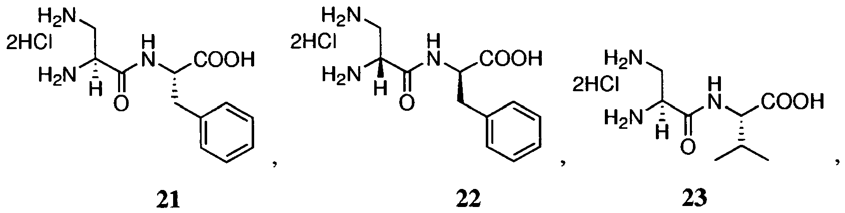 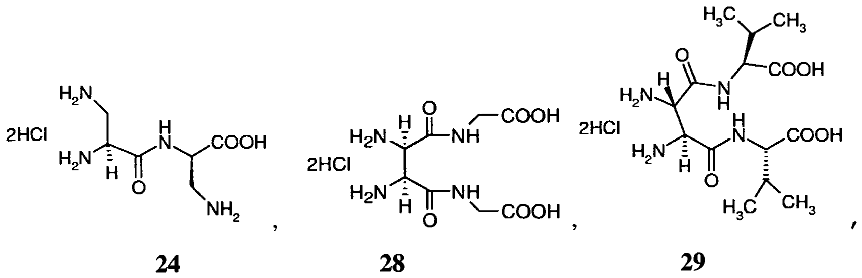 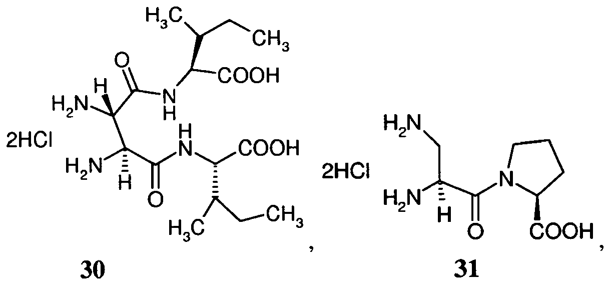 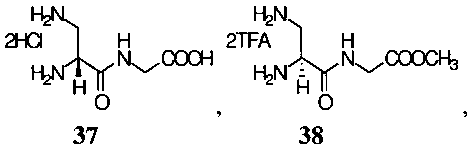 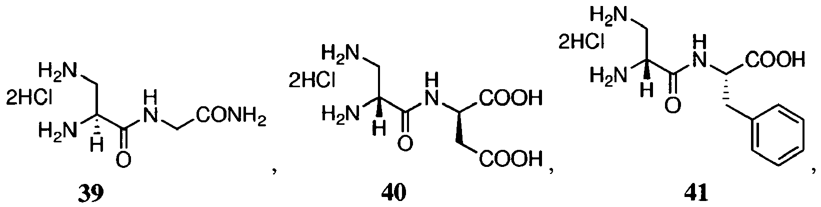 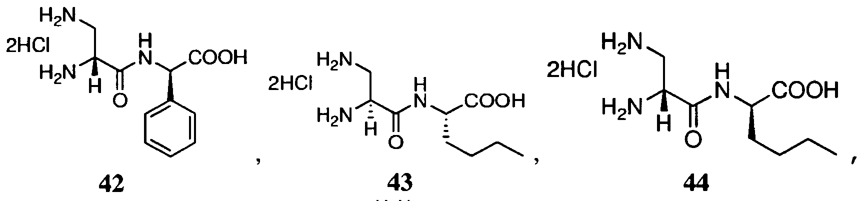 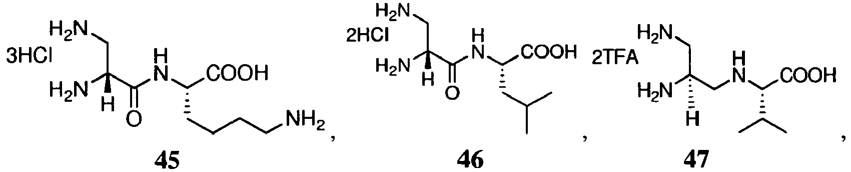 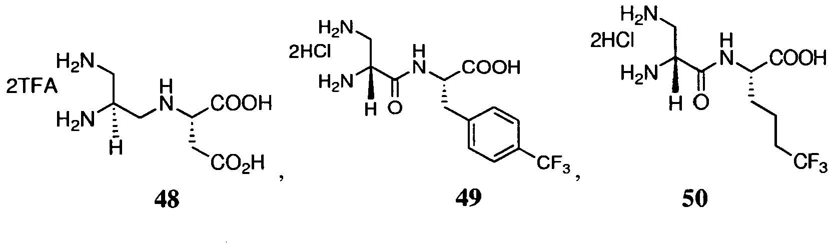 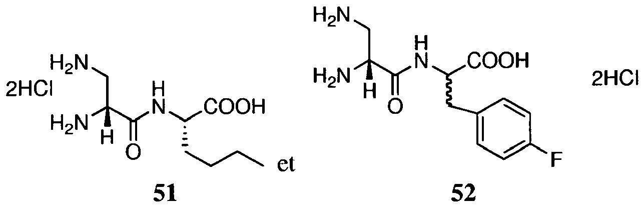 ou ses sels d'addition pharmaceutiquement acceptables, isomères, énantiomères, diastéréoisomères, ainsi que leurs mélanges.

Dans un mode de réalisation particulier, le médicament selon la présente invention est un piégeur de composés carbonyle réactifs, avantageusement un inhibiteur de formation de produits de glycation avancée.

De façon avantageuse le médicament selon la présente invention est destiné à la prévention et/ou au traitement d'un état ou de maladies du à la formation de produits de glycation avancée ou à la réticulation de protéines, à la prévention et/ou au traitement des effets délétères du vieillissement d'un organisme, lesdits effets étant la formation de produits de glycation avancée ou la réticulation de protéines, ou au ralentissement ou à l'arrêt de la progression chez un patient de complications résultant d'un diabète, lesdites complications résultant de la formation de produits de glycation avancée ou de la réticulation de protéines.

Avantageusement, le médicament selon la présente invention est destiné à traiter, prévenir et/ou ralentir la progression chez un patient de maladies choisies parmi la polyarthrite rhumatoïde, la maladie d'Alzheimer, l'urémie, les maladies neurodégénératives, l'athérosclérose, les complications micro et macrovasculaires du diabète dont la rétinopathie diabétique et l'insuffisance rénale due à la néphropathie diabétique, les micro et macroangiopathies, la cataracte, l'amyloïdose associée à la dialyse ou à la maladie d'Alzheimer, la maladie de Parkinson, les gingivites, les caries, les problèmes bucco-dentaires, l'ulcère diabétique, l'insuffisance rénale chronique, la dialyse rénale chronique, les maladies inflammatoires, les troubles rhumatismaux liés à l'âge et la porphyrie et à traiter les cancers de stade précoce.

De façon encore plus avantageuse, le médicament selon la présente invention est destiné à une administration par voie orale.

La présente invention concerne de plus l'utilisation d'un composé de formule générale I ou II telles que définies ci-dessus pour la préparation d'un médicament piégeur de composés carbonyle réactifs, avantageusement un inhibiteur de formation de produits de glycation avancée et avantageusement destiné à
- la prévention et/ou au traitement d'un état ou de maladies du à la formation de produits de glycation avancée ou à la réticulation de protéines, à la prévention et/ou au traitement des effets délétères du vieillissement d'un organisme, lesdits effets étant la formation de produits de glycation avancée ou la réticulation de protéines, ou au ralentissement ou à l'arrêt de la progression chez un patient de complications résultant d'un diabète, lesdites complications résultant de la formation de produits de glycation avancée ou de la réticulation de protéines ;
- traiter, prévenir et/ou ralentir la progression chez un patient de maladies choisies parmi la polyarthrite rhumatoïde, la maladie d'Alzheimer, l'urémie, les maladies neurodégénératives, l'athérosclérose, les complications micro et macrovasculaires du diabète dont la rétinopathie diabétique et l'insuffisance rénale due à la néphropathie diabétique, les micro et macroangiopathies, la cataracte, l'amyloïdose associée à la dialyse ou à la maladie d'Alzheimer, la maladie de Parkinson, les gingivites, les caries, les problèmes bucco-dentaires, l'ulcère diabétique, l'insuffisance rénale chronique, la dialyse rénale chronique, les maladies inflammatoires, les troubles rhumatismaux liés à l'âge et la porphyrie et à traiter les cancers de stade précoce.

La présente invention concerne de plus une méthode de prévention et/ou de traitement d'un état ou de maladies du à la formation de produits de glycation avancée ou à la réticulation de protéines, de prévention et/ou traitement des effets délétères du vieillissement d'un organisme, lesdits effets étant la formation de produits de glycation avancée ou la réticulation de protéines, de ralentissement ou d'arrêt de la progression chez un patient de complications résultant d'un diabète, lesdites complications résultant de la formation de produits de glycation avancée ou de la réticulation de protéines de traitement, de prévention et/ou de ralentissement de la progression chez un patient de maladies choisies parmi la polyarthrite rhumatoïde, la maladie d'Alzheimer, l'urémie, les maladies neurodégénératives, l'athérosclérose, les complications micro et macrovasculaires du diabète dont la rétinopathie diabétique et l'insuffisance rénale due à la néphropathie diabétique, les micro et macroangiopathies, la cataracte, l'amyloïdose associée à la dialyse ou à la maladie d'Alzheimer, la maladie de Parkinson, les gingivites, les caries, les problèmes bucco-dentaires, l'ulcère diabétique, l'insuffisance rénale chronique, la dialyse rénale chronique, les maladies inflammatoires, les troubles rhumatismaux liés à l'âge et la porphyrie, et à traiter les cancers de stade précoce,
ladite méthode comprenant l'administration à un patient ayant besoin d'un tel traitement d'une quantité efficace d'un composé de formule générale I ou II selon la présente invention et telles que définies ci-dessus.

La présente invention concerne donc un médicament ou une composition pharmaceutique comprenant un composé selon la présente invention.

Ces compositions ou médicaments peuvent être formulés pour l'administration aux mammifères, y compris l'homme. La posologie varie selon le traitement et selon l'affection en cause. Ces compositions ou médicaments sont réalisés de façon à pouvoir être administrées par la voie digestive ou parentérale.

Dans les compositions pharmaceutiques ou médicaments de la présente invention pour l'administration orale, sublinguale, sous-cutanée, intramusculaire, intraveineuse, transdermique, locale ou rectale, l'ingrédient actif peut être administré sous formes unitaires d'administration, en mélange avec des supports pharmaceutiques classiques, aux animaux ou aux êtres humains. Les formes unitaires d'administration appropriés comprennent les formes par voie orale telles que les comprimés, les gélules, les poudres, les granules et les solutions ou suspensions orales, les formes d'administration sublinguale et buccale, les formes d'administration sous-cutanée, intramusculaire, intraveineuse, intranasale ou intraoculaire et les formes d'administration rectale.

Lorsque l'on prépare une composition ou un médicament solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, un antiseptique, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, de même qu'avec des correcteurs du goût ou des édulcorants.

Pour une administration rectale, on recourt à des suppositoires qui sont préparés avec des liants fondant à la température rectale, par exemple du beurre de cacao ou des polyéthylène glycols.

Pour une administration parentérale, intranasale ou intraoculaire, on utilise des suspensions aqueuses, des solutions salines isotoniques ou des solutions stériles et injectables qui contiennent des agents de dispersion et/ou des agents mouillants pharmacologiquement compatibles.

Le principe actif peut être formulé également sous forme de microcapsules, éventuellement avec un ou plusieurs supports additifs.

Le principe actif peut également être administré par voie topique.

La présente invention concerne en outre l'utilisation cosmétique d'un composé selon la présente invention en tant qu'actif antivieillissement et restructurant de l'épiderme et du derme papillaire et/ou en tant qu'actif antirides.

Les composés selon la présente invention ont un effet tenseur sur la peau. Ils peuvent être administrés par voie orale ou topique.

Les compositions cosmétiques ou pharmaceutiques selon la présente invention peuvent être formulée pour une administration par voie topique. Ils pourront se présenter sous les formes qui sont habituellement connues pour ce type d'administration, c'est à dire notamment les lotions, les mousses, les gels, les dispersions, les sprays, les shampoings, les sérums, les masques, les laits corporels ou les crèmes par exemple, avec des excipients permettant notamment une pénétration cutanée afin d'améliorer les propriétés et l'accessibilité du principe actif. Les formes peuvent être à véhicule monophasique constituées d'un gel neutre d'hydroxypropylcellulose ou d'un gel chargé formé de carboxyméthylcellulose de sodium. On peut également préparer des crèmes, formes à véhicule biphasique, comportant une phase hydrophile dispersée dans une phase lipophile.

Ces compositions ou médicaments contiennent généralement outre la composition selon la présente invention, un milieu physiologiquement acceptable, en général à base d'eau ou de solvant, par exemple des alcools, des éthers ou des glycols. Ils peuvent également contenir un excipient cosmétiquement ou pharmaceutiquement acceptable. Ces excipients peuvent être choisis parmi des composés présentant une bonne compatibilité avec ce principe actif. Il s'agit par exemple des polymères hydrosolubles de type polymère naturel, tels les polysaccharides (gomme xanthane, gomme de caroube, peptine...) ou polypeptides, des dérivés cellulosiques type méthylcellulose, hydroxypropylcellulose, hydroxypropyl-méthylcellulose ou encore des polymères synthétiques, polaxamers, carbomers, PVA ou PVP.

Enfin, il est à la portée de tout homme de l'art d'ajouter dans cette composition cosmétique ou pharmaceutique divers excipients type cosolvant comme l'éthanol, le glycérol, l'alcool benzylique, des humectants (glycérol), des agents facilitant la diffusion (transcurol, urée), ou encore des conservateurs anti-bactériens (p-hydroxybenzoate de méthyle à 0,15%). Elle peut également contenir des agents tensioactifs, des agents stabilisants, des émulsifiants, des épaississants, d'autres principes actifs conduisant à un effet complémentaire ou éventuellement synergique, des oligo-éléments, des huiles essentielles, des parfums, des colorants, du collagène, des filtres chimiques ou minéraux, des agents hydratants ou des eaux thermales.

La présente invention concerne également un procédé de traitement cosmétique du vieillissement de la peau par application d'une composition comprenant un composé selon la présente invention.

Les abréviations utilisées dans le cadre de la présente demande sont les suivantes: DAPA = acide 2,3-diaminopropionique, DABA = acide 2,3-diaminobutylique (sauf spécification contraire) ou acide 2,4-diaminobutylique, DASA = acide diaminosuccinique, EDC = chlorhydrate de 1-[3-(diméthylamino)propyl]-3-éthylcarbodiimide, HOBT = hydrate de 1-hydroxybenzotriazole, Boc = *t-*butoxycarbonyle, TFA = acide trifluoroacétique, THF = tétrahydrofurane.

La présente invention sera mieux comprise en référence aux figures dans lesquelles :

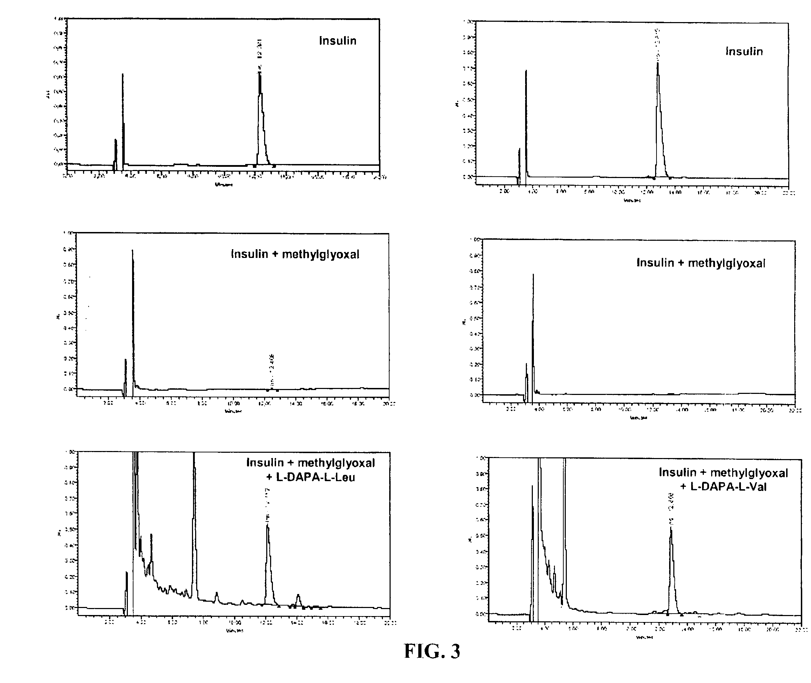

La figure 1 représente les effets comparatifs des piégeurs de dicarbonyle sous la forme de dichlorhydrate selon la présente invention et la comparaison avec le DAPA sur la modification de l'insuline (Ins) par le méthylglyoxal (MG). Le pourcentage d'insuline est indiqué après des incubations avec du méthylglyoxal en présence ou en l'absence de piégeurs de dicarbonyle. On a incubé de l'insuline (0,034 mM) *in vitro* dans un tampon phosphate 10 mM, pH 7,45 (contenant du NaCl 0,1 M) avec du méthylglyoxal (3,4 mM) en présence de piégeurs de dicarbonyle (4,08 mM) pendant 21 heures à 37°C. On a mesuré la concentration en insuline par CLHP (mêmes conditions que dans la figure 3).
La figure 2 représente les effets comparatifs de piégeurs de dicarbonyle connus dans l'art antérieur sur la modification de l'insuline par le méthylglyoxal. Les conditions expérimentales sont identiques à celles décrites sur la figure 1. Les composés marqués de * sont utilisés sous la forme de chlorhydrate.
La figure 3 représente les résultats obtenus par CLHP de deux composés selon la présente invention le L-DAPA-L-Leu (exemple 1) et le L-DAPA-L-Val (exemple 23) sur les modifications de l'insuline induites par le méthylglyoxal. On observe que ces modifications sont empêchées. Les conditions de CLHP sont les suivantes : colonne C-18, Symmetry 300 (4,6 x 250 mm), volume d'injection 100 µL de mélange réactionnel ; débit 1 mL/min ; température 40°C ; solvant A : H₂O + TFA 0,1 % ; solvant B : CH₃CN/H₂O 60/40 + TFA 0,1 % ; gradient linéaire de 50 % de B à 55 % de B en 15 minutes ; détection : UV à 215 nm : PDA (chromatogrammes extraits à 220 nm).

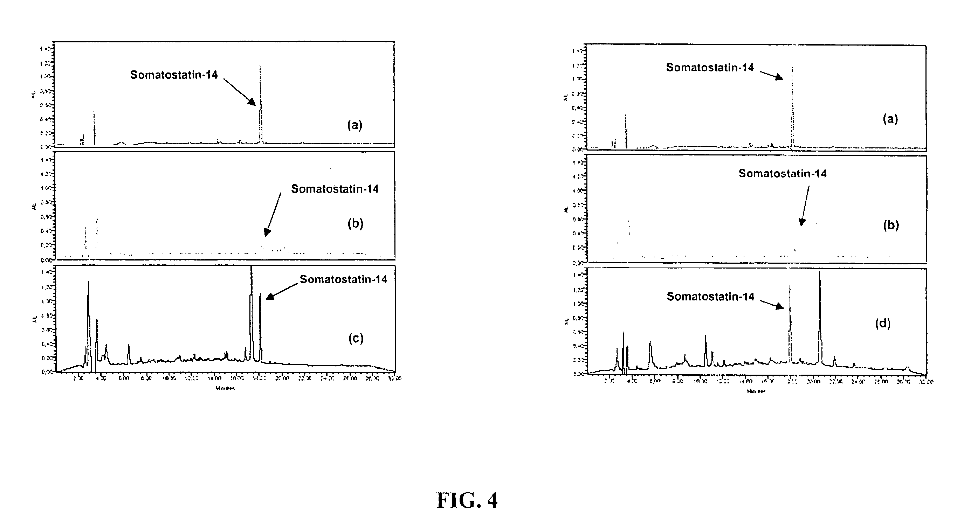

La figure 4 représente les résultats obtenus par CLHP de deux composés selon la présente invention le L-DAPA-L-Leu (exemple 1) et le L-DAPA-L-Val (exemple 23) sur les modifications de la somatostatine-14 induites par le méthylglyoxal. Les conditions de CLHP sont les suivante : colonne C-18, Symmetry 300 (4,6 x 250 mm), volume d'injection 100 µL de mélange réactionnel; débit 1 mL/min ; solvant A: H₂O + TFA 0,1% ; solvant B : CH₃CN[H₂O 80/20 + TFA 0,1 % ; gradient linéaire : de 20 % de B à 60 % de B en 15 minutes et en isocratique de 60 % de B durant 10 minutes ; température ambiante ; détection : PDA (chromatogrammes extraits à 215 nm). Les composés selon la présente invention empêchent les modifications induites par le méthylglyoxal.
   Dans cette figure, (a) représente les résultats de la somatostatine-14 seule; (b) représente les résultats de la somatostatine-14 + méthylglyoxal ; (c) représente les résultats de la somatostatine-14 + méthylglyoxal + L-DAPA-L-Val (exemple 23) et (d) représente les résultats de la somatostatine-14 + méthylglyoxal + L-DAPA-L-Leu (exemple 1). Pour obtenir ces résultats, on a incubé la somatostatine-14 (0,03 mM) *in vitro* dans un tampon phosphate 10 mM, pH 7,45 contenant du NaCl 0,1 M avec ou sans (a) méthylglyoxal (3,6 mM) en présence ((c) et (d)) ou en l'absence (b) de composés selon la présente invention (4,3 mM) pendant 24 heures à 37°C.

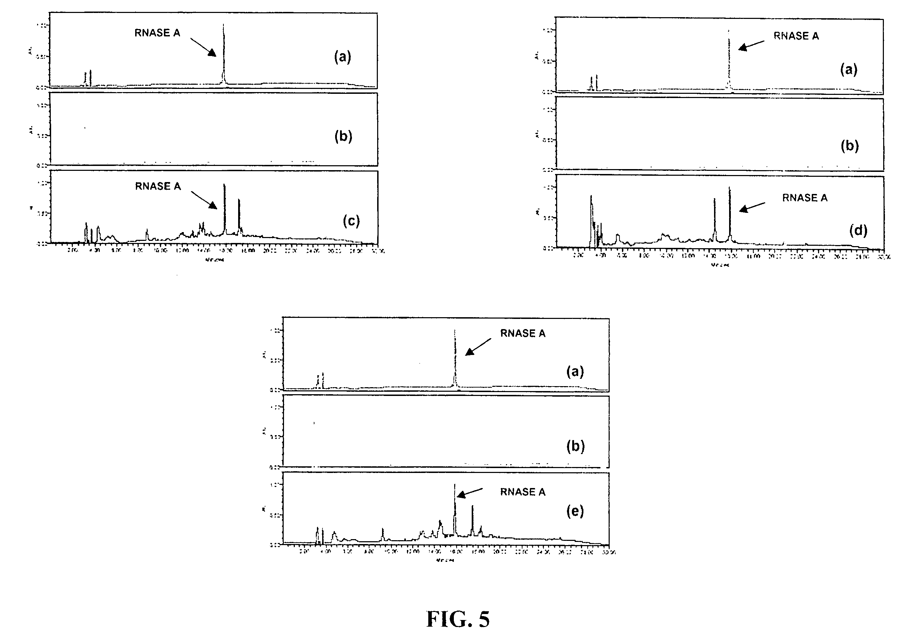

La figure 5 représente les résultats obtenus par CLHP de trois composés selon la présente invention L-DAPA-L-Ile (exemple 18), L-DAPA-L-Val (exemple 23) et L-DAPA-L-Leu (exemple 1) sur les modifications de la RNASE A induites par le méthylglyoxal. Les conditions de CLHP sont les suivantes : colonne C-18, Symmetry 300 (4,6 x 250 mm), volume d'injection 100 µL de mélange réactionnel dilué à 1/10 ; débit 1 mL/min ; température 40°C ; solvant A : H₂O + TFA 0,1 % ; solvant B : CH₃CN/H₂O 60/40 + TFA 0,1 % ; gradient linéaire de 20 % de B à 80 % de B en 20 minutes ; détection : UV à 215 nm : PDA (chromatogrammes extraits à 215 nm). Les composés selon la présente invention empêchent les modifications de la RNASE A. Dans cette figure : (a) représente les résultats de la RNASE A ; (b) représente les résultats de la RNASE A + méthylglyoxal; (c) représente les résultats de la RNASE A + méthylglyoxal + L-DAPA-L-Ile (exemple 18) ; (d) représente les résultats de la RNASE A + méthylglyoxal + L-DAPA-L-Val (exemple 23) ; (e) représente les résultats de la RNASE A + méthylglyoxal + L-DAPA-L-Leu (exemple 1). Pour obtenir ces résultats, on a incubé la RNASE A (0,08 mM) *in vitro* dans un tampon phosphate 100 mM, pH 7,45 avec ou sans (a) le méthylglyoxal (32 mM) en présence ((c), (d) et (e)) ou en l'absence (b) de composés selon la présente invention (38 mM) pendant 21 heures à 37°C.
Les figures 6 et 7 représente la croissance des cellules endothéliales EA en présence de composés selon la présente invention et de composés selon l'art antérieur, en particulier l'aminoguanidine (AG) et l'acide diaminopropionique (DAPA), en présence ou en absence de méthylglyoxal (MG).

La méthode générale de fabrication des composés selon la présente invention comprend l'étape (a) ou les étapes (a) et (b) ou (a), (b) et (c) ou (a) et (d) ou (a), (d) et (e) suivantes :
-a) Couplage d'un ester alkylique d'acide aminé ou de peptide sur un acide diaminé (par exemple, DAPA, DABA, DASA ou Orn, Lys selon la valeur de n) N-protégé dans un solvant organique, avantageusement le dichlorométhane, avantageusement en utilisant des réactifs formant un ester actif par exemple, EDC et HOBt, de façon avantageuse sous agitation à température ambiante ;
-b) Hydrolyse alcaline de l'ester alkylique obtenu à l'étape (a), avantageusement avec LiOH, avantageusement dans du solvant THF/MeOH/H₂O, MeOH/H₂O ou H₂O, puis acidification, avantageusement avec une solution aqueuse de KHSO₄, à pH 5 pour obtenir l'acide pur ;
-c) Déprotection des groupes N-protecteurs de l'acide obtenu à l'étape (b) avantageusement avec du 3M HCl-dioxane (ou THF) et élimination des composants volatils ;
-d) Préparation des thioamides par ajout du réactif de Lawesson au peptide obtenu à l'étape (a), avantageusement sous atmosphère inerte, et chauffage, avantageusement à 80°C pendant 2 heures ;
-e) Déprotection des thioamides à protection di-Boc, *tert*-butyl ester obtenu à l'étape (d) par ajout de TFA dans un solvant organique, avantageusement le dichlorométhane, à basse température, avantageusement 0°C.

Dans un mode de réalisation avantageux, les composés selon la présente invention peuvent être fabriqués selon le procédé décrit ci-après c'est à dire la mise en oeuvre de l'étape (1), ou des étapes (1) et (2), ou des étapes (1), (2) et (3) ou des étapes (1) et (4) ou des étapes (1), (4) et (5).

### 1. Réaction de couplage d'acides carboxyliques N-protégés et d'ester alkylique d'acides aminés.

On a ajouté, à une solution d'un acide diaminé (par exemple, DAPA, DABA, DASA, Orn, Lys) (1,0 mmol) correctement N-protégé (de préférence par un groupe Boc) et d'un ester alkylique d'acide aminé (1,1 mmoles) dans du dichlorométhane (5,0 mL), des réactifs formant un ester actif ((par exemple, EDC (1,2 mmoles) et HOBt (1,1 mmoles)) et on a agité le mélange réactionnel à température ambiante pendant une nuit. On a ajouté de l'eau et on a extrait la phase aqueuse avec de l'EtOAc. On a lavé les couches organiques combinées avec du HCl 1 N, H₂O, du NaHCO₃ saturé, de la saumure, successivement, on les a séchées sur du Na₂SO₄, puis filtrées. On a fait évaporer le solvant sous pression réduite puis on a purifié le résidu par chromatographie éclair sur colonne pour obtenir le dipeptide.

### 2. Hydrolyse alcaline de l'ester alkylique

On a ajouté, à une solution de l'ester alkylique (1,0 mmol) dans du THF/MeOH/H₂O ou MeOH/H₂O à température ambiante une solution alcaline (de préférence du LiOH (1,0 mmol)) puis on a agité le mélange réactionnel à température ambiante jusqu'à la disparition de la totalité de l'ester de départ (environ une nuit). On a acidifié le mélange réactionnel avec une solution aqueuse de KHSO₄ à pH 5, puis on l'a extrait avec un solvant organique (de préférence CH₂Cl₂). On a séché la phase organique (Na₂SO₄) et on l'a fait évaporer sous pression réduite pour obtenir l'acide brut qui est utilisé directement dans la réaction suivante, sans purification supplémentaire.

### 3. Déprotection des groupes N-protecteurs.

On a agité à température ambiante, pendant 3 à 9 heures, une solution d'acide dipeptide carboxylique N-protégé (1 mmol) dans du 3M HCl-dioxane (ou THF). On a éliminé les composants volatils par évaporation pour obtenir le chlorhydrate dipeptide.

### 4. Préparation des thioamides

On a ajouté le réactif de Lawesson (1,1 mmol) en une fois à une solution du dipeptide mentionné ci-dessous (étape 1) (2,0 mmoles) dans du toluène (10 mL) à température ambiante sous une atmosphère d'Argon. On a agité le mélange réactionnel à 80°C pendant 2 heures. On a éliminé le solvant par évaporation sous pression réduite. On a purifié le résidu par chromatographie sur colonne de gel de silice (CH₂Cl₂ puis CH₂Cl₂/Et₂O = 10/1) pour obtenir le thioamide correspondant.

### 5. Déprotection des thioamides à protection di-Boc, tert-butyl ester

On a ajouté à une solution du *tert*-butyl ester de thioamide à protection di-Boc (1 mmol) dans du dichlorométhane (5 mL) à 0°C, du TFA (5 mL) puis on a conservé la solution résultante à 0°C pendant une nuit. On a éliminé les composants volatils par évaporation pour obtenir le dithiopeptide sous la forme du sel de l'acide trifluoroacétique.

Les exemples suivants sont donnés à titre indicatif non limitatif.

Les composés suivants selon la présente invention ont été préparés en mettant en oeuvre le procédé indiqué ci-dessus.

### Exemple 1 : L-DAPA-L-Leu.2HCl

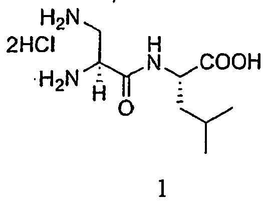

RMN ¹H (300 MHz, CD₃OD) δ 4,37 (t, *J* = 5,8 Hz, 1H), 4,30 (dd, *J* = 9,4, 5,6 Hz, 1H), 3,45 (dd, *J* = 13,9, 6,0 Hz, 1H), 3,34 (dd, *J* = 13,9, 5,3 Hz, 1H), 1,64-1,49 (m, 3H), 0,78 (d, *J* = 6,4 Hz, 3H), 0,74 (d, *J* = 6,4 Hz, 3H) ;
RMN ¹³C (75 MHz, CD₃OD) δ 176,6, 167,3, 53,0, 51,7, 41,4, 40,6, 26,1, 23,4, 21,5 ;
MS (ESI) *m*/*z* 218 [M+H]⁺ ;
SMHR calculée pour C₉H₂₀N₃O₃ (M+H) 218,1505 ; trouvée : 218,1512.
[α]_{D}²⁶+3,41 (c 1,0,6NHCl)

### Exemple 2 : L-DAPA-D-Leu.2HCl

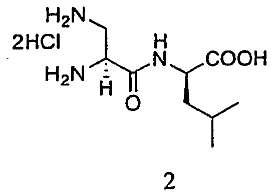

[α]_{D}²² + 7,8 (c 1,0 H₂O)
[α]_{D}²⁶ +39,59 (c 1,0, 6N HCl)
RMN ¹H (300 MHz. CD₃OD) δ 4,38 (dd, *J*= 5,0, 6,7 Hz, 1H), 4,30 (t, *J*= 7,8 Hz, 1H), 3,60-3,43 (m, 2H), 1,67-1,56 (m, 3H), 0,86 (d, *J*=6,0 Hz, 3H), 0,83 (d, *J* = 6,0 Hz, 3H) ;
RMN ¹³C (75 MHz, D₂O) δ 175,9, 166,0, 52,3, 50,7, 39,6, 39,1, 24,5, 22,0, 20,9 ;
   MS (ESI) *m*/*z* 218 [M+H]⁺ ;
SMHR calculée pour C₉H₂₀N₃O₃ (M+H) 218,1505 ; trouvée : 218,1552.

### Exemple 3 : L-DAPA-L-Leu.2TFA

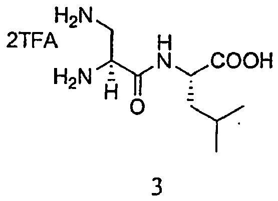

[α]_{D}²² +20 (c 0,5, MeOH;
[α]_{D}²⁴ +1,13 (c 1,0, 6N HCl)
RMN ¹H (300 MHz, CD₃OD) δ 4,51-4,46 (m, 1H), 4,12 (t, *J* = 6,2 Hz, 1H), 3,36 (d, *J* = 5,9 Hz, 2H), 1,78-1,62 (m, 3H), 0,98 (d, *J* = 6,1 Hz, 3H), 0,95 (d, *J* = 6,1 Hz, 3H) ;
RMN ¹³C (62,5 MHz, D₂O) δ 176,6, 166,8, 52,6, 51,1, 40,3, 39,7, 25,1, 22,7, 21,1 ;
MS (ESI) *m*/*z* 218 [M+H]⁺, 240 [M+Na]⁺ ;
SMHR calculée pour C₉H₂₀N₃O₃ (M+H) 218,1505 ; trouvée : 218,1512, calculée pour C₉H₁₉N₃O₃Na (M+Na) 240,1324 ; trouvée : 240,1364.

### Exemple 4 : L-DAPA-L-LeuOMe.2TFA

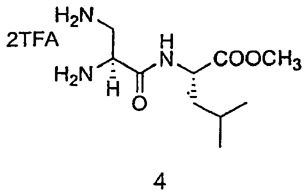

[α]_{D} + 3,8 (c 1,2, MeOH) ;
RMN ¹H (300 MHz, CD₃OD) δ 4,55 (t, *J* = 7,4 Hz, 1H), 4,42 (t, *J* = 5,9 Hz, 1H), 3,75 (s, 3H), 3,52 (d, *J* = 5,9 Hz, 2H), 1,81-1,61 (m, 3H), 0,95 (d, *J* = 6,8 Hz, 3H), 0,93 (d, *J* = 6,6 Hz, 3H) ;
RMN ¹³C (62,5 MHz, CD₃OD) δ 175,0, 167,4, 53,4, 52,8, 51,9, 41,3, 40,9, 25,9, 23,3, 21,6 ;
MS (ESI) *m*/*z* 232 (M+H]⁺, 254 [M+Na]⁺ ;
SMHR calculée pour C₁₀H₂₂N₃O₃ (M+H) 232-1661 ; trouvée : 232,1660.

### Exemple 5 : L-DAPA-L-LeuOMe.2HCl

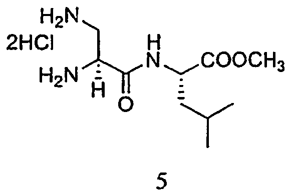

RMN ¹H (300 MHz, CD₃OD) δ 4,46 (dd, *J*=7,7, 5,6 Hz, 1H), 4,44 (t, *J* = 4,9 Hz, 1H), 3,67 (s, 3H), 3,48 (d, *J* = 5,6 Hz, 2H), 1,75-1,55 (m, 3H), 0,89 (d, *J* = 6,4 Hz, 3H), 0,86 (d, *J* = 6,4 Hz, 3H) ;
RMN ¹³C (75 MHz, CD₃OD) δ 175,0, 167,1, 53,4, 52,9, 51,8, 41,4, 40,8, 26,0, 23,3, 21,6 ;
MS (ESI) *m*/*z* 232 [M+H]⁺ ;
SMHR calculée pour C₁₀H₂₂N₃O₃ (M+H) 232,1661 ; trouvée : 232,1660.
[α]_{D}²⁴ +6,2 (c 0,7, MeOH)

### Exemple 6 : L-DAPA-L-IleNH₂.2HCl

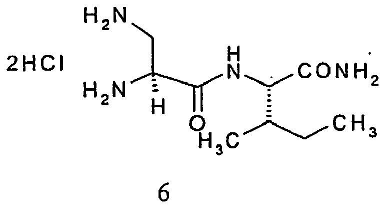

[α]_{D} + 25 (c 0,5, MeOH) ;
RMN ¹H (300 MHz, CD₃OD) δ 4,59 (dd, *J*= 7,0, 5,5 Hz, 1H), 4,37 (d, *J* = 5,5 Hz, 1H), 3,57 (dd, *J=* 13,4, 5,5 Hz, 1H), 3,36 (dd, *J=* 13,4, 7,2 Hz, 1H), 2,01-1,91 (m, 1H), 1,58-1,47 (m, 1H), 1,44-1,28 (m, 1H), 1,05 (d; *J*= 6,8 Hz, 3H), 0,95 (t, *J* = 7,4 Hz, 3H) ;
RMN ¹³C (62,5 MHz, CD₃OD) δ 176,3, 167,6, 60,6, 51,3, 41,2, 37,8, 25,5, 16,3, 12,0 ;
MS (ESI) *m*/*z* 217 [M+H]⁺, 239 [M+Na]⁺ ;
SMHR calculée pour C₉H₂₁N₄O₂ (M+H) 217,1665 ; trouvée : 217,1674, calculée pour C₉H₂₀N₄O₂Na (M+Na) 239,1484 ; trouvée : 239,1499.

### Exemple 7 : D-DAPA-D-Leu·2HCl

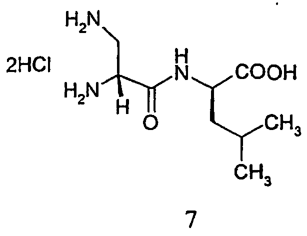

RMN ¹H (300 MHz, D₂O) δ 4,38-4,31 (m, 2H), 3,50-3,34 (m, 2H), 1,62-1,50 (m, 3H), 0,79 (d, *J =* 6,4 Hz, 3H), 0,75 (d, *J =* 6,4 Hz, 3H).
[α]_{D} -11,7 (c 1,0, H₂O)
MS (ESI) *m*/*z* 218 [M+H]⁺ ; 240 [M+Na]⁺ ;
SMHR 218,1505 calculée pour C₉H₂₀N₃O₃ (M+H) ; trouvée 218,1537

### Exemple 8: D-DAPA-D-Ala.2HCl

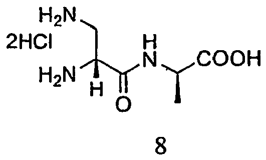

[α]_{D} -21,9 (c 1,0, MeOH) ;
[α]_{D}²⁶ -6,15 (c 1,0, 6N HCl)
RMN ¹H (300 MHz, D₂O) δ 4,53 (q, *J* = 7,4 Hz, 1H), 4,48 (t, *J* = 6,0 Hz, 1H), 3,64 (d, *J* = 6,0 Hz, 2H), 1,50 (d, *J* = 7,4 Hz, 3H) ;
RMN ¹³C (75 MHz, D₂O) δ 176,7, 166,5, 51,2, 49,9, 40,3, 16,6 ;
MS (ESI) *m*/*z* 176 [M+H]⁺ ;
Anal, calculée pour C₆H₁₅N₃O₃Cl₂ : C, 29,05 ; H, 6,09 ; N, 16,94 ; Cl, 28,58 ; trouvée : C, 28,67 ; H, 6,24 ; N, 16,67 ; Cl, 27,64.

### Exemple 9 : L-DAPA-L-Ala.2HCl

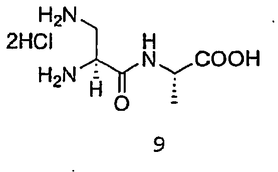

RMN ¹H (300 MHz, D₂O) δ 4,51 (q, *J* = 7,5 Hz, 1H), 4,46 (t, *J* = 6,0 Hz, 1H), 3,62 (d, *J* = 6,0 Hz, 2H), 1,49 (d, *J* = 7,5 Hz, 3H) ;
RMN ¹³C (75 MHz, D₂O) δ 176,8, 166,5, 51,2, 49,9, 40,3, 16,6 ;
MS (ESI) *m*/*z* 176 [M+H]⁺ ;
SMHR calculée pour C₆H₁₄N₃O₃, (M+H) 176,1035 ; trouvée : 176,1037.
[α]_{D}²⁶ +7,83 (c 1,0, 6N HCl)

### Exemple 10 : L-DAPA-D-Ala.2HCl

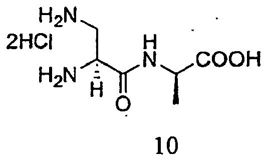

RMN ¹H (300 MHz, D₂O) δ 4,33-4,24 (m, 2H), 3,50-3,39 (m, 2H), 1,41 (d, *J* = 7,4 Hz, 3H) ;
RMN ¹³C (75 MHz, D₂O) δ 176,0, 166,0, 50,9, 49,5, 39,8, 16,3 ;
MS (ESI) *m*/*z* 176 [M+H]⁺ ;
SMHR calculée pour C₆H₁₄N₃O₃ (M+H) 176,1035 ; trouvée : 176,1044:
[α]_{D}²⁶ +64,56 (c 1,0, 6N HCl)

### Exemple 11 : D-DAPA-L-Ala.2HCl

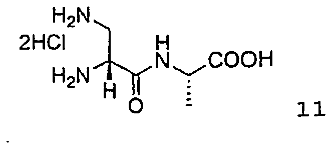

RMN ¹H (300 MHz, D₂O) δ 4,49-4,41 (m, 2H), 3,68-3,54 (m, 2H), 1,49 (d, *J* = 7,3 Hz, 3H) ;
RMN ¹³C (75 MHz, D₂O) δ 176,4, 166,3, 51,3, 49,8, 40,1, 16,6 ;
MS (ESI) *m*/*z* 176 [M+H]⁺ ;
SMHR calculée pour C₆H₁₄N₃O₃ (M⁺H) 176,1035 ; trouvée : 176,1043.
[α]_{D}²⁶ -60,9 (c 1,0, 6N HCl)

### Exemple 12 : (2S,3S)-DABA-L-Leu.2HCl

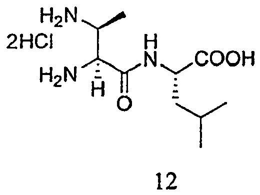

RMN ¹H (300 MHz, D₂O) δ 4,34 (t, *J* = 7,2 Hz, 1H), 4,22 (d, *J = 4,2* Hz, 1H), 3,87 (dq, *J =* 4,2, 7,2 Hz, 1H), 1,58-1,56 (m, 3H), 1,34 (d, *J =* 6,8 Hz, 3H), 0,79 *(d, J* = 6,0 Hz, 3H), 0,76 (d, J= 6,0 Hz, 3H) ;
RMN ¹³C (75 MHz, D₂O) δ 175,9, 165,6, 54,4, 52,0, 47,9, 39,0, 24,3, 22,0, 20,5, 14,0 ;
MS (ESI) *m*/*z* 232 [M+H]⁺ ;
SMHR calculée pour C₁₀H₂₂N₃O₃ (M+H) 232,1661 ; trouvée : 232,1663.
[α]_{D}²² +9,6 (c 0,2, H₂O)

### Exemple 13 : L-DAPA-Gly-OC₁₆H₃₃.2HCl

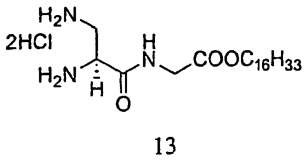

RMN ¹H (300 MHz, CD₃OD) δ 4,44 (t, *J* = 5,8 Hz, 1H), 4,20 (t, *J* = 6,7 Hz, 2H), 4,17, 4,07 (AB q, *J* = 17,8 Hz, 2H), 3,53 (d, *J* = 5,8 Hz, 2H), 1,73-1,64 (m, 2H), 1,43-1,30 (m, 26 H), 0,91 (t, *J =* 6,7 Hz, 3H) ;
RMN ¹³C (75 MHz, CD₃OD) δ 171,6, 167,5, 67,0, 51,9, 42,3, 41,2, 33,1, 30,8, 30,7, 30,5, 30,4, 29,7 ;
MS (ESI) *m*/*z* 386 [M+H]⁺ ;
SMHR calculée pour C₂₁H₄₄N₃O₃ (M+H) 386,3383 ; trouvée : 386,3352.
[α]_{D}²⁶ -5,98 (c 0,5, MeOH)

### Exemple 14 : L-DAPA-L-Leu-OC₁₆H_{33·}2HC1

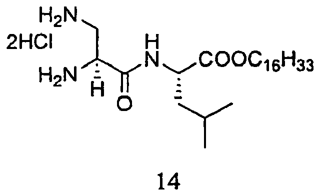

RMN ¹H (300 MHz, CD₃OD) δ 4,34-4,28 (m, 2H), 4,01-3,86 (m, 2H), 3,41-3,29 (m, 2H), 1,61-1,39 (m, 5H), 1,16-1,05 (m, 26H), 0,76 (d, *J* = 6,4 Hz, 3H), 0,72 (d, *J* = 6,4 Hz, 3H), 0,66 (t, *J* = 6,7 Hz, 3H) ;
RMN ¹³C (75 MHz, CD₃OD) δ 174,6, 167,2, 67,2, 53,0, 51,8, 41,4, 40,9, 33,2, 30,9, 30,6, 30,6, 30,6, 30,4, 29,7, 27,0, 26,1, 23,8, 23,4, 21,8, 14,6 ;
MS (ESI) *m*/*z* 442 [M+H]⁺ ;
SMHR calculée pour C₂₅H₅₂N₃O₃ (M+H) 442,4009 ; trouvée: 441,3983.
[α]_{D}²⁶ +13,1 (c 2,0, MeOH)

### Exemple 15 : L-DAPA-L-(S)-Leu.2TFA

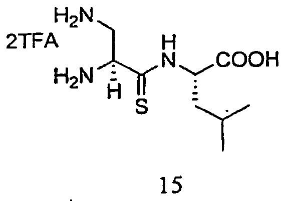

RMN ¹H (300 MHz, D₂O) δ 4,79 (dd, *J* = 9,3, 5,4 Hz, 1H), 4,56 (t, *J* = 6,4 Hz, 1H), 3,48 (dd, *J*= 13,8, 5,8 Hz, 1H), 3,41 (dd, *J*= 13,8, 6,6 Hz, 1H), 1,79-1,53 (m, 3H), 0,81 (d, *J* = 6,4 Hz, 3H), 0,77 (d, *J* = 6,4 Hz, 3H) ;
RMN ¹³C (62,5 MHz, D₂O) δ 194,5, 174,9, 58,0, 54,6, 41,5, 38,8, 24,7, 22,0, 20,7 ;
MS(ESI) *m*/*z* 234[M+H]⁺;
SMHR 234,1276 calculée pour C₉H₂₀N₃O₂S ; trouvée : 234,1306.
[α]_{D}²⁶ +60,6 (c 2,5, MeOH)

### Exemple 16 : L-DAPA-L-(S)-Leu.2HCl

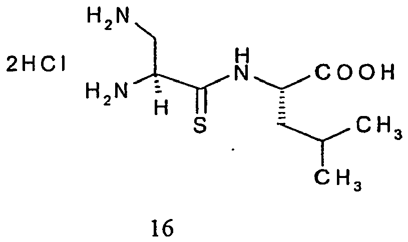

RMN ¹H (300 MHz, D₂O) δ 4,80 (dd, *J* = 9,6, 4,9 Hz, 1H), 4,60 (t, J= 6,2 Hz, 1H), 3,52-3,39 (m, 2H), 1,81-1,58 (m, 3H), 0,82 (d, *J* = 6,4 Hz, 3H), 0,78 (d, *J* = 6,2 Hz, 3H);
RMN ¹³C (62,5 MHz, D₂O) δ 194,5, 174,8, 57,9, 54,6, 41,5, 38,8, 24,7, 22,0, 20,7 ;
MS(ESI) *m*/*z* 234[M+H]⁺;
SMHR 234,1276 calculée pour C₉H₂₀N₃O₂S ; trouvée : 234,1306.
[α]_{D}²⁶ +90,6 (c 1,0, MeOH)

### Exemple 17 : L-DAPA-Gly·2HCl

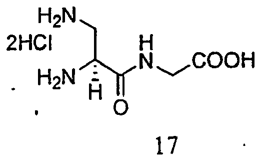

RMN ¹H (300 MHz, D₂O) δ 4,54 (t, *J*= 5,8 Hz, 1H), 4,20 (d, *J*= 18,0 Hz, 1H), 4,10 (d, *J*=18,0 Hz, 1H), 3,65 (d, *J*= 5,8 Hz, 1H) ;
RMN ¹³C (75 MHz, D₂O) δ 173,1, 166,5, 50,5, 41,6, 39,5 ;
MS (ESI) *m*/*z* 162[M+H]⁺
SMHR calculée pour C₅H₁₂N₃O₃ (M+H) 162,0879; trouvée: 162,0864.
[α]_{D}²⁵ +28 (c 1,8, H₂O)

### Exemple 18 : L-DAPA-L-Ile·2HCl

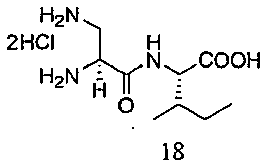

RMN ¹H (300 MHz, D₂O) δ 4,52 (t, *J* = 5,9 Hz, 1H) ; 4,46 (d, *J* = 4,9 Hz, 1H) ; 3,60 (d, *J* = 5,9 Hz, 2H) ; 2,05 (m, 1H) ; 1,45 (m, 1H) ; 1,27 (m, 1H) ; 0,97 (d, *J* = 6,9 Hz, 3H) ; 0,90 (t, *J* = 7,3 Hz, 3H) ;
RMN ¹³C (62,5 MHz, D₂O) δ 175,5, 166,8, 58,8, 51,1, 40,3, 37,0, 25,3, 15,7, 11,6;
MS (ESI) *m*/*z* 218 [M+H], 240 [M+Na]⁺ ;
SMHR 218,1505 calculée pour C₉H₂₀N₃O₃ ; trouvée: 218,1537.
[α]_{D}²⁶ +22,4 (c 1,2, H₂O)

### Exemple 19 : L-DAPA-β-Ala·2 HCl

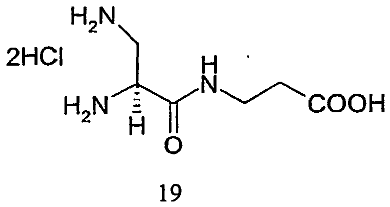

RMN ¹H (300 MHz, CD₃OD) δ 4,40 (t, *J* = 5,8 Hz, 1H), 3,54 (m, 4H) ; 2,64 (m, 2H) ;
RMN ¹³C (62,5 MHz, CD₃OD) δ 174,0, 166,6, 52,1, 41,1, 36,9, 34,2 ;
MS (ESI) *m*/*z* 176 [M+H]⁺ ;
SMHR calculée pour C₆H₁₄N₃O₃ (M+H) 176,1035; trouvée : 176,1068 ;
[α]_{D}²⁶+3,8 (c 0,5, H₂O)

### Exemple 20 : D-DAPA-β-Ala·2HCl

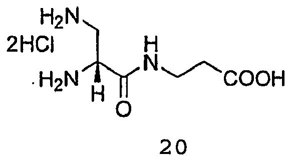

RMN ¹H (300 MHz, CD₃OD) δ 4,40 (t, *J* = 5,8 Hz, 1H), 3,54 (m, 4H) ; 2,64 (m, 2H) ;
RMN ¹³C (62,5 MHz, CD₃OD) δ 174,0, 166,6, 52,1, 41,1, 36,9, 34,2 ;
MS (ESI) *m*/*z* 176 [M+H]⁺ ;

### Exemple 21 : L-DAPA-L-Phe·2HCl

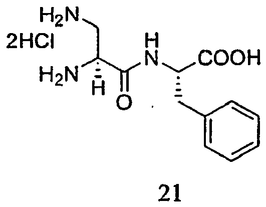

RMN ¹H (300 MHz, CD₃OD) δ 7,32 (m, 5H) ; 4,79 (dd, *J* = 9,9, 4,4 Hz, 1H) ; 4,48 (t, *J* = 5,9 Hz, 1H) ;3,61 (dd, *J* = 13,9, 6,1 Hz, 1H) ; 3,51 (dd, J *= 13,9,* 5,7 Hz, 1H) ; 3,35 (dd, *J* = 14,2, 3,4 Hz, 1H) ; 3,08 (dd, *J* = 14,2, 9,9 Hz, 1H) ; RMN ¹³C, (62,5 MHz, CD₃OD) δ 175,1, 167,2, 138,2, 130,3, 129,7, 128,1, 68,2, 56,2, 51,7, 41,3, 37,5 ;
MS (ESI) *m*/*z* 252 [M+H]⁺.
SMHR calculée pour C₁₂H₁₈N₃O₃ (M+H) 252,1348; trouvée : 252,1341.
[α]_{D}²⁶ +41,8 (c 1,0, H₂O)

### Exemple 22 : D-DAP A-D-Phe·2HCl

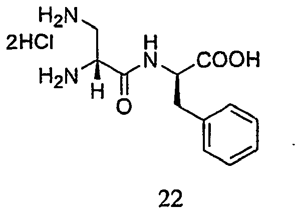

RMN ¹H (300 MHz, CD₃OD) δ 7,32 (m, 5H) ; 4,79 (dd, *J* = 9,9, 4,4 Hz, 1H) ; 4,48 (t, *J* = 5,9 Hz, 1H) ; 3,61 (dd, *J* = 13,9, 6,1 Hz, 1H) ; 3,51 (dd, *J* = 13,9, 5,7 Hz, 1H) ; 3,35 (dd, *J = 14,2, 3,4* Hz, 1H) ; 3,08 (dd, *J =* 14,2, 9,9 Hz, 1H) ; RMN ¹³C (62,5 MHz, CD₃OD) δ 175,1, 167,2, 138,2 130,3, 129,7, 128,1, 68,2, 56,2,51,7,41,3,37,5;
MS (ESI) *m*/*z* 252 [M+H]⁺, 269 [M+H₂O]⁺.
SMHR calculée pour C₁₂H₁₈N₃O₃ (M+H) 252,1348; trouvée : 252,1349.
[α]_{D}²⁶ -38,0 (c 1,9, H₂O)

### Exemple 23 : L-DAPA-L-Val·2HCl

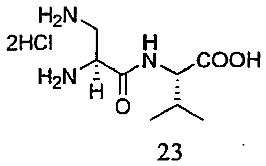

RMN ¹H (300 MHz, D₂O) δ 4,54 (t, *J* = 5,9 Hz, 1H) ; 4,42 (d, *J* = 5,0 Hz, 1H) ; 3,60 (d, *J* = 5,9 Hz, 2H) ; 2,29 (m, 1H) ; 0,97 (t, *J* = 6,7 Hz, 6H) ;
RMN ¹³C (62,5 MHz, D₂O) δ 175,5, 166,9, 59,5, 51,1, 40,3, 30,4, 19,0, 17,6 ; MS (ESI) *m*/*z* 204 [M+H]⁺ ;
SMHR 204,1348 calculée pour C₈H₁₈N₃O₃ trouvée 204,1365.
[α]_{D}²⁶ +22,2 (c 2,0, H₂O)

### Exemple 24 : L-DAPA-D-DAPA·3HCl

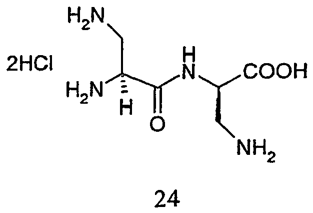

RMN ¹H (300 MHz, D₂O) δ 4,60 (m, 1H) ; 4,55 (dd, *J* = 6,5, 4,9 Hz, 1H) ; 3,65 (m, 3H) ; 3,45 (dd, *J* = 13,5, 7,7 Hz, 1H) ;
RMN ¹³C (62,5 MHz, D₂O) δ 171,6, 167,6, 51,8, 51,5, 40,2, 40,1 ;
MS (ESI) *m*/*z* 191 [M+H]⁺ ;
SMHR 191,1144 calculée pour C₆H₁₅N₄O₃ ; trouvée : 191,1146.
[α]_{D}²⁶ -43,4 (c,0,4, H₂O)

### Exemple 28 : (2S,3R)-DASA-1-Gly-4-Gly-2HCl

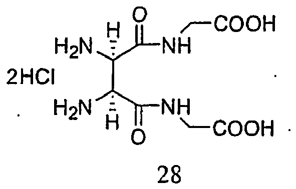

RMN ¹H (300 MHz, D₂O) δ 4,61 (s, 1H) ; 4,43 (s, 1H) ; 4,06, 4,04 (2s, 4H) ;
RMN ¹³C (62,5 MHz, D₂O) δ 172,9, 172,7, 52,7, 42,5 ;
MS (ESI) *m*/*z* 263 [M+H]⁺ ; 285 [M+Na]⁺;
SMHR 263,0992 calculée pour C₈H₁₅N₄O₆ ; trouvée 263,0970.
[α]_{D}²⁶ -2,2 (c 1,5, H₂O)

### Exemple 29 : (2S,3S)-DASA-1-L-Val-4-L-Val.2HCl

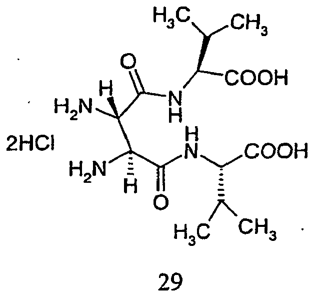

RMN ¹H (300 MHz, D₂O) δ 4,78 (m, 1H) ; 4,65 (m, 1H) ; 4,35 (m, 1H), 4,21 (m, 1H) ; 2,12 (m, 2H) ; 0,85 (m, 12H) ;
RMN ¹³C (62,5 MHz, D₂O) 175,7, 174,8, 59,2, 59,0, 52,8, 52,3, 30,0, 29,7, 18,4, 18,3, 17,2, 16,9.
MS (ESI) *m*/*z* 347 [M+H]⁺, 369 [M+Na]⁺ ;
SMHR 369,1750 calculée pour C₁₄H₂₆N₄O₆Na; trouvée : 369,1760.
[α]_{D}²⁶ -38,8 (c 0,5, H₂O).

### Exemple 30 : (2S,3S)-DASA-1-L-Ile-4-L-Ile.2HCl

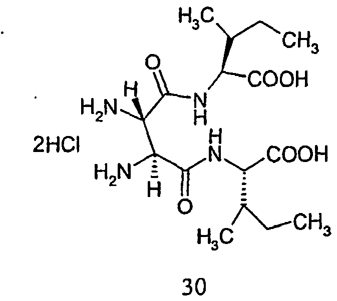

RMN ¹H (300 MHz, D₂O) δ 4,78 (m, 1H) ; 4,62 (m, 1H) ; 4,40 (m, 2H) ; 1,75 (m, 6H) ; 0,85 (m, 12H) ;
RMN ¹³C (62,5 MHz, D₂O) 173,9, 173,1, 164,9, 163,7, 56,3, 54,8, 52,0, 51,8, 39,4, 24,5, 24,4, 22,5, 22,2, 20,5 ;
MS (ESI) *m*/*z* 375 [M+H]⁺, 397 [M+Na]⁺ ;
SMHR 397,2063 calculée pour C₁₆H₃₀N₄O₆Na ; trouvée : 397,1995.
[α]_{D}²⁶ -18,7 (c 0,3, MeOH)

### Exemple 31 : L-DAPA-L-Pro·2HCl

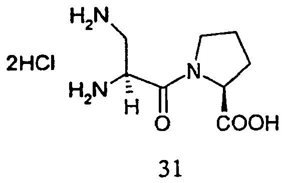

RMN ¹H (300 MHz, D₂O) δ 4,62 (m, 1H) ; 4,39 (m, 1H) ; 3,62 (d, *J* = 7,6 Hz, 1H) ; 3,57 (m, 2H) ; 3,42 (dd, *J* = 13,6, 6,1 Hz, 1H) ; 2,05 (m, 2H) ; 1,95 (m, 2H) ;
RMN ¹³C (62,5 Hz, D₂O) δ 173,1, 164,9, 59,6, 52,6, 46,0, 39,2, 28,2, 22,3 ;
MS (ESI) *m*/*z* 202 [M+H] ;
SMHR 202,1192 calculée pour C₈H₁₆N₃O₃ ; trouvée 202,1196.
[α]_{D}²⁶ -72,2 (c 1,3, H₂O)

### Exemple 34 : L-DAPA-L-Ala- L-Ala·2HCl hors invention

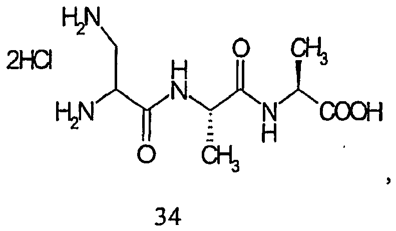

### Exemple 35 : L-DAPA-L-Ala- L-Val·2HCl hors invention

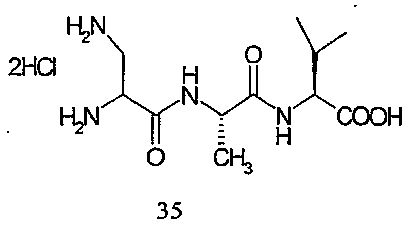

### Exemple 36 : L-DAPA-L-Ala- L-Pro·2HCl hors invention

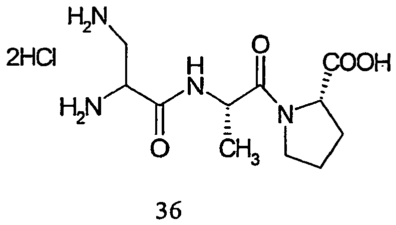

### Exemple 37: D-DAPA-Gly.2HCl

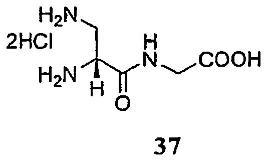

RMN ¹H (300 MHz, D₂O) δ 4,42 (t, *J* = 5,8 Hz, 1H), 4,00 (d, *J* = 18,1 Hz, 1H), 4,10 (d, *J* = 18,1 Hz, 1H), 3,53 (d, *J* = 5,8 Hz, 2H) ;
RMN ¹³C (75 MHz, D₂O) δ 172,7, 166,4, 50,5, 41,4, 39,5 ;
MS (ESI) *m*/*z* 162 [M+H]⁺;
SMHR calculée pour C₅H₁₂N₃O₃ (M+H) 162,0879; trouvée : 162,0863.
[α]_{D}²⁵ -28,8 (c 1,3, H₂O)

### Exemple 38: L-DAPA-GlyOMe.2THF

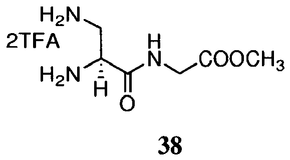

RMN ¹H (300 MHz, CD₃OD) δ 4,39 (t, *J* = 5,7 Hz, 1H), 4,15 (d, *J* = 17,8 Hz, 1H), 4,05 (d, *J* = 17,8 Hz, 1H), 3,75 (s, 3H), 3,52 (dd, *J* = 5,7, 1,5 Hz, 2H) ;
RMN ¹³C (75 MHz, CD₃OD) δ 171,9, 167,5, 53,1, 51,9, 42,1, 41,0 ;
MS (ESI) *m*/*z* 176 [M+H]⁺ ;
SMHR calculée pour C₆H₁₄N₃O₃ (M+H) 176,1035; trouvée : 176,1005.
[α]_{D}²⁵ +24,0 (c 1,1, H₂O)

### Exemple 39: L-DAPA-GlyNH₂.2HCl

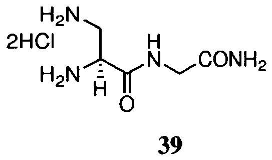

RMN ¹H (300 MHz, D₂O) δ 4,43 (t, *J* = 5,9 Hz, 1H), 4,0 (m, 2H), 3,52 (d, *J* = 5,9 Hz, 2H) ;
RMN ¹³C (75 MHz, D₂O) δ 173,1, 166,7, 50,6, 42,1, 39,4 ;
MS (ESI) *mlz* 161 [M+H]⁺ ;
SMHR calculée pour C₅H₁₃N₄O₂ (M+H) 161,1039; trouvée : 161,1042.
[α]_{D}²⁵ +38,6 (c 0,23, H₂O)

### Exemple 40: D-DAPA-D-Asp.2HCl

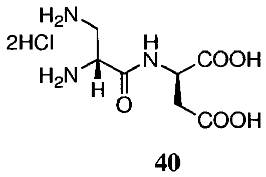

RMN ¹H (300 MHz, D₂O) δ 4,75 (m, 1H), 4,38 (dd, *J* = 6,3, 5,3 Hz, 1H), 3,50 (dd, *J* = 14,4, 5,3 Hz, 1H), 3,42 (dd, *J=* 14,4, 6,4 Hz, 1H), 2,93 (d, *J* = 6,3 Hz, 2H) ;
RMN ¹³C (75 MHz, D₂O) δ 174,2,173,2, 165,9, 50,6, 49,4, 39,4, 35,1 ;
MS (ESI) *m*/*z* 220 [M+H]⁺ ;
SMHR calculée pour C₇H₁₄N₃O₅ (M+H) 220,0933; trouvée : 220,0903.
[α]_{D}²⁵ -32,4 (c 0,7, H₂O)

### Exemple 41: D-DAPA-L-Phe.2HCl

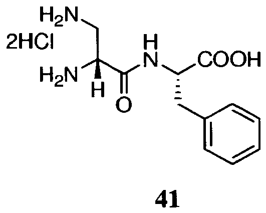

RMN ¹H (300 MHz, D₂O) δ 7,25 (m, 5H), 4,72 (dd, *J* = 8,7, 5,6 Hz, 1H), 4,28 (dd, *J =* 6,1, 5,7 Hz, 1H), 3,49 (dd, *J* = 14,3, 6,2 Hz, 1H), 3,43 (dd, *J* = 14,3, 5,7 Hz, 1H), 3,22 (dd, *J* = 14,3, 5,6 Hz, 1H), 3,03 (dd, *J=* 14,3, 8,7 Hz, 1H) ;
RMN ¹³C (75 MHz, D₂O) δ 174,4, 165,8, 136,3, 129,2, 128,8, 127,3, 66,6, 54,6, 50,4, 39,6 ;
MS (ESI) *m*/*z* 252 [M+H]⁺
SMHR calculée pour C₁₂H₁₈N₃O₃ (M+H) 252,1348; trouvée : 252,1357.
[α]_{D}²⁵ -42,3 (c 1,0, H₂O)

### Exemple 42: D-DAPA-D-Ph₂.2HCl

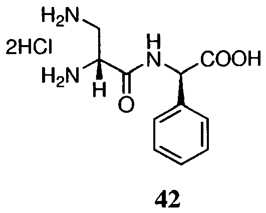

RMN ¹H (300 MHz, D₂O) δ 7,32 (s, 5H), 5,43 (s, 1H), 4,38 (m, 1H), 3,52 (d, *J* = 5,9 Hz, 2H) ;
RMN ¹³C (75 MHz, D₂O) δ 173,2, 165,5, 134,4, 129,3, 127,8, 57,6, 50,4, 39,6 ;
MS (ESI) *m*/*z* 238 [M+H]⁺ ;
SMHR calculée pour C₁₁H₁₆N₃O₃ (M+H) 238,1192; trouvée : 238,1190.
[α]_{D}²⁵ -81,4 (c 1,0, H₂O)

### Exemple 43: L-DAPA-L-Nle.2HCl

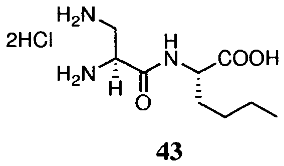

RMN ¹H (300 MHz, D₂O) δ 4,35 (m, 2H), 3,48 (d, *J* = 5,9Hz, 2H), 1,70 (m, 1H), 1,60 (m, 1H), 1,20 (m, 4H), 0,72 (t, *J* = 7,2 Hz, 3H) ;
RMN ¹³C (75 MHz, D₂O) δ 175,6, 166,1, 57,1, 53,6, 50,4, 39,6, 29,9, 27,0, 21,5, 13,0 ;
MS (ESI) *m*/*z* 218 [M+H]⁺ ;
SMHR calculée pour C₉H₂₀N₃O₃ (M+H) 218,1505; trouvée : 218,1518.
[α]_{D}²⁵ + 6,8 (c 0,5, H₂O)

### Exemple 44 : D-DAPA-D-L-Nle.2HCl

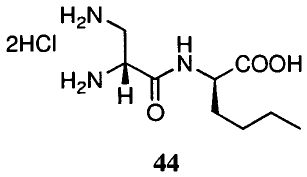

### Exemple 45: D-DAPA-L-Lys.3HCl

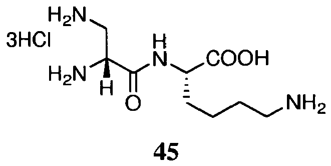

RMN ¹H (300 MHz, D₂O) δ 4,42 (dd, *J* = 6,6, 5,1 Hz, 1H), 4,28 (dd, *J* = 8,0, 5,8 Hz, 1H), 3,52 (m, 2H), 2,90 (t, *J* = 7,6 Hz, 2H), 1,82 (m, 2H), 1,60 (m, 2H), 1,37 (m, 2H) ;
RMN ¹³C (75 MHz, D₂O) δ 174,9, 166,1, 53,5, 50,7, 39,5, 39,2, 29,7, 26,3, 22,1 ;
MS (ESI) *m*/*z* 233 [M+H]⁺ ;
SMHR calculée pour C₉H₁₀N₄O₃ (M+H) 233,1614; trouvée 233,1624.
[α]_{D}²⁵ -41,0 (c 0,6, H₂O)

### Exemple 46: D-DAPA-L-Leu.2HCl

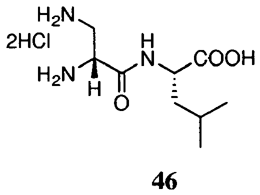

RMN ¹H (300 MHz, D₂O) δ 4,37 (dd, *J* = 6,8, 4,9 Hz, 1H), 4,28 (dd, *J* = 8,0, 6,5 Hz, 1H), 3,49 (m, 2H), 1,7-1,5 (m, 3H), 0,85 (d, *J* = 6,2 Hz, 3H), 0,81 (d, *J =* 6,2 Hz, 3H) ;
RMN ¹³C (75 MHz, D₂O) δ 176,0, 166,0, 52,4, 50,7, 39,6, 39,1, 24,5, 22,0, 20,9 ; MS (ESI) *m*/*z* 218 [M+H]⁺ ;
SMHR calculée pour C₉H₂₀N₃O₃ (M+H) 218,1505; trouvée : 218.1506.
[α]_{D}²⁵ -2,5 (c 1,4, H₂O)

### Exemple 47 : L-DAPA-(CH₂)-L-Val.2TFA

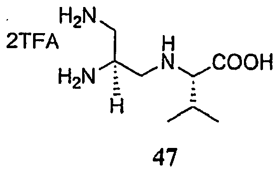

RMN ¹H (300 MHz, CD₃OD) δ 3,74 (quintet, *J* = 6,2 Hz, 1H), 3,42 (d, *J=* 4,3 Hz, 1H), 3,36 (dd, *J* = 6,2 Hz, 2H), 3,12-3,10 (m, 2H), 2,25-2,14 (m, 1H), 1,05 (d, *J* = 7,0 Hz, 1H);
RMN ¹³C (62,5 MHz, CD₃OD) δ 175,9, 68,3, 49,7, 49,1, 41,6, 32,1, 18,9, 18,8;
MS (ESI) *m*/*z* 190 [M+H]⁺;
HRMS calculée pour C₈H₁₉N₃O₂ (M+H) 190,1556; trouvée: 190,1552.
[α]_{D}²⁶ +3,0 (c 1,0, MeOH)

### Exemple 48: L-DAPA-L-Asp.2TFA

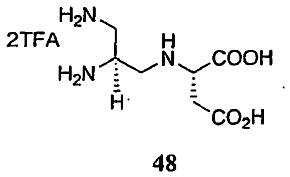

RMN ¹H (300 MHz, CD₃OD) δ 4,86 (dd, *J* = 6,3, 4,8 Hz, 1H), 4,42 (t, *J* = 5,7 Hz, 1H), 3,57 (dd, *J=* 13,9, 5,8 Hz, 1H), 3,52 (dd, *J* = 13,9, 5,8 Hz, 1H), 2,99 (dd, *J* = 17,2, 6,2 Hz, 1H), 2,91 (dd, *J* = 17,2,4,6 Hz, 1H);
RMN ¹³C (62,5 MHz, D₂O) δ 174,5, 73,8, 167,1, 51,8, 50,8,41,2, 36,2;
MS (ESI) *m*/*z* 220 [M+H]⁺;
HRMS calculée pour C₇H₁₄N₃O₅ (M+H) 220,0933; trouvée: 220,0950.
[α]_{D}²⁶+36,6 (*c* 1,0, MeOH)

### Exemple 49 : D-DAPA-L-(4-trifluorométhyle)-Phe.OH.2HCl

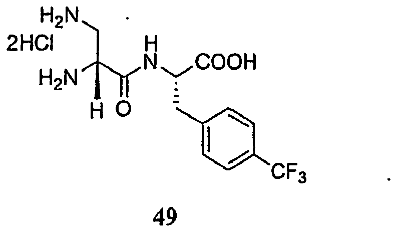

RMN ¹H (300 MHz, D₂O) δ 7,58 (d, *J* = 7,9 Hz, 2H), 7,35 (d, *J* = 8,1 Hz, 2H), 4,69 (m, 1H), 4,25 (t, *J* = 6,0 Hz, 1H), 3,27 (dd, *J* = 13,9, 5,8 Hz, 1H); 3,17 (d, *J* = 6,0 Hz, 2H), 3,04 (dd, *J* = 14,0, 9,3 Hz, 1H);
RMN ¹³C (75 MHz, D₂O) δ 174,1, 165,7, 140,7, 130,0, 129,6 (q, *J* = 32,9 Hz), 125,5, 125,0 (q, *J* = 271,1 Hz), 54,3, 50,7, 39,5, 36,3;
MS (ESI) *m*/*z* 320 [M+H]⁺,;
HRMS calculée pour C₁₃H₁₇F₃N₃O₃ (M+H) 320,1222; Trouvé: 320,1236.

### Exemple 50 : D-DAPA-L-ε-trifluorométhyle-Nle.OH.2HCl

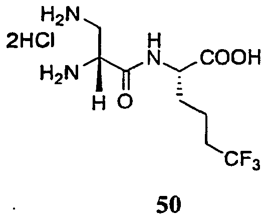

### Exemple 51 : D-DAPA-L-Nle.2HCl

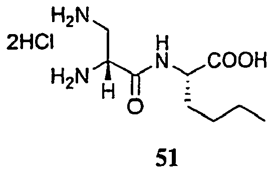

RMN ¹H (300 MHz, D₂O) δ 4,43 (dd, *J* = 6,6, 5,1 Hz, 1H), 4,33 (dd, *J* = 8,1, 5,7 Hz, 1H), 3,60 (dd, *J* = 14,5, 5,1 Hz, 1H), 3,53 (dd, *J* = 14,5, 6,6 Hz, 1H), 1,82 (m, 2H), 1,34 (m, 4H), 0,86 (t, *J* = 7,2 Hz, 3H) ;
RMN ¹³C (75 MHz, D₂O) δ 175,6, 166,0, 53,8, 50,6, 39,4, 29,9, 27,1, 21,6, 13,0 ;
MS (ESI) *m*/*z* 218 [M+H]⁺;
SMHR calculée pour C₉H₂₀N₃O₃ (M+H) 218,1505; trouvée : 218,1506.
[α]_{D}²⁵ -43,0 (c 1,4, H₂O)

### Exemple 52 D-DAPA-DL-p-fluoroPhe.2HCl

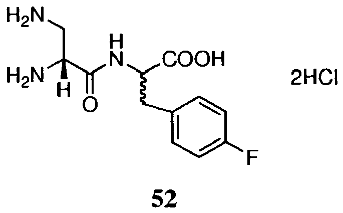

RMN ¹H (300 MHz, D₂O) δ 7,20-7,15 (m, 2H), 7,03-6,95 (m, 2H), 4,66-4,59 (m, 1H), 4,27 (t, *J* = 5,7 Hz, 0,5H), 4,25 (t, *J* = 5,8 Hz, 0,5H), 3,43 (d, *J* = 6,2 Hz, 0,5H), 3,42 (d, *J* = 5,7 Hz, 0,5H), 3,20-3,10 (m, 1H), 3,17 (d, *J* = 6,0 Hz, 1H), 2,97 (dd, *J* = 14,1, 8,6 Hz, 0,5H), 2,94 (dd, *J* = 14,1, 9,2 Hz, 0,5H);
RMN ¹³C (75 MHz, D₂O) δ 174,3, 174,2, 165,8, 165,7, 162,8 (d, *J* = 243,7 Hz), 133,0, 132,8, 131,7, 131,6, 116,3, 116,0, 54,7, 54,5, 50,7, 50,4, 39,5, 35,7, 35,5;
MS (ESI) *m*/*z* 270 [M+H]⁺;
HRMS calculée pour C₁₂H₁₇FN₃O₃ (M+H) 270,1254; Trouvée: 270,1255.

### Exemple 53 : résultats biochimiques et biologiques

L'efficacité des nouveaux composés selon la présente invention est démontrée de la manière suivante :

### Modification de l'insuline par le méthylglyoxal et effet inhibiteurs des composés selon la présente invention : comparaison entre ces produits et les inhibiteurs de l'art antérieur

L'insuline humaine (Ins) est incubée avec du méthylglyoxal (MG) dans des conditions physiologiques. Après 24 heures, l'insuline est complètement modifiée tel que ceci est illustré sur la figure 1 (Ins + MG).

En revanche, l'insuline est incubée avec du méthylglyoxal et en présence d'une quantité équimolaire d'inhibiteurs d'AGEs selon la présente invention dans des conditions physiologiques. Après 24 heures, la modification de l'insuline par le MG est considérablement réduite tel que ceci est illustré sur la figure 1.

La figure 2 illustre l'efficacité de certains piégeurs de dicarbonyles réactifs connus pour inhiber la modification de l'insuline par le MG.

Les analyses par CLPH mettent clairement en évidence que certains des inhibiteurs d'AGEs selon la présente invention piègent le méthylglyoxal qui peut autrement modifier l'insuline (figure 3). Certains exemples des effets des inhibiteurs d'AGEs selon la présente invention sur la somatostatine-14 (contient 2 Lys) et la ribonucléase (RNase) A (contient 10 Lys et 4 Arg) sont illustrés sur la figure 4 et sur la figure 5, respectivement.

### Etudes par électrophorèse des capacités inhibitrices des nouveaux composés selon la présente invention, piégeurs de MG, contre la formation d'AGEs

La ribonucléase A et la lysozyme (10 mg /ml) sont incubées en présence du méthylglyoxal (10 mM) ou en présence du méthylglyoxal et de l'un des inhibiteurs selon la présente invention en quantité équimolaire à 37°C. Après 48 heures d'incubation, les protéines sont analysées par électrophorèse sur gel de polyacrilamide (gel SDS PAGE 8-16%).

L'analyse des résultats montre qu'en présence du méthylglyoxal, la ribonucléase A et la lysozyme présentent une forte modification qui se manifeste par l'apparition d'une forme dimérique de la protéine.

L'addition de l'un des inhibiteurs selon la présente invention : L-DAPA-L-leu (exemple 1), L-DAPA-L-Ile (exemple 18), L-DAPA-L-Val (exemple 23), D-DAPA-D-Ala (exemple 8), (2S,3S)-DASA-L-Val (exemple 29), L-DAPA-L-Gly (exemple 17), L-DABA-L-Leu (exemple 12), protège contre cette modification structurale, exercée par le méthylglyoxal. La présence d'inhibiteurs selon la présente invention empêche largement la formation de protéines réticulées en piégeant le méthylglyoxal.

Des mesures d'activités enzymatiques sont réalisées sur la ribonucléase A après traitement par le méthylglyoxal et les différents inhibiteurs selon la présente invention en utilisant la technique de coloration de l'ARN au bleu de méthylène selon Greiner-Stoeffele et al. (Anal. Biochem. (1996) 240, 24).

Les résultats sont rassemblés dans le tableau 1 ci-après :

| **conditions** | **Activités enzymatiques (%)** |
|---|---|
| **RNase témoin** | 100 |
| **RNase + méthylglyoxal** | 10,11% |
| **MG + D-DAPA** | 20,32% |
| **MG (+) AG** | 70,52% |
| **MG + L-DABA-L-Leu (exemple 12)** | 80,97% |
| **MG + D-DAPA-D-Ala (exemple 8)** | 87,37 |
| **MG + L-DAPA-L-Gly (exemple 17)** | 85,61% |
| **MG + L-DAPA-L-Phe (exemple 21)** | 70,20% |
| **MG + (2S,3S)-DASA-L-Val (exemple 29)** | 75,09% |
| **MG + L-DAPA-L-(S)-Leu (exemple 16)** | 75,69% |
| **MG + L-DAPA-L-Leu (exemple 1)** | 93,91% |
| **MG + L-DAPA-L-Ile (exemple 18)** | 90,77% |
| **MG + L-DAPA-L-Val (exemple 23)** | 90,77% |

Les valeurs des cinétiques enzymatiques suivies au spectrophotomètre à 688 nm montre que l'inhibition d'activité enzymatique causée par le méthylglyoxal est considérablement réduite en présence d'inhibiteurs selon la présente invention.

Des analyses comparatives (par électrophorèse et par mesure d'activité enzymatique) sont réalisées sur la ribonucléase A ou la lysozyme, en utilisant comme inhibiteur l'aminoguanidine (AG). Les résultats montrent clairement que les inhibiteurs selon la présente invention sont largement plus efficaces que l'AG.

On observe la même tendance pour le lysozyme (contient 6 Lys et 11 Arg) lors de tests effectués dans les mêmes conditions que la ribonucléase A.

Le MG réagit avec les résidus lysine et arginine dans les protéines, ce qui altère les charges sur le polypeptide modifié. Ceci a été mis en évidence par l'électrophorèse de la glyoxalase 1 traitée au MG dans des conditions non dénaturantes. L'exposition de la glyoxalase 1 au MG (10 mM) pendant 24 heures fait augmenter la mobilité de la protéine vers l'électrode positive, un changement qui est cohérent avec la perte des charges positives des groupes ε-amino et guanidino et le gain de charges négatives. Lorsque des inhibiteurs selon la présente invention (L-DAPA-L-Leu (exemple 1) ou L-DAPA-L-Ile (exemple 18)) sont inclus dans le mélange d'incubation. La présence de ces composés inhibe le gain de charge négative.

L'incubation de la glyoxalase I, protéine clef du système de détoxication contre les composés α-oxoaldéhydes, en présence du méthylglyoxal, modifie la protéine. Cette modification provoque un changement au niveau de la charge et une diminution de l'activité enzymatique de 50 % par rapport au témoin.

L'addition des composés selon la présente invention (L-DAPA-L-Leu ou L-DAPA-L-Ile) empêche l'inhibition exercée par le méthylglyoxal et protège contre les modifications structurales.

Des résultats comparatifs obtenus par électrophorèse montrent que l'aminoguanidine (AG) est bien moins efficace que les inhibiteurs d'AGEs (selon la présente invention) vis-à-vis de l'effet protecteur de ces composés contre les modifications structurales de la ribonucléase A induites par MG. Les inhibiteurs d'AGEs selon la présente invention L-DAPA-L-Leu (exemple 1) et le L-DAPA-L-Ile (exemple 18) ou d'AG (10 mM) sont incubés avec du MG et la ribonucléase A pendant 40 heures à 37°C.

### Croissance de cellules EA en présence de piégeurs du MG selon la présente invention et de l'art antérieur et/ou de méthylglyoxal

Les cellules utilisées pour le test sont les cellules EA.hy926. Ce sont des cellules endothéliales obtenues par fusion de cellules endothéliales de la veine ombilicale humaine (HUVECS) avec des cellules oncogènes pulmonaires (A 549). Les cellules endothéliales EA. Hy 926 sont incubées dans le milieu Dulbecco's modified Eagle (DMEM) enrichi avec 10 % de sérum de veau foetal. Les cellules sont incubées dans des boîtes de 12 puits. Chaque puits contient initialement 100 000 cellules. La croissance cellulaire est réalisée en incubant les cellules dans 2 ml de milieu de culture après addition ou non des différents inhibiteurs potentiels (1mM) et ou de méthylglyoxal (600 µM) pendant 48 heures à 37°C en atmosphère humide et en présence de 5% de CO₂.

L'évaluation du nombre de cellules est réalisée de la façon suivante :
Les cellules sont colorées par la méthode de MTT (bromure de 4,5-diméthyltiazol-2-yl) 2,5-diphényl-tétrazolium). Le MTT pénètre dans la cellule où il est converti en formazan. La quantité de formazan formée est proportionnelle au nombre de cellules vivantes.
100*DO(cellules traitées)/DO(cellules témoins).

Les résultats sont exprimés en pourcentage relatif du nombre de cellules après traitement par rapport au nombre de cellules témoins sans traitement.

[100 x DO (cellules traitées) / DO (cellules témoins)]. La détection est réalisée par spectrophotométrie UV/visible à 570 nm.

Principe : Le MTT(jaune) pénètre dans la cellule et est converti en un composé bleu insoluble le formazan par clivage du noyau tétrazolique par les enzymes deshydrogénase mitochondrial des cellules vivantes. Le formazan est solubilisé par l'isopropanol. Le nombre de cellules est proportionnel à la quantité de formazan formé et a son absorbance

Tel que ceci est illustré sur la figure 6, le méthylglyoxal (MG) supprime la croissance cellulaire.

Les résultats sont rassemblés dans le tableau 2 ci-après.

**Croissance de cellules en présence ou non du MG et de piégeurs du MG**

| Piégeurs du MG | Cellules + Piégeurs du MG (A) | Ecart type | Cellules + Piégeurs du MG + Méthylglioxal (B) | Ecart type | Différence (B)-(A) |
|---|---|---|---|---|---|
| 0 | 100 | 0 | 24 | | |
| Aminoguanidine | 97 | 6 | 92 | 5 | -5 |
| L-DAPA-L-Val.2HCl (exemple 23) | 94 | 8 | 93 | 3 | -1 |
| L-DAPA-L-Leu.2HCl (exemple 1) | 94 | 16 | 81 | 7 | -13 |
| Carnosine | 99 | 0,2 | 50 | 3 | -49 |
| L-DAPA-L-Ile.2HCl (exemple 18) | 86 | 6 | 79 | 4 | -7 |
| (2S,3S)-DASA-L-Val.2HCl (exemple 29) | 85 | 5 | 70 | 9 | -15 |
| L-DABA-L-leu.2HCl (exemple 12) | 77 | 15 | 73 | 9 | -4 |
| L-DAPA-L-Phe.2HCl (exemple 21) | 77 | 6 | 71 | 7 | -6 |
| Metformine | 91 | 4 | 45 | 4 | -46 |
| D-DAPA | 83 | 3 | 68 | 13 | -15 |
| D-DAPA-D-Ala.2HCl (exemple 8) | 77 | 6 | 71 | 7 | -6 |
| L-DAPA-L-(S)-Leu.2HCl (exemple 16) | 71 | | 74 | | +3 |
| L-DAPA-Gly.2HCl (exemple 17) | 54 | 8 | 50 | 10 | -4 |
| (2S,3R)-DASA-Gly.2HCl (exemple 28) | 61 | 8 | 54 | 11 | -7 |
| L-Lys | 81 | | 43 | | -38 |

| | | | | | |
|---|---|---|---|---|---|
| Moyenne de 3 expérimentations | | | | | |

L'addition d'aminoguanidine (AG), un piégeur du MG connu, supprime ce processus de manière spectaculaire. On observe la même tendance avec les composés selon la présente invention en particulier le L-DAPA-L-Val (exemple 23), le L-DAPA-L-Leu (exemple 1), et le L-DAPA-L-Ile (exemple 18). D'autres piégeurs du MG connus, tels que la carnosine et la metformine, se sont avérés moins efficaces dans ce test. Des exemples supplémentaires de l'effet inhibiteur des composés selon la présente invention par rapport à la suppression de la croissance cellulaire par le MG sont illustrés sur la figure 7.

Ces résultats montrent que les composés selon la présente invention ne sont pas toxiques vis-à-vis des cellules EA. Il s'agit en particulier des composés L-DAPA-L-Val-2 HCl (exemple 23), L-DAPA-L-Leu-2HCl (exemple 1), L-DAPA-L-Ile-2 HCl (exemple 18), (2S,3S)-DASA-L-Val-2 HCl (exemple 29), et le L-DAPA-L-Leu-2 TFA (exemple 3) pour lesquels le nombre de cellules est abaissé de moins de 15% par rapport au nombre de cellules témoins croissant sans addition de produit. La composition de la partie diamino n'interviendrait pas dans la toxicité des molécules ainsi que le sel associé à la molécule. En effet, dans les produits toxiques et non toxiques se retrouvent les L-DAPA, (2S,3S)-DASA ou D-DAPA et également les sels HCl ou TFA. On peut noter que la non toxicité des composés entraîne une augmentation du score de leur action de piégeur de MG par rapport aux cellules croissant avec le MG seul. La différence entre les valeurs du nombre de cellules relatives croissant avec le composé analysé et les cellules croissant en présence de MG et du composé analysé permet d'évaluer le rôle piégeur du produit sur MG. Huit composés selon la présente invention possèdent particulièrement cette activité. Il s'agit de L-DAPA-L-Val·2 HCl (-3) (exemple 23), L-DAPA-L-Leu·2HCl (-13) (exemple 1), L-DAPA-L-Ile·2 HCl (-9) (exemple 18), (2S,3S)-DASA-L-Val·2 HCl (-15) (exemple 29), L-DAPA-L-Leu·2 TFA (-18) (exemple 3), L-DABA-L-Leu·2HCl (-4) (exemple 12) et L-DAPA-L-Phe,·2 HCl (-6) (exemple 21). Deux autres composés montrent également une activité de piégeur le D-DAPA-D-Ala·2 HCl (+1) (exemple 8), L-DAPA-Gly·2 HCl(+3) (exemple 17). En revanche, leur toxicité vis-à-vis des cellules est plus importante (39% et 43%). On peut noter le rôle de piégeur faible de la metformine bien que cette molécule ait une toxicité extrêmement faible à cette concentration.

### Test de mutagénicité de deux composés selon la présente invention le L-DAPA-L-Leu (exemple 1) et L-DAPA-L-Val (exemple 23)

Un test de Ames a été réalisé avec le L-DAPA-L-Leu (exemple 1) et le L-DAPA-L-Val (exemple 23) seul et en combinaison avec le méthylglyoxal sur la fraction S9 de foie humain et sur 7 souches de *Salmonella.*

Les concentrations dans le test Ames ont été choisies de la manière suivante : L-DAPA-L-Leu seul ou L-DAPA-L-Val seul : 10 µM, 1 µM et 0,1 µM. Mélange L-DAPA-L-Leu/méthylglyoxal ou mélange L-DAPA-L-Val/méthylglyoxal : 10 µM, 1 µM et 0,1 µM.

Méthylglyoxal : 10 µM.

Les résultats sont rassemblés dans le tableau 3 ci-après.

| Substance de test | Concentrations (µM) | S9 humaine | 6 souches mixtes | Souche TA98 |
|---|---|---|---|---|
| L-DAPA-L-Leu (exemple 1) | 10/1/0,1 | Non | - | - |
| L-DAPA-L-Val (exemple 23) | 10/1/0,1 | Non | - | - |
| L-DAPA-L-Leu exemple 1 : (Métabolites) | 10/1/0,1 | Oui | - | - |
| L-DAPA-L-Val exemple 23 : (Métabolites) | 10/1/0,1 | Oui | - | - |
| L-DAPA-L-Leu (exemple 1) + | 10/1/0,1 | Non | - | - |
| méthylglyoxal | | | | |
| L-DAPA-L-Val (exemple 23) + Méthylglyoxal | 10/1/0,1 | Non | - | - |
| L-DAPA-L-Leu (exemple 1) + méthylglyoxal (métabolites) | 10/1/0,1 | Oui | - | - |
| L-DAPA-L-Val (exemple 23) + méthylglyoxal (Métabolites) | 10/1/0,1 | Oui | - | - |
| Méthylglyoxal | 10 | Non | - | - |
| Méthylglyoxal (métabolites) | 10 | Oui | - | - |
| Témoin positif (4NQO)/2NF) | 2,6/9,5 | Non | + | + |
| Témoin positif (2AA) (métabolites) | 51,7 | Oui | + | + |
| Témoin négatif (Solvant) | - | Non | - | - |
| Témoin négatif (Solvant) | - | Oui | - | - |

| | | | | |
|---|---|---|---|---|
| - : non mutagène + : mutagène | | | | |

La substance de test seule (L-DAPA-L-Leu ou L-DAPA-L-Val) ou en combinaison avec le méthylglyoxal n'était pas mutagène contre TA98 et les souches mixtes aux concentrations testées, ni sur la fraction S9 de foie humain. Les métabolites ont été produits par la fraction S9 de foie humain et n'étaient pas mutagènes aux concentrations testées.

## Revendications

1. Composé de formule générale I suivante 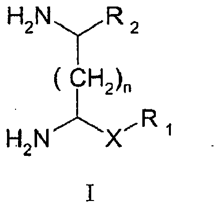 dans laquelle :
X représente CH₂, C=O, C=S ou CHOH, R₁ représente un acide aminé, éventuellement substitué par un ou plusieurs atomes d'halogène, avantageusement de fluor, ou par un ou plusieurs groupes CF₃, et n = 0,1 ou 2
ou XR₁ représentent PO₃H ou SO₃H et n = 0,1 ou 2;
R₂ représente H, XR1, un groupe alkyle en C₁-C₆ un groupe aralkyle en C₁-C₆ ou un groupe aryle, les groupes alkyles, aralkyles et aryles pouvant être substitués par une amine NH₂, un groupe carboxylique COOH, un ou plusieurs atomes d'halogène, avantageusement de fluor, ou un ou plusieurs groupes CF₃ ;
ou ses sels d'addition pharmaceutiquement acceptables, isomères, énantiomères, diastéréoisomères, ainsi que leurs mélanges,
et à l'exception des composés
- pour lesquels R₂ représente un atome d'hydrogène, X représente C=O, R₁ représente -NH-(CH₂)ₘ-COOH et m = 1, 2 ou 3 et n = 0, 1 ou 2 ;
- représentés par les formules suivantes : 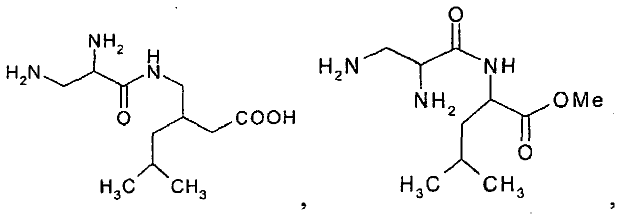 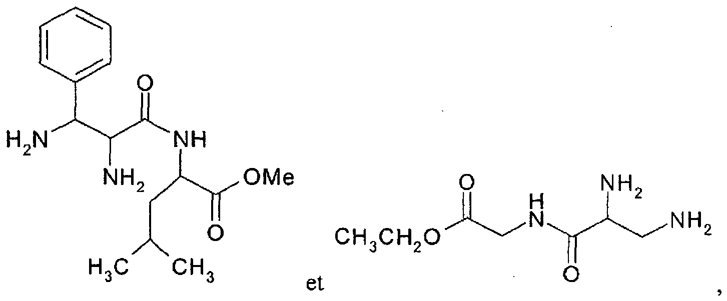 et des composés L-omithyl-taurine, L-diaminobutyryl-taurine et L-diaminopropionyl-taurine.

2. Composé selon la revendication 1 **caractérisé en ce que** X représente C=O, CH₂ ou C=S.

3. Composé selon l'une quelconque des revendications précédentes **caractérisé en ce que** R₂ représente XR₁ ou H.

4. Composé selon l'une quelconque des revendications précédentes **caractérisé en ce que** R₁ représente un acide aminé, avantageusement choisi parmi alanine, valine, isoleucine, proline, leucine, Norleucine, phénylalanine ou tert-leucine et n = 0, 1 ou 2.

5. Composé selon l'une quelconque des revendications précédentes **caractérisé en ce qu'**il est représenté par la formule générale II suivante : 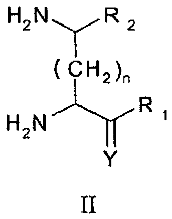 dans laquelle :
R₁ représente -NH-R₃-(C=O)R₄ ou 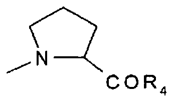
R₃ représente
- un groupe alkyle en C₁-C₁₂, avantageusement en C₁-C₆, éventuellement substitué par un ou plusieurs groupes choisis parmi un atome d'halogène, avantageusement de fluor, un groupe -CF₃, phényle, phénol, -COOH, amine ou phényle substitué par un ou plusieurs atomes d'halogène, avantageusement de fluor, ou par un ou plusieurs groupes CF₃ ;
- un groupe phényle, éventuellement substitué par une amine, un groupe OH, un ou plusieurs atomes d'halogène, avantageusement de fluor, ou un ou plusieurs groupes CF₃ et
R₄ représente OH, NH₂, un alcoxy en C₁-C₃₀, avantageusement en C₁-C₂₀;
R₂ représente H, COR₁, ou un groupe alkyle en C₁-C₆ éventuellement substitué par un ou plusieurs atomes d'halogène, avantageusement de fluor, ou par un ou plusieurs groupes CF₃;
n=0, 1 ou 2 ;
Y représente un atome d'oxygène ou de soufre, avantageusement un atome d'oxygène;
ou ses sels d'addition pharmaceutiquement acceptables, isomères, énantiomères, diastéréoisomères, ainsi que leurs mélanges.

6. Composé selon l'une quelconque des revendications précédentes **caractérisé en ce que** n = 0.

7. Composé selon l'une quelconque des revendications précédentes **caractérisé en ce qu'**il est choisi parmi 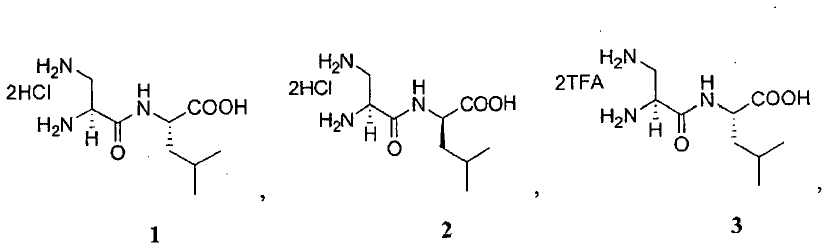 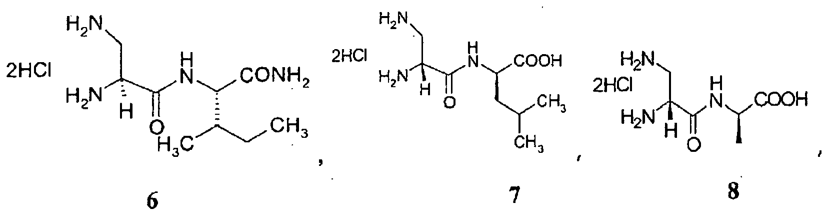 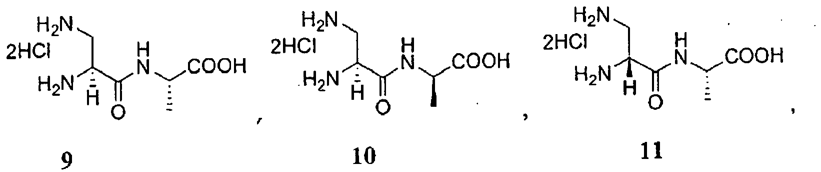 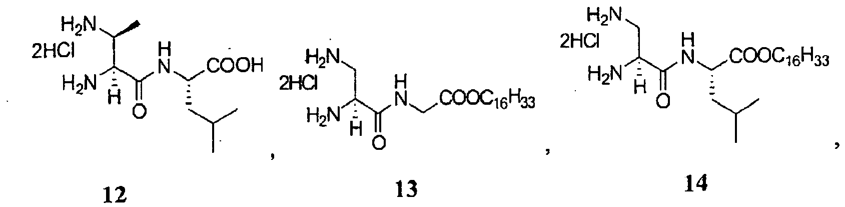 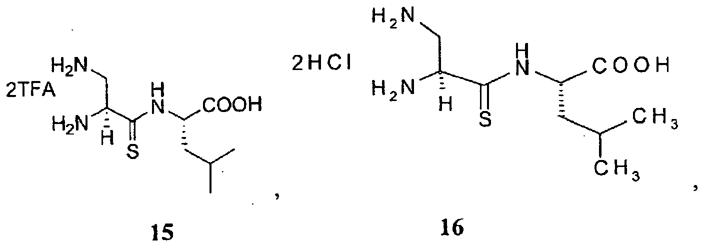 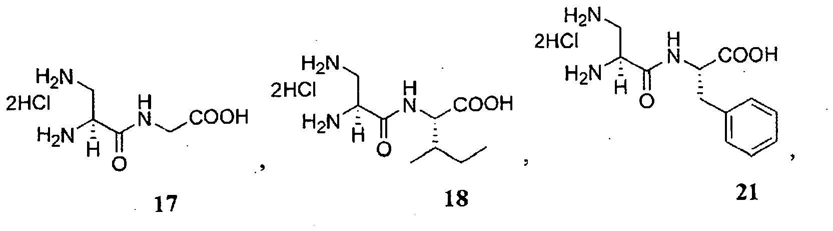 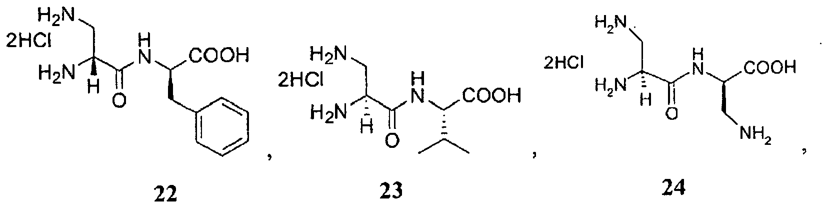 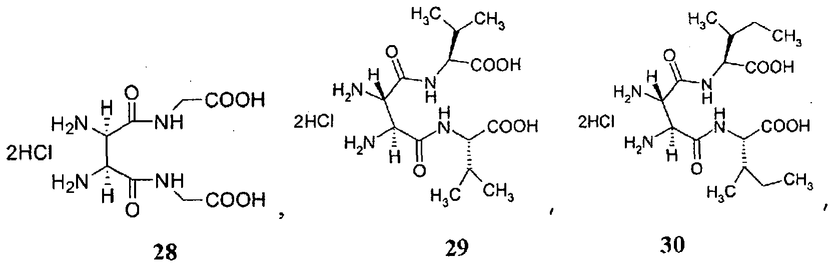 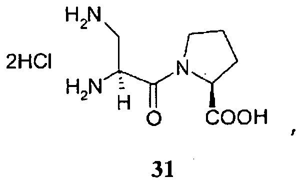 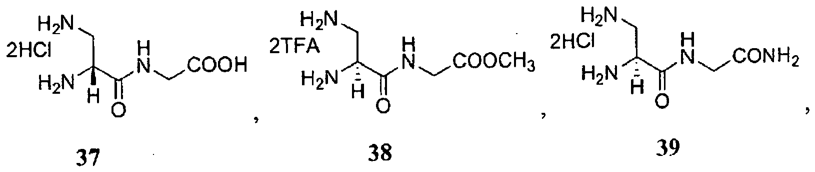 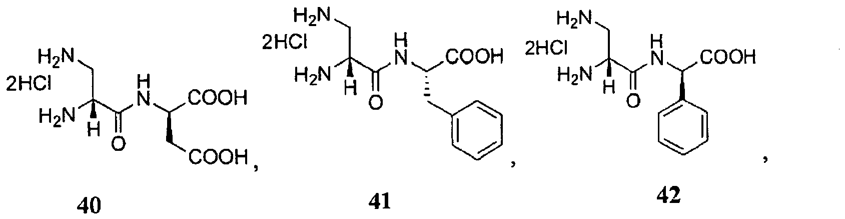 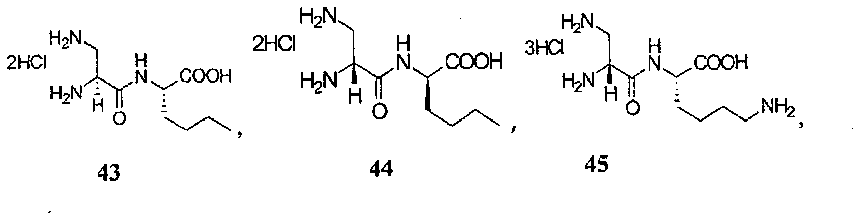 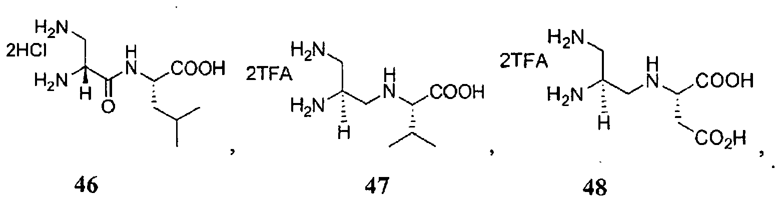 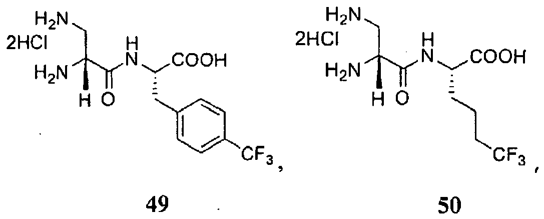 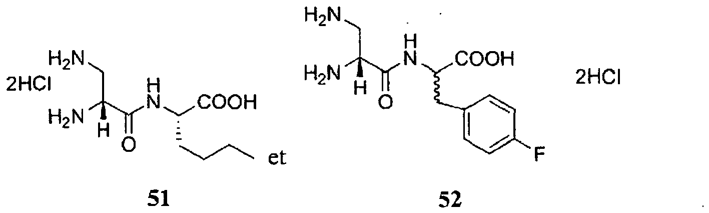 ou ses sels d'addition pharmaceutiquement acceptables, isomères, énantiomères, diastéréoisomères, ainsi que leurs mélanges.

8. Utilisation d'un composé selon l'une quelconque des revendications 1 à 7 pour empêcher l'altération de protéines dans les aliments.

9. Composition pharmaceutique ou cosmétique comprenant un composé selon l'une quelconque des revendications 1 à 7 et un excipient pharmaceutiquement ou cosmétiquement acceptable.

10. Composé de formule générale I suivante 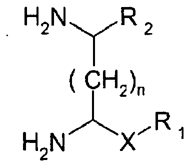 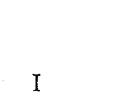 dans laquelle :
X représente CH₂, C=O, C=S ou CHOH, R₁ représente un acide aminé, éventuellement substitué par un ou plusieurs atomes d'halogène, avantageusement de fluor, ou par un ou plusieurs groupes CF₃, et n = 0,1 ou 2
ou XR₁ représentent PO₃H ou SO₃H et n = 0,1 ou 2;
R₂ représente H, XR1, un groupe alkyle en C₁-C₆ un groupe aralkyle en C₁-C₆ ou un groupe aryle, les groupes alkyles, aralkyles et aryles pouvant être substitués par une amine NH₂, un groupe carboxylique COOH, un ou plusieurs atomes d'halogène, avantageusement de fluor, ou un ou plusieurs groupes CF₃ ;
ou ses sels d'addition pharmaceutiquement acceptables, isomères, énantiomères, diastéréoisomères, ainsi que leurs mélanges,
à titre de médicament.

11. Composé selon la revendication 10 **caractérisé en ce qu'**il est représenté par la formule générale II suivante 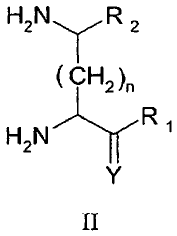 dans laquelle :
R₁ représente NH-R₃-(C=O)R₄ ou 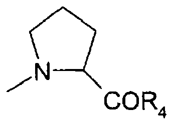
R₃ représente
- un groupe alkyle en C₁-C₁₂, avantageusement en C₁-C₆, éventuellement substitué par un ou plusieurs groupes choisis parmi un atome d'halogène, avantageusement de fluor, un groupe -CF₃, phényle, phénol, -COOH, amine ou phényle substitué par un ou plusieurs atomes d'halogène, avantageusement de fluor, ou par un ou plusieurs groupes CF₃ ;
- un groupe phényle, éventuellement substitué par une amine, un groupe OH, un ou plusieurs atomes d'halogène, avantageusement de fluor, ou un ou plusieurs groupes CF₃ et
R₄ représente OH, NH₂, un alcoxy en C₁-C₃₀, avantageusement en C₁-C₂₀;
R₂ représente H, COR₁, ou un groupe alkyle en C₁-C₆ éventuellement substitué par un ou plusieurs atomes d'halogène, avantageusement de fluor, ou par un ou plusieurs groupes CF₃;
n=0, 1 ou 2 ;
Y représente un atome d'oxygène ou de soufre, avantageusement un atome d'oxygène;
ou ses sels d'addition pharmaceutiquement acceptables, isomères, énantiomères, diastéréoisomères, ainsi que leurs mélanges.

12. Composé selon la revendication 10 ou 11 **caractérisé en ce qu'**il est choisi parmi 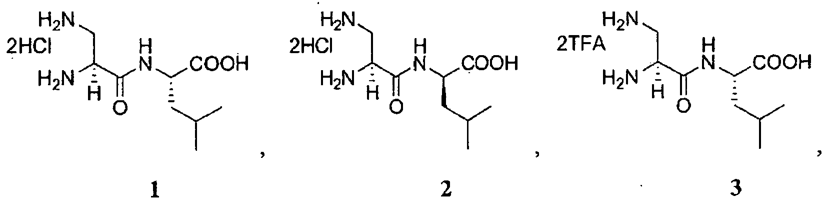 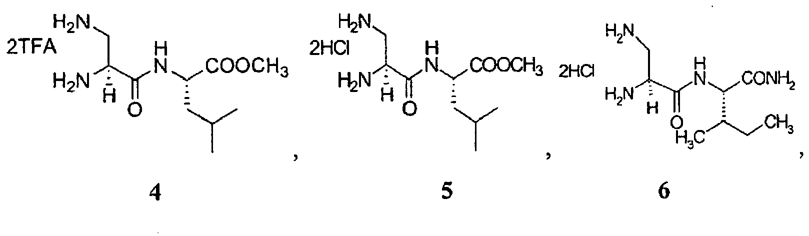 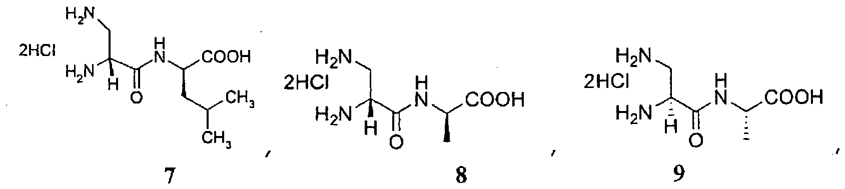 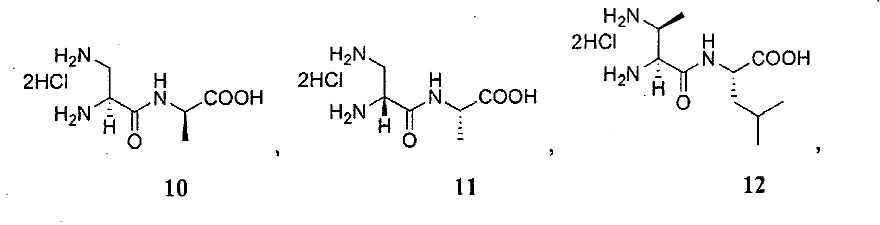 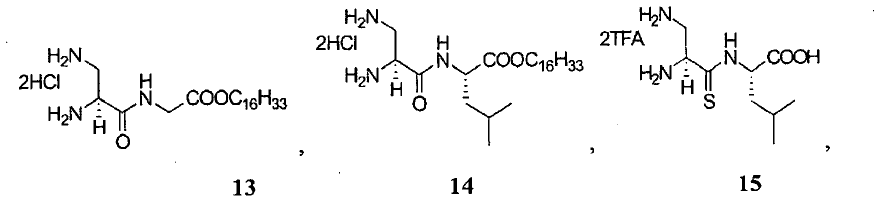 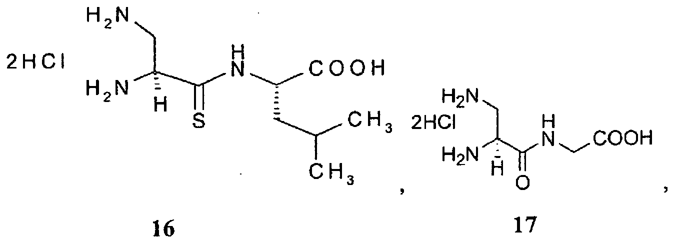 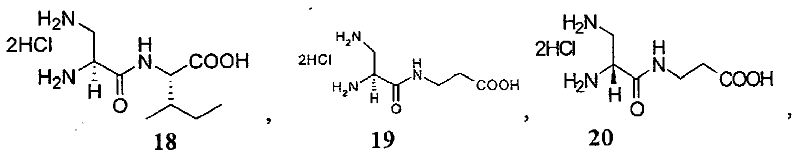 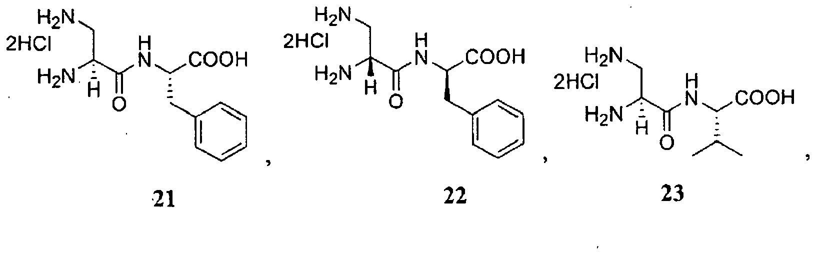 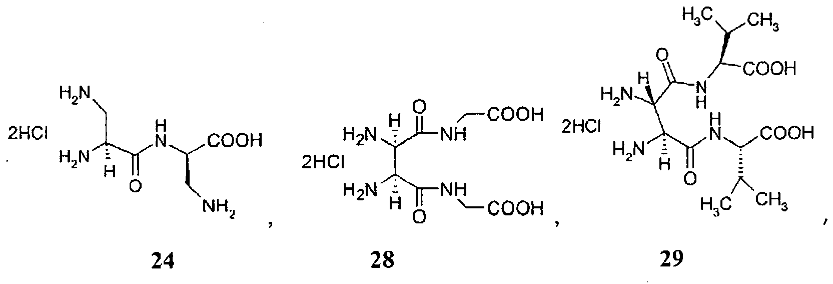 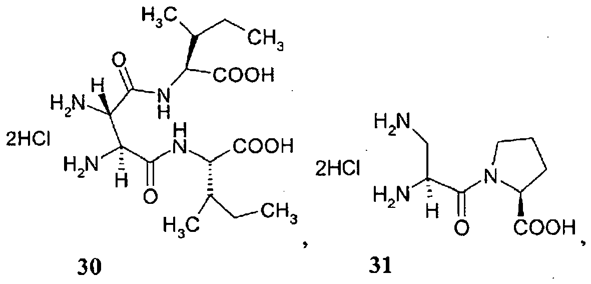 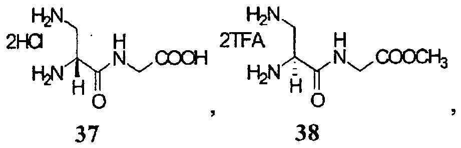 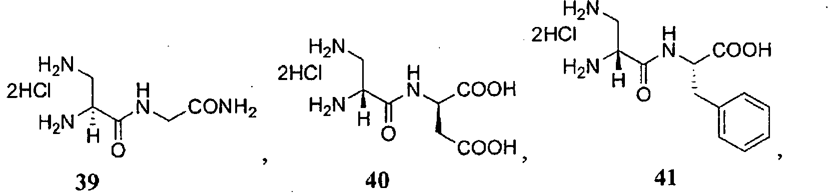 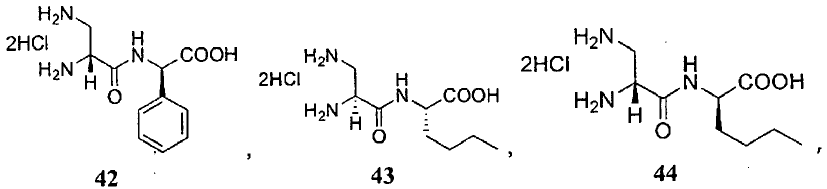 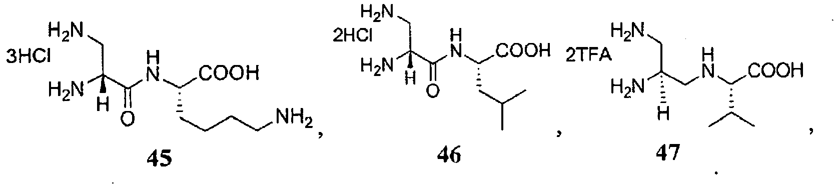 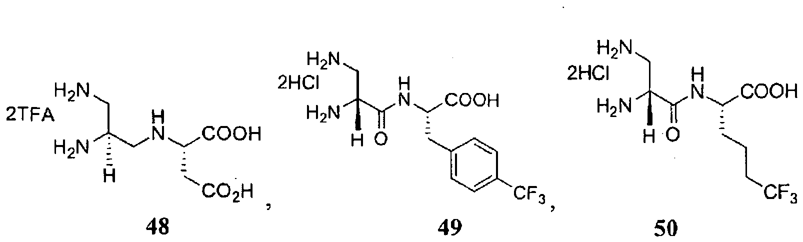 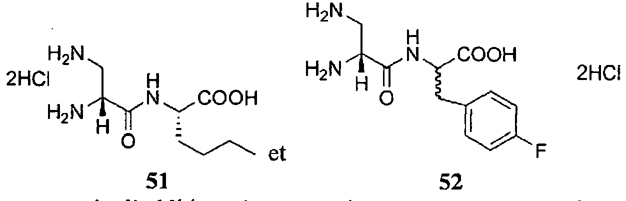 ou ses sels d'addition pharmaceutiquement acceptables, isomères, énantiomères, diastéréoisomères, ainsi que leurs mélanges.

13. Composé selon les revendications 10 à 12 **caractérisé en ce que** le médicament est un piégeur de composés carbonyle réactifs, avantageusement un inhibiteur de formation de produits de glycation avancée.

14. Composé selon les revendications 10 à 13 **caractérisé en ce que** le médicament est destiné à la prévention et/ou au traitement d'un état ou de maladies du à la formation de produits de glycation avancée ou à la réticulation de protéines, à la prévention et/ou au traitement des effets délétères du vieillissement d'un organisme, lesdits effets étant la formation de produits de glycation avancée ou la réticulation de protéines, ou au ralentissement ou à l'arrêt de la progression chez un patient de complications résultant d'un diabète, lesdites complications résultant de la formation de produits de glycation avancée ou de la réticulation de protéines.

15. Composé selon l'une quelconque des revendications 10 à 14 **caractérisé en ce que** le médicament est destiné à traiter, prévenir et/ou ralentir la progression chez un patient de maladies choisies parmi la polyarthrite rhumatoïde, la maladie d'Alzheimer, l'urémie, les maladies neurodégénératives, l'athérosclérose, les complications micro et macrovasculaires du diabète dont la rétinopathie diabétique et l'insuffisance rénale due à la néphropathie diabétique, les micro et macroangiopathies, la cataracte, l'amyloïdose associée à la dialyse ou à la maladie d'Alzheimer, la maladie de Parkinson, les gingivites, les caries, les problèmes bucco-dentaires, l'ulcère diabétique, l'insuffisance rénale chronique, la dialyse rénale chronique, les maladies inflammatoires, les troubles rhumatismaux liés à l'âge et la porphyrie et à traiter les cancers de stade précoce.

16. Composés selon l'une quelconque des revendications 10 à 15 **caractérisé en ce que** le médicament est destiné à une administration par voie orale.

17. Utilisation cosmétique d'un composé selon l'une quelconque des revendications 1 à 7 en tant qu'actif antivieillissement et restructurant de l'épiderme et du derme papillaire et/ou en tant qu'actif antirides.

## Claims

1. A compound of the following general formula I: 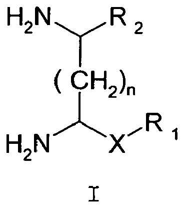 wherein:
X represents CH₂, C=O, C=S or CHOH, R₁ represents an amino acid, optionally substituted by one or more halogen atoms, advantageously fluorine, or by one or more CF₃ groups, and n=0, 1 or 2
or XR₁ represents PO₃H or SO₃H and n=0, 1 or 2;
R₂ represents H, XR₁, a C₁-C₆ alkyl group, a C₁-C₆ aralkyl group or an aryl group, the alkyl, aralkyl and aryl groups being able to be substituted by an amine (NH₂), a carboxylic group (COOH), one or more halogen atoms, advantageously fluorine, or one or more CF₃ groups;
or the pharmaceutically acceptable addition salts, isomers, enantiomers and diastereoisomers of same, as well as mixtures of same,
with the exception of compounds
- wherein R₂ represents a hydrogen atom, X represents C=O, R₁ represents -NH-(CH₂)ₘ-COOH and m=1, 2 or 3 and n=0, 1 or 2;
- represented by the following formulas: 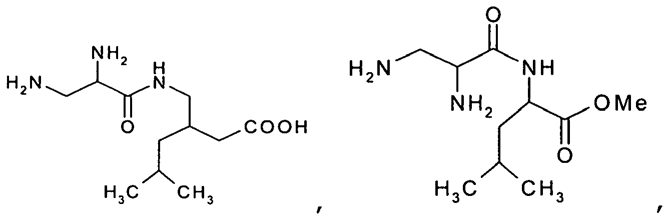 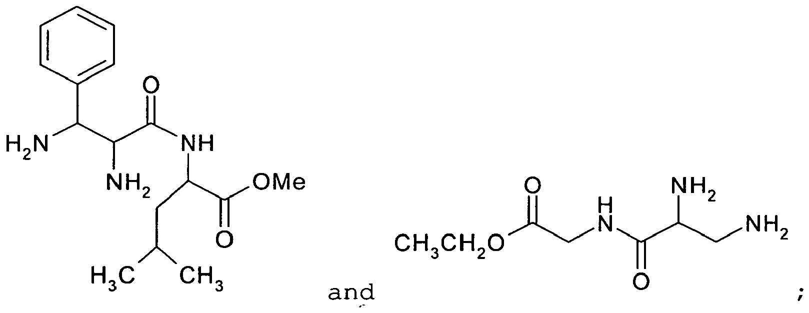
and the compounds L-ornithyl-taurine, L-diaminobutyryl-taurine and L-diaminopropionyl taurine.

2. A compound according to claim 1, wherein X represents C=O, CH₂ or C=S.

3. A compound according to any of the preceding claims, wherein R₂ represents XR₁ or H.

4. A compound according to any of the preceding claims, wherein R₁ represents an amino acid, advantageously selected among alanine, valine, isoleucine, proline, leucine, norleucine, phenylalanine or *tert*-leucine, and n=0, 1 or 2.

5. A compound according to any of the preceding claims, wherein said compound is represented by the following general formula II: 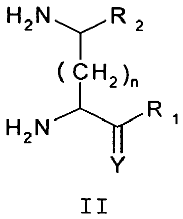 wherein:
R₁ represents -NH-R₃-(C=O)R₄ or 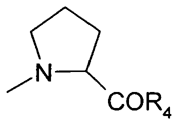
R₃ represents
- a C₁-C₁₂ alkyl group, advantageously C₁-C₆, optionally substituted by one or more groups chosen among a halogen atom, advantageously fluorine, a -CF₃, phenyl, phenol, -COOH, amine or phenyl group substituted by one or more halogen atoms, advantageously fluorine, or by one or more CF₃ groups;
- a phenyl group, optionally substituted by an amine, an OH group, one or more halogen atoms, advantageously fluorine, or one or more CF₃ groups and
R₄ represents OH, NH₂, a C₁-C₃₀ alkoxy, advantageously C₁-C₂₀;
R₂ represents H, COR₁, or a C₁-C₆ alkyl group optionally substituted by one or more halogen atoms, advantageously fluorine, or by one or more CF₃ groups; n=0, 1 or 2;
Y represents an oxygen or sulfur atom, advantageously an oxygen atom;
or the pharmaceutically acceptable addition salts, isomers, enantiomers and diastereoisomers of same, as well as mixtures of same.

6. A compound according to any of the preceding claims, wherein n=0.

7. A compound according to any of the preceding claims, wherein said compound is selected among 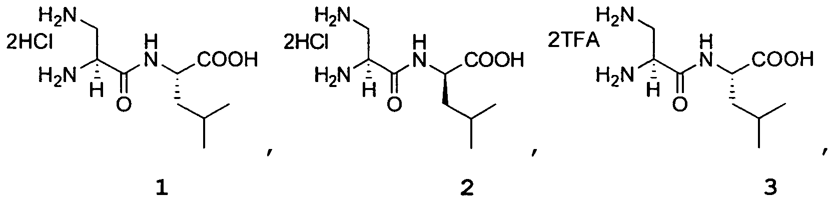 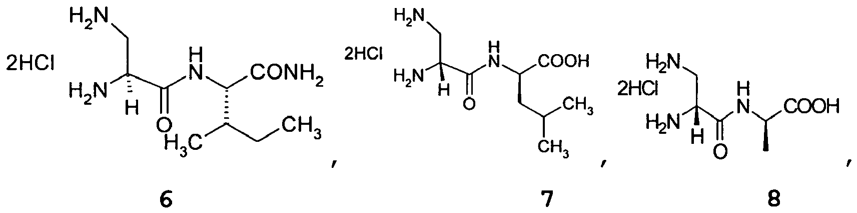 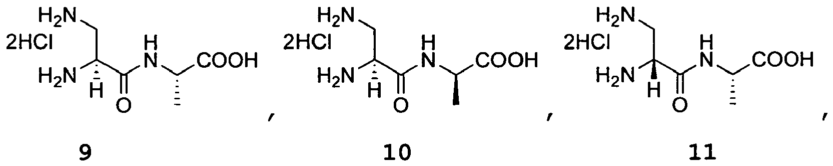 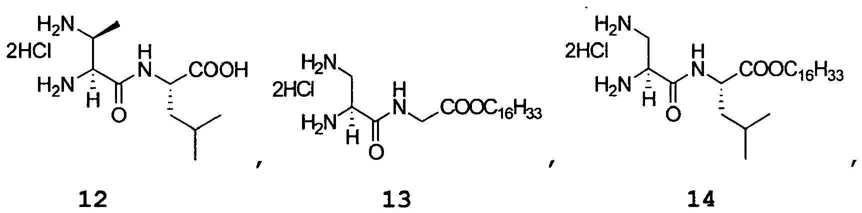 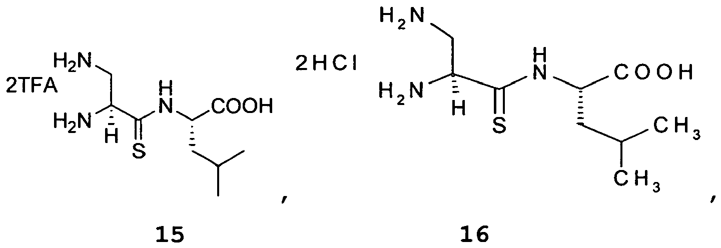 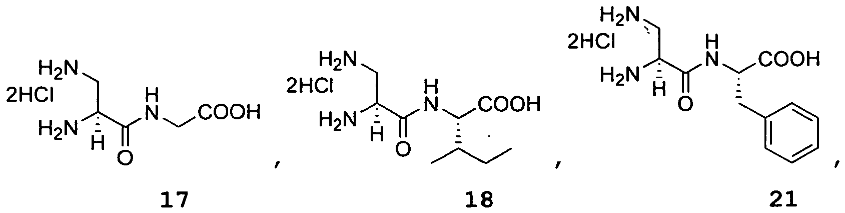 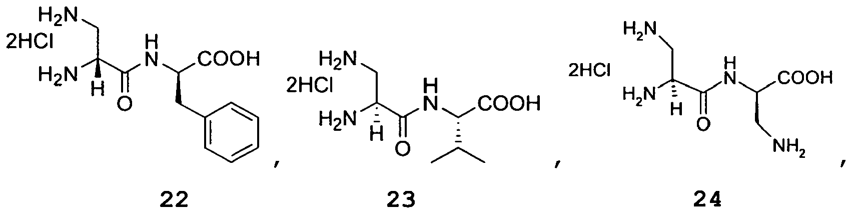 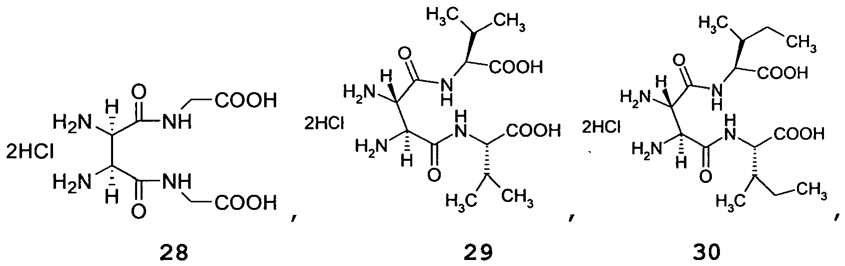 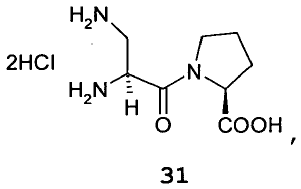 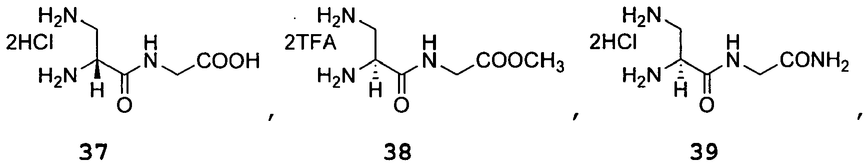 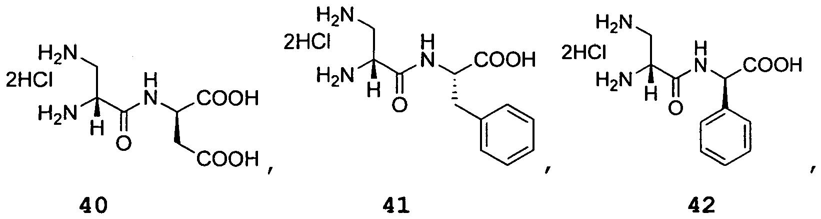 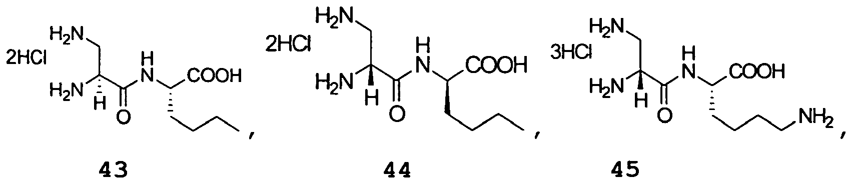 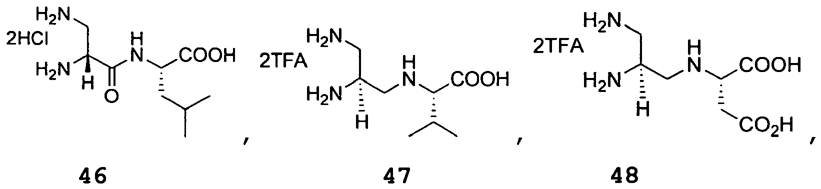 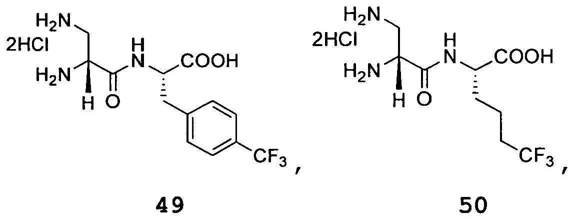 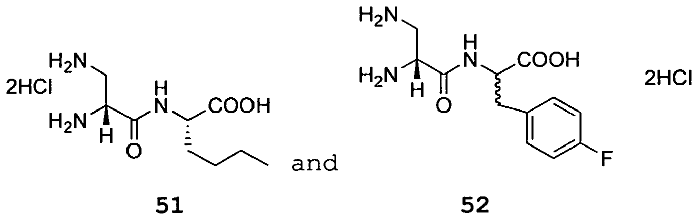 or the pharmaceutically acceptable addition salts, isomers, enantiomers and diastereoisomers of same, as well as mixtures of same.

8. The use of a compound according to any of the claims 1 to 7, to prevent the deterioration of proteins in foods.

9. A pharmaceutical or cosmetic composition comprising a compound according to any of the claims 1 to 7 and a pharmaceutically or cosmetically acceptable excipient.

10. A compound of following general, formula I: 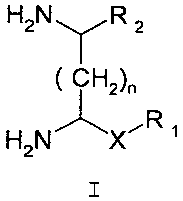 wherein:
X represents CH₂, C=O, C=S or CHOH, R₁ represents an amino acid, optionally substituted by one or more halogen atoms, advantageously fluorine, or by one or more CF₃ groups, and n=0, 1 or 2
or XR₁ represents PO₃H or SO₃H and n=0, 1 or 2;
R₂ represents H, XR₁, a C₁-C₆ alkyl group, a C₁-C₆ aralkyl group or an aryl group, the alkyl, aralkyl and aryl groups being able to be substituted by an amine (NH₂), a carboxylic group (COOH), one or more halogen atoms, advantageously fluorine, or one or more CF₃ groups;
or the pharmaceutically acceptable addition salts, isomers, enantiomers and diastereoisomers of same, as well as mixtures of same,
for use as a drug.

11. A compound according to claim 10, wherein said compound is represented by the following general formula II: 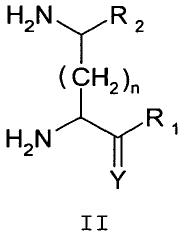 wherein:
R₁ represents NH-R₃-(C=O)R₄ or 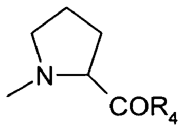
R₃ represents
- a C₁-C₁₂ alkyl group, advantageously C₁-C₆, optionally substituted by one or more groups chosen among a halogen atom, advantageously fluorine, a -CF₃, phenyl, phenol, -COOH, amine or phenyl group substituted by one or more halogen atoms, advantageously fluorine, or by one or more CF₃ groups;
- a phenyl group, optionally substituted by an amine, an OH group, one or more halogen atoms, advantageously fluorine, or one or more CF₃ groups and
R₄ represents OH, NH₂, a C₁-C₃₀ alkoxy, advantageously C₁-C₂₀;
R₂ represents H, COR₁, or a C₁-C₆ alkyl group optionally substituted by one or more halogen atoms, advantageously fluorine, or by one or more CF₃ groups; n=0, 1 or 2;
Y represents an oxygen or sulfur atom, advantageously an oxygen atom;
or the pharmaceutically acceptable addition salts, isomers, enantiomers and diastereoisomers of same, as well as mixtures of same.

12. A compound according to claim 10 or claim 11, wherein said compound is selected among 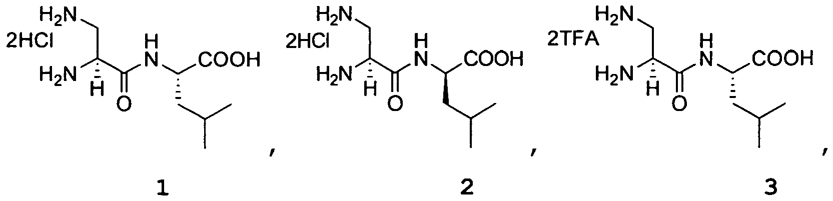 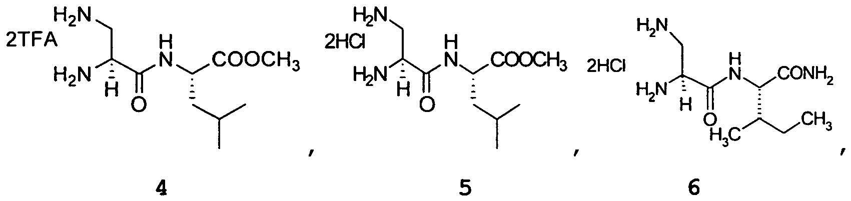 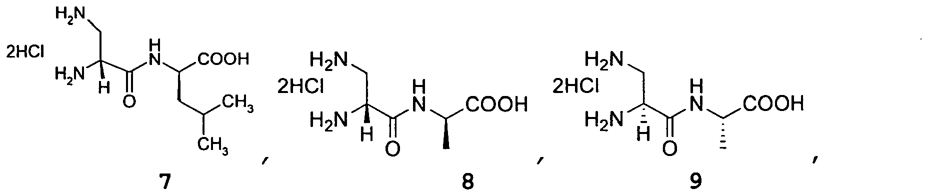 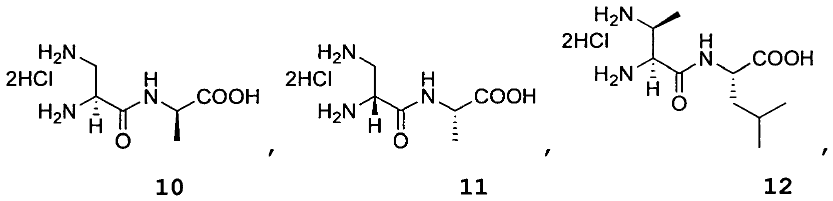 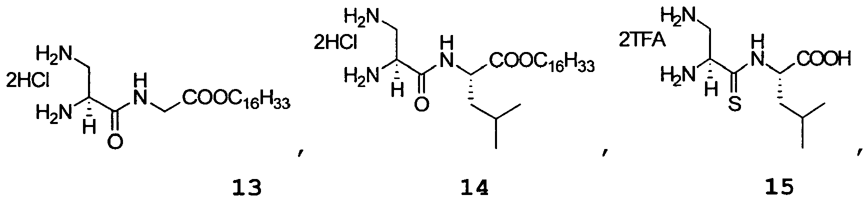 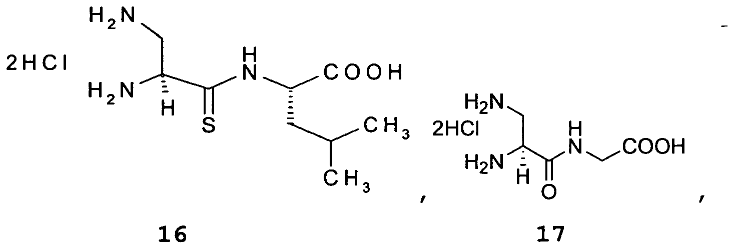 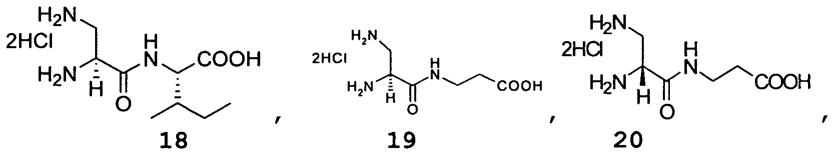 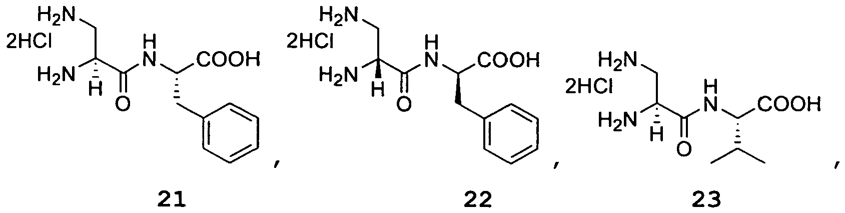 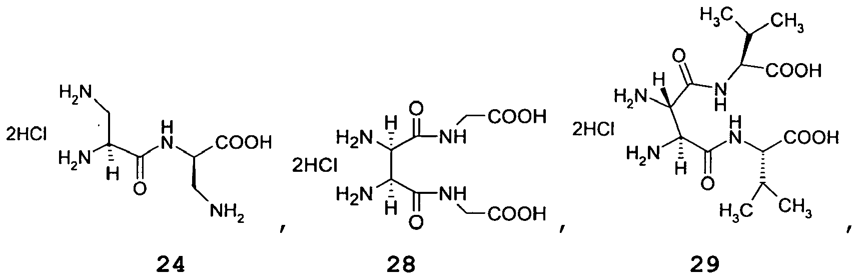 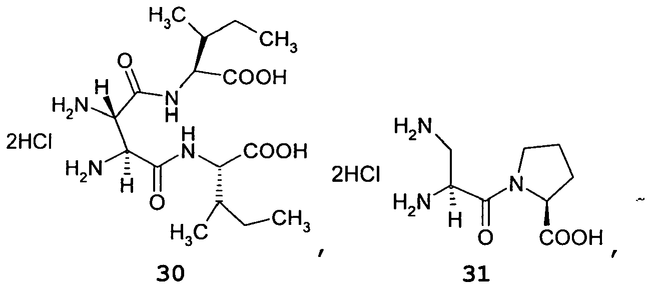 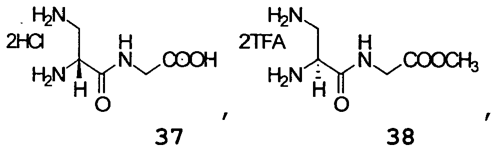 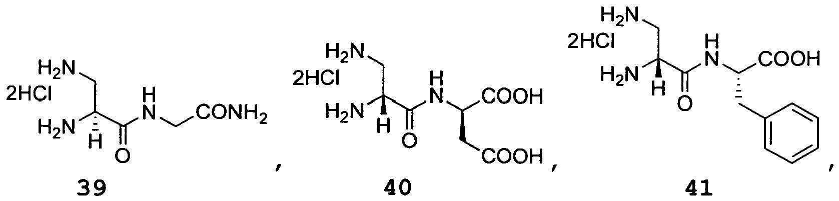 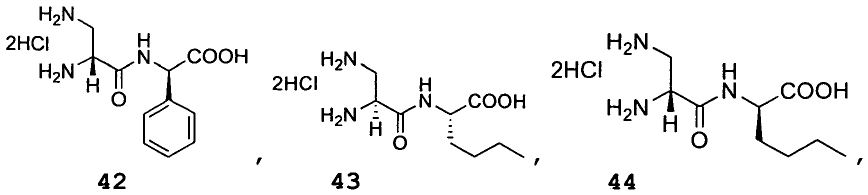 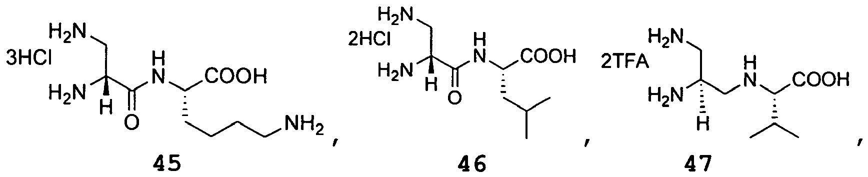 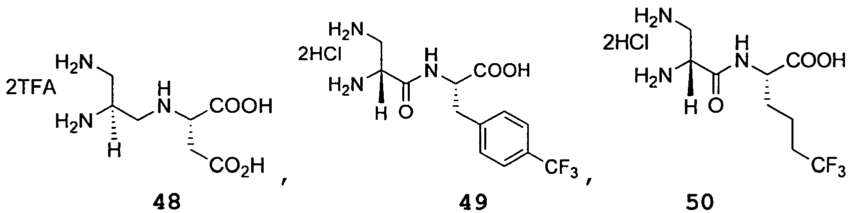 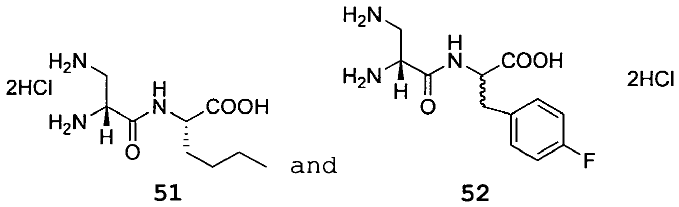 or the pharmaceutically acceptable addition salts, isomers, enantiomers and diastereoisomers of same, as well as mixtures of same.

13. A compound according to the claims 10 to 12, wherein the drug is a scavenger of reactive carbonyl compounds, advantageously an inhibitor of the formation of advanced glycation end-products.

14. A compound according to the claims 10 to 13, wherein the drug is for the prevention and/or the treatment of a state or disease due to the formation of advanced glycation end-products or to the cross-linking of proteins, for the prevention and/or the treatment of the deleterious effects of the ageing of an organism, said effects being the formation of advanced glycation end-products or the cross-linking of proteins, or in a patient for the slowing or the stopping of the progression of complications resulting from diabetes, said complications resulting from the formation of advanced glycation end-products or from the cross-linking of proteins.

15. A compound according to any of the claims 10 to 14, wherein the drug is intended to treat, prevent and/or slow in a patient the progression of diseases chosen among rheumatoid polyarthritis, Alzheimer's disease, uremia, neurodegenerative diseases, atherosclerosis, microvascular and macrovascular complications of diabetes including diabetic retinopathy and renal failure due to diabetic nephropathy, microangiopathies and macroangiopathies, cataracts, amyloidosis associated with dialysis or with Alzheimer's disease, Parkinson's disease, gingivitis, cavities, bucco-dental problems, diabetic ulcers, chronic renal failure, chronic renal dialysis, inflammatory diseases, age-related rheumatic disorders and porphyria and to treat early-stage cancers.

16. Compounds according to any of the claims 10 to 15, wherein the drug is intended for administration by oral route.

17. The cosmetic use of a compound according to any of the claims 1 to 7 as an anti-ageing and restructuring active ingredient for the epidermis and the papillary dermis and/or as an anti-wrinkle active ingredient.

## Patentansprüche

1. Verbindung gemäß der folgenden allgemeinen Formel I 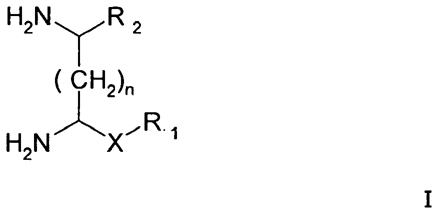 wobei:
X CH₂, C=O, C=S oder CHOH repräsentiert, R₁ eine Aminosäure repräsentiert, die gegebenenfalls durch ein oder mehrere Halogenatome, bevorzugt Fluoratome, oder durch eine oder mehrere CF₃-Gruppen substituiert ist, und n = 0, 1 oder 2 ist,
oder XR₁ PO₃H oder SO₃H repräsentiert und n = 0, 1 oder 2 ist;
R₂ H, XR₁, eine C₁-C₆-Alkylgruppe, eine C₁-C₆-Aralkylgruppe oder eine Arylgruppe repräsentiert, wobei die Alkyl-, Aralkyl- und Arylgruppen durch ein Amin NH₂, eine Carboxylgruppe COOH, ein oder mehrere Halogenatome, bevorzugt Fluoratome, oder durch eine oder mehrere CF₃-Gruppen substituiert sein können;
oder ihre pharmazeutisch akzeptablen Additionssalze, Isomere, Enantiomere, Diastereoisomere sowie deren Mischungen,
und mit Ausnahme der Verbindungen
- für die R₂ ein Wasserstoffatom repräsentiert, X C=O repräsentiert, R₁ -NH-(CH₂)ₘ-COOH repräsentiert und m = 1, 2 oder 3 ist und n = 0, 1 oder 2 ist;
- repräsentiert durch die folgenden Formeln: 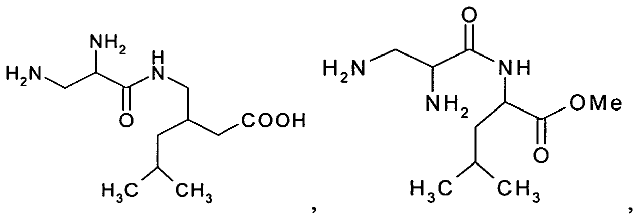 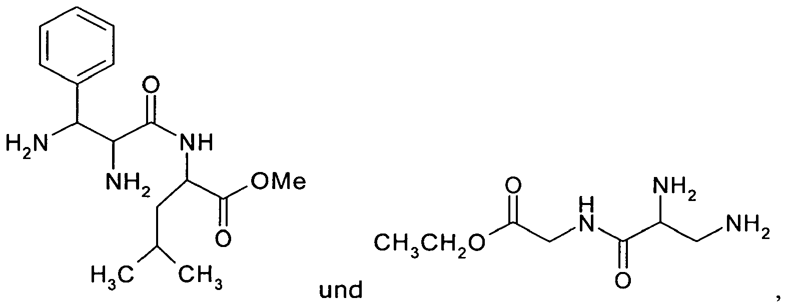 und der Verbindungen L-Ornithyl-taurin, L-Diaminobutyryl-taurin und L-Diaminopropionyl-taurin.

2. Verbindung nach Anspruch 1, **dadurch gekennzeichnet, dass** X C=O, CH₂ oder C=S repräsentiert.

3. Verbindung nach einem der vorangehenden Ansprüche, **dadurch gekennzeichnet, dass** R₂ XR₁ oder H repräsentiert.

4. Verbindung nach einem der vorangehenden Ansprüche, **dadurch gekennzeichnet, dass** R₁ eine Aminosäure repräsentiert, bevorzugt ausgewählt aus Alanin, Valin, Isoleucin, Prolin, Leucin, Norleucin, Phenylalanin oder tert-Leucin, und n = 0, 1 oder 2 ist.

5. Verbindung nach einem der vorangehenden Ansprüche, **dadurch gekennzeichnet, dass** sie durch folgende allgemeine Formel II repräsentiert wird: 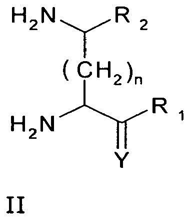 wobei:
R₁ -NH-R₃-(C=O)R₄ oder 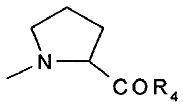 repräsentiert;
R₃
- eine C₁-C₁₂-Alkylgruppe repräsentiert, bevorzugt eine C₁-C₆-Alkylgruppe, gegebenenfalls substituiert durch eine oder mehrere Gruppen ausgewählt aus einem Halogenatom, bevorzugt einem Fluoratom, einer CF₃-Gruppe, Phenyl, Phenol, -COOH, Amin oder Phenyl substituiert durch ein oder mehrere Halogenatome, bevorzugt Fluoratome, oder durch eine oder mehrere CF₃-Gruppen;
- eine Phenylgruppe repräsentiert, gegebenenfalls substituiert durch ein Amin, eine OH-Gruppe, eine oder mehrere Halogenatome, bevorzugt Fluoratome, oder eine oder mehrere CF₃-Gruppen, und
R₄ OH, NH₂ oder ein C₁-C₃₀-Alkoxy, bevorzugt ein C₁-C₂₀-Alkoxy, repräsentiert;
R₂ H, COR₁ oder eine C₁-C₆-Alkylgruppe repräsentiert, gegebenenfalls substituiert durch ein oder mehrere Halogenatome, bevorzugt Fluoratome, oder durch eine oder mehrere CF₃-Gruppen;
n = 0, 1 oder 2 ist;
Y ein Sauerstoff- oder ein Schwefelatom repräsentiert, bevorzugt ein Sauerstoffatom;
oder ihre pharmazeutisch akzeptablen Additionssalze, Isomere, Enantiomere, Diastereoisomere sowie deren Mischungen.

6. Verbindung nach einem der vorangehenden Ansprüche, **dadurch gekennzeichnet, dass** n = 0 ist.

7. Verbindung nach einem der vorangehenden Ansprüche, **dadurch gekennzeichnet, dass** sie ausgewählt ist aus 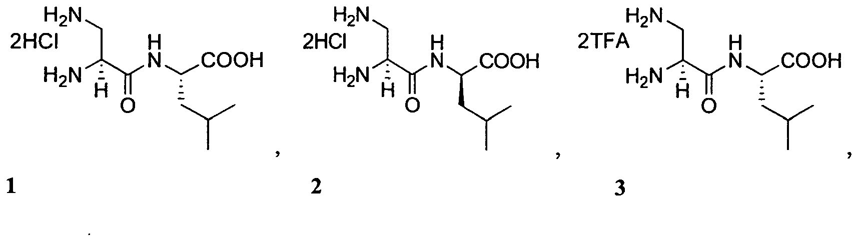 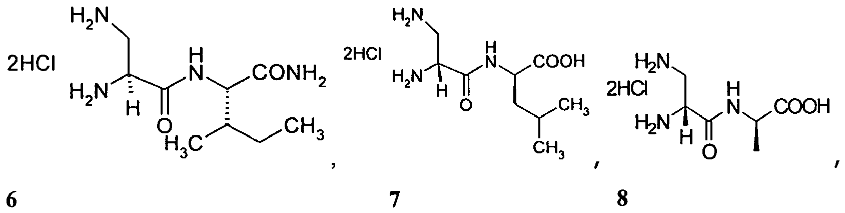 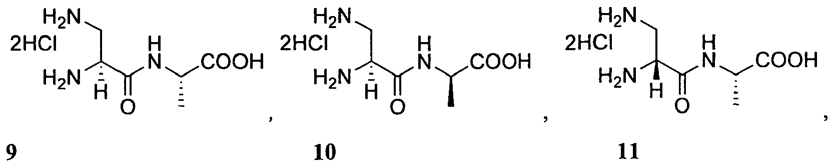 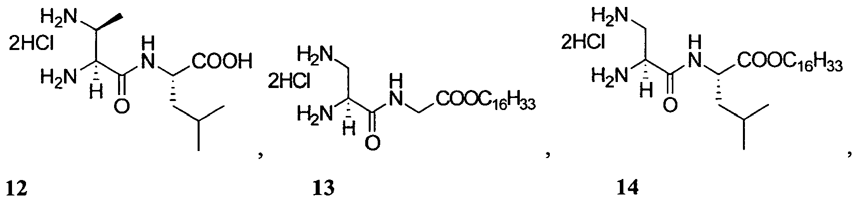 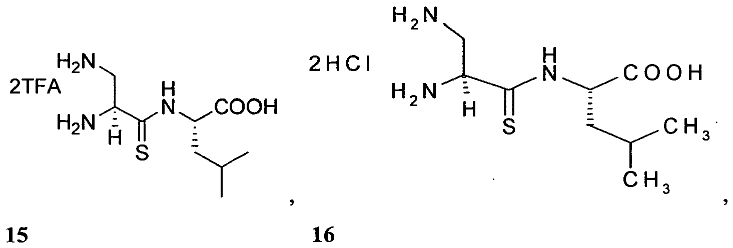 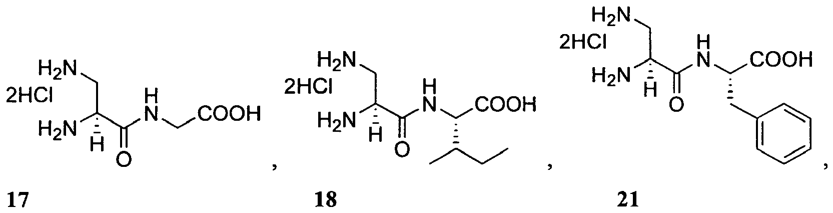 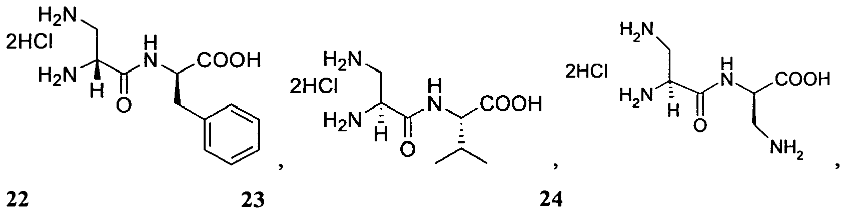 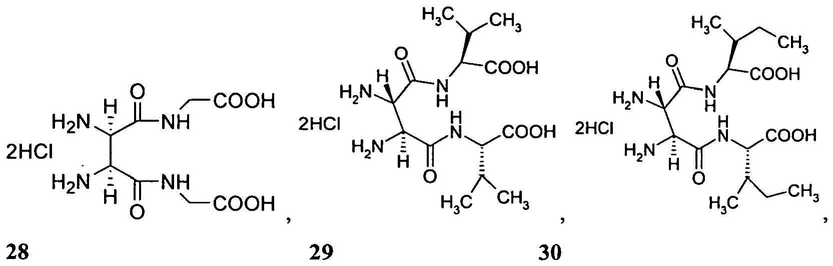 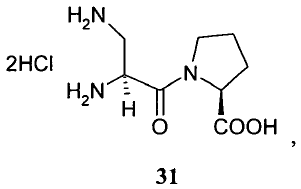 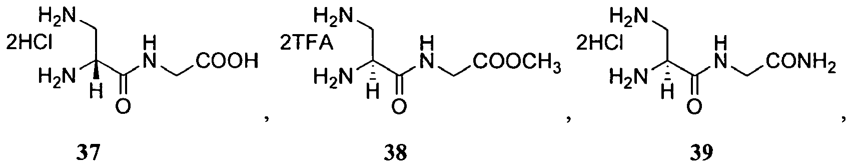 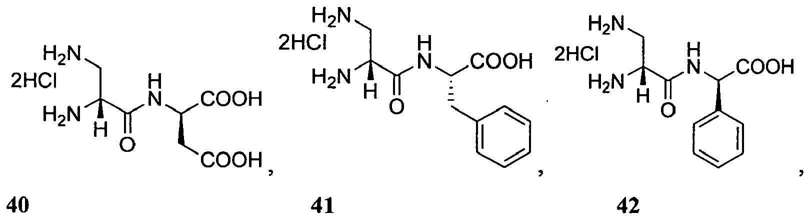 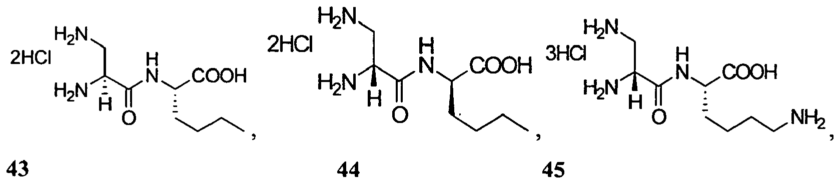 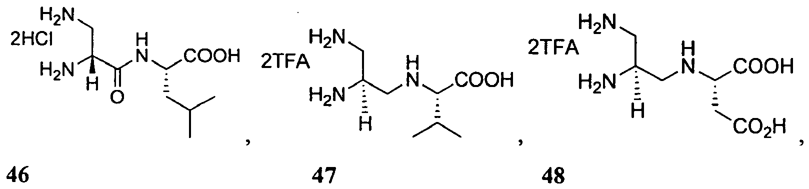 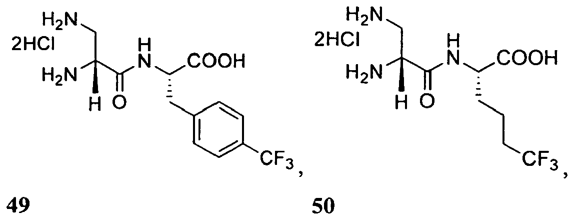 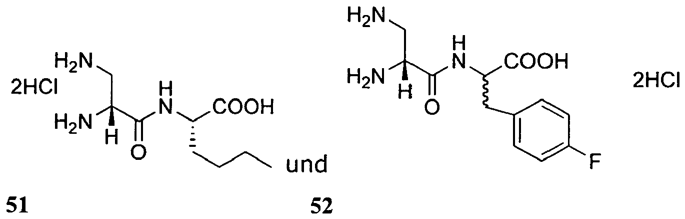 oder ihren pharmazeutisch akzeptablen Additionssalzen, Isomeren, Enantiomeren, Diastereoisomeren sowie deren Mischungen.

8. Verwendung einer Verbindung nach einem der Ansprüche 1 bis 7 zum Verhindern der Veränderung von Proteinen in Lebensmitteln.

9. Pharmazeutische oder kosmetische Zusammensetzung, umfassend eine Verbindung nach einem der Ansprüche 1 bis 7 und einen pharmazeutisch oder kosmetisch akzeptablen Träger.

10. Verbindung gemäß der folgenden allgemeinen Formel I 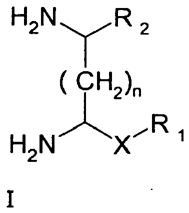 wobei:
X CH₂, C=O, C=S oder CHOH repräsentiert, R₁ eine Aminosäure repräsentiert, die gegebenenfalls durch ein oder mehrere Halogenatome, bevorzugt Fluoratome, oder durch eine oder mehrere CF₃-Gruppen substituiert ist, und n = 0, 1 oder 2 ist,
oder XR₁ PO₃H oder SO₃H repräsentiert und n = 0, 1 oder 2 ist;
R₂ H, XR₁, eine C₁-C₆-Alkylgruppe, eine C₁-C₆-Aralkylgruppe oder eine Arylgruppe repräsentiert, wobei die Alkyl-, Aralkyl- und Arylgruppen durch ein Amin NH₂, eine Carboxylgruppe COOH, ein oder mehrere Halogenatome, bevorzugt Fluoratome, oder durch eine oder mehrere CF₃-Gruppen substituiert sein können;
oder ihre pharmazeutisch akzeptablen Additionssalze, Isomere, Enantiomere, Diastereoisomere sowie deren Mischungen,
als Medikament.

11. Verbindung nach Anspruch 10, **dadurch gekennzeichnet, dass** sie durch die folgende allgemeine Formel II repräsentiert wird: 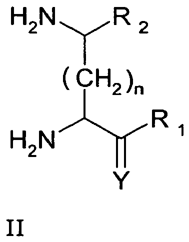 wobei:
R₁ -NH-R₃-(C=O)R₄ oder 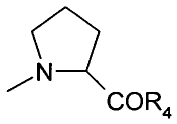 repräsentiert;
R₃
- eine C₁-C₁₂-Alkylgruppe repräsentiert, bevorzugt eine C₁-C₆-Alkylgruppe, gegebenenfalls substituiert durch eine oder mehrere Gruppen ausgewählt aus einem Halogenatom, bevorzugt einem Fluoratom, einer CF₃-Gruppe, Phenyl, Phenol, -COOH, Amin oder Phenyl substituiert durch ein oder mehrere Halogenatome, bevorzugt Fluoratome, oder durch eine oder mehrere CF₃-Gruppen;
- eine Phenylgruppe repräsentiert, gegebenenfalls substituiert durch ein Amin, eine OH-Gruppe, ein oder mehrere Halogenatome, bevorzugt Fluoratome, oder eine oder mehrere CF₃-Gruppen, und
R₄ OH, NH₂ oder ein C₁-C₃₀-Alkoxy, bevorzugt ein C₁-C₂₀-Alkoxy, repräsentiert;
R₂ H, COR₁ oder eine C₁-C₆-Alkylgruppe repräsentiert, gegebenenfalls substituiert durch ein oder mehrere Halogenatome, bevorzugt Fluoratome, oder durch eine oder mehrere CF₃-Gruppen;
n = 0, 1 oder 2 ist;
Y ein Sauerstoff- oder ein Schwefelatom repräsentiert, bevorzugt ein Sauerstoffatom;
oder ihre pharmazeutisch akzeptablen Additionssalze, Isomere, Enantiomere, Diastereoisomere sowie deren Mischungen.

12. Verbindung nach Anspruch 10 oder 11, **dadurch gekennzeichnet, dass** sie ausgewählt ist aus 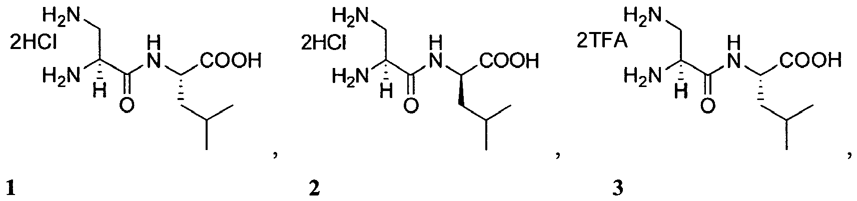 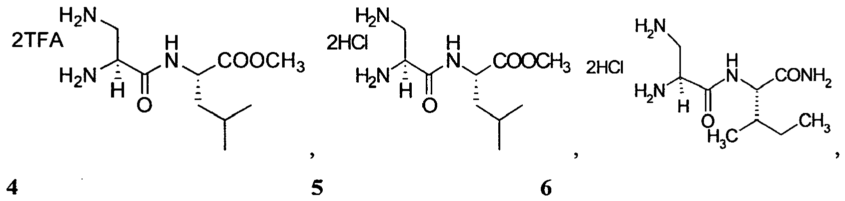 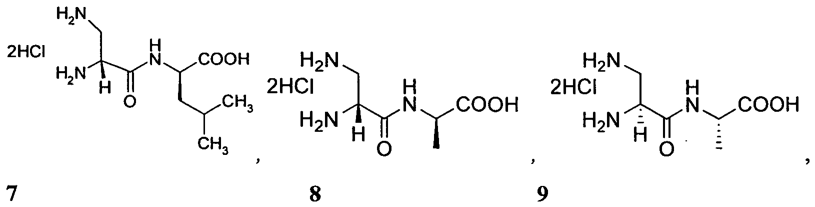 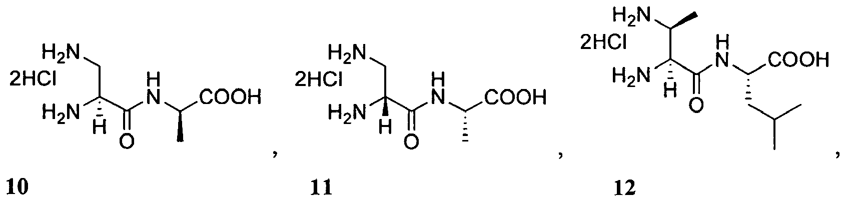 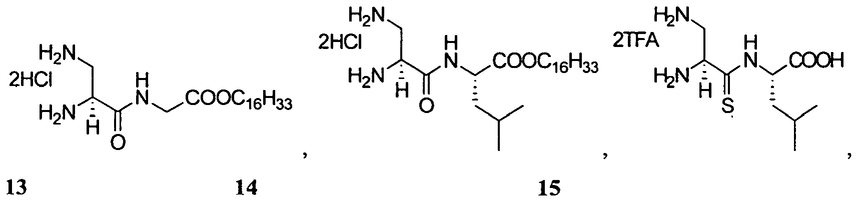 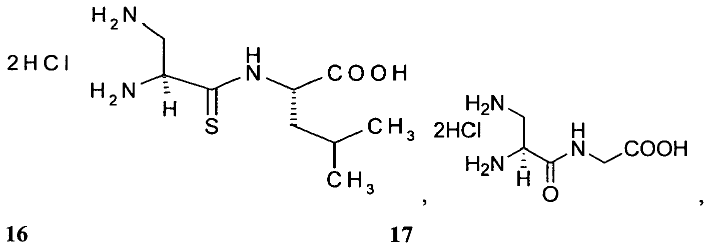 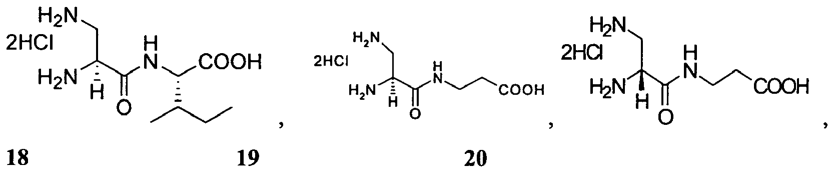 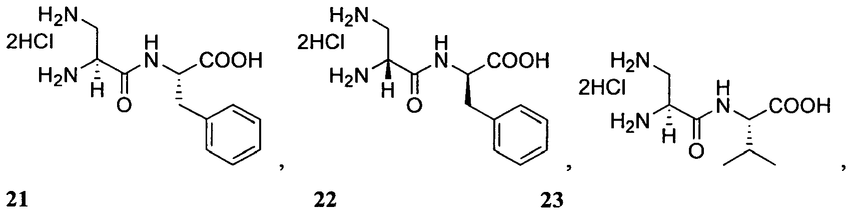 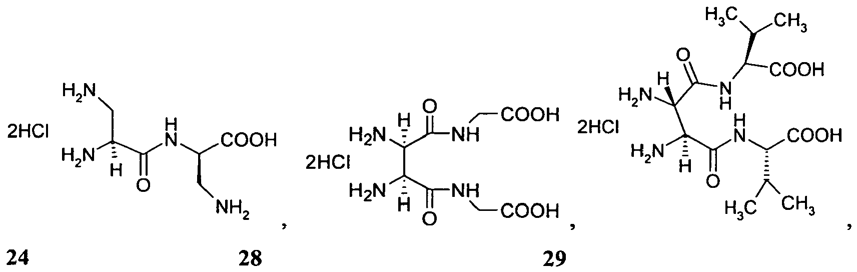 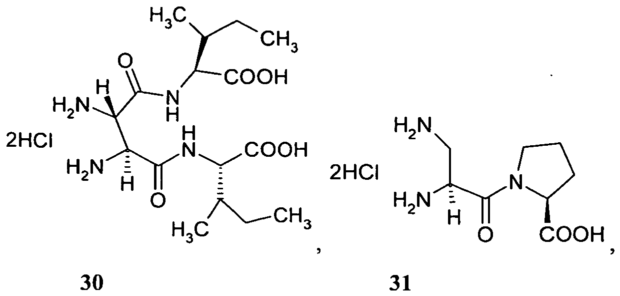 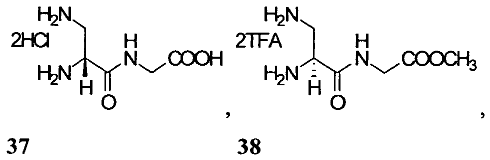 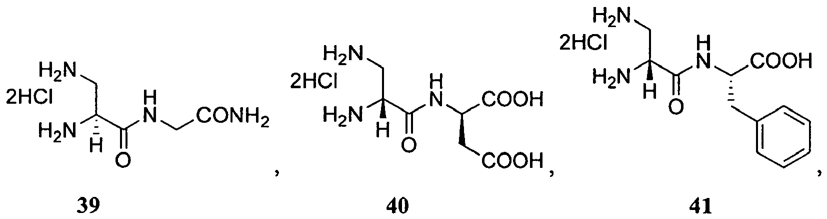 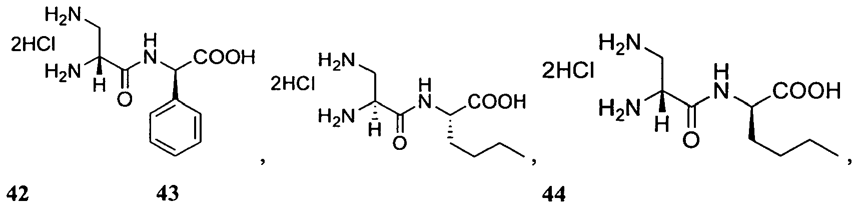 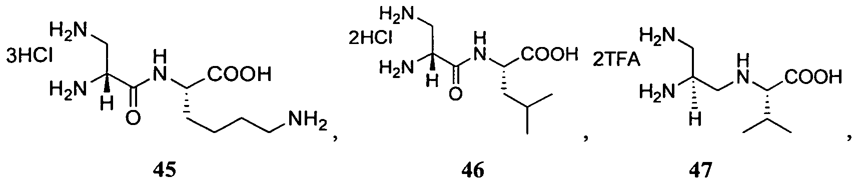 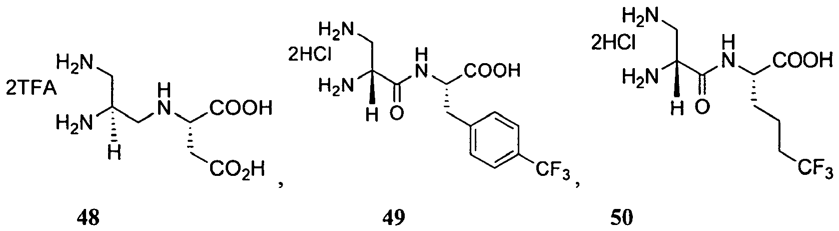 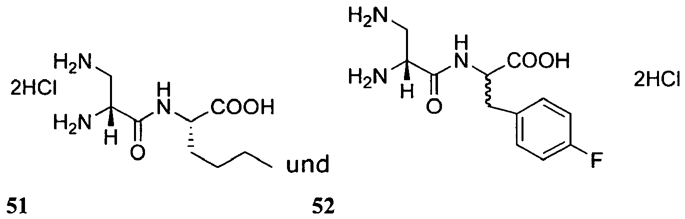 oder ihren pharmazeutisch akzeptablen Additionssalzen, Isomeren, Enantiomeren, Diastereoisomeren sowie deren Mischungen.

13. Verbindung nach einem der Ansprüche 10 bis 12, **dadurch gekennzeichnet, dass** das Medikament ein Fänger für reaktive Carbonylverbindungen ist, bevorzugt ein Inhibitor der Bildung von Advanced Glycation End Products.

14. Verbindung nach einem der Ansprüche 10 bis 13, **dadurch gekennzeichnet, dass** das Medikament bestimmt ist zur Vorbeugung und/oder Behandlung eines Zustandes oder von Krankheiten, die durch die Bildung von Advanced Glycation End Products oder durch die Vernetzung von Proteinen bedingt sind, zur Vorbeugung und/oder Behandlung von schädlichen Effekten der Alterung eines Organismus, wobei die Effekte die Bildung von Advanced Glycation End Products oder die Vernetzung von Proteinen sind, oder zur Verlangsamung oder zum Anhalten des Fortschreitens bei einem Patienten von Komplikationen, die aus einer Diabetes resultieren, wobei die Komplikationen aus der Bildung von Advanced Glycation End Products oder aus der Vernetzung von Proteinen resultieren.

15. Verbindung nach einem der Ansprüche 10 bis 14, **dadurch gekennzeichnet, dass** das Medikament bestimmt ist zum Behandeln, Vorbeugen und/oder Verlangsamen des Fortschreitens bei einem Patienten von Krankheiten, die ausgewählt sind aus rheumatoider Polyarthritis, Alzheimer-Krankheit, Urämie, neurodegenerativen Erkrankungen, Arteriosklerose, mikro- und makrovaskulären Komplikationen der Diabetes, darunter diabetische Retinopathie und renale Insuffizienz aufgrund von diabetischer Nephropathie, Mikro- und Makroangiopathien, Katarakt, mit Dialyse oder der Alzheimer-Krankheit assoziierte Amyloidose, Parkinson-Krankheit, Gingivitis, Karies, bukko-dentalen Problemen, diabetischem Ulcus, chronischer renaler Insuffizienz, chronischer renaler Dialyse, Entzündungskrankheiten, altersbedingten rheumatischen Störungen und Porphyrie, und zum Behandeln von Krebserkrankungen im frühen Stadium.

16. Verbindung nach einem der Ansprüche 10 bis 15, **dadurch gekennzeichnet, dass** das Medikament für eine Verabreichung durch orale Aufnahme bestimmt ist.

17. Kosmetische Verwendung einer Verbindung nach einem der Ansprüche 1 bis 7 als Wirkstoff gegen Alterung und zur Restrukturierung der Epidermis und der papillären Dermis, und/oder als Wirkstoff gegen Falten.
